# EUROPEAN PATENT APPLICATION

(11) **EP 4 155 298 A1**
(43) Date of publication of application: **29.03.2023**
(21) Application number: 21934691.3
(22) Date of filing: 22.12.2021
(51) Int. Cl.: C07D 307/91, C09K 11/06, H01L 51/54

(54) **ORGANIC ELECTROLUMINESCENT MATERIAL, ELECTRONIC ELEMENT, AND ELECTRONIC APPARATUS**

(30) Priority: 02.04.2021 CN 202110363862; 07.07.2021 CN 202110770912
(71) Applicant: Shaanxi Lighte Optoelectronics Material Co., Ltd., Xi'an Shaanxi 710065 (CN)
(72) Inventor: LIU, Wenqiang, Xi'an, Shaanxi 710100 (CN); KIM, Youngkook, Xi'an, Shaanxi 710065 (CN); LI, Yingwen, Xi'an, Shaanxi 710065 (CN); ZHANG, Heming, Xi'an, Shaanxi 710077 (CN)
(74) Representative: Noréns Patentbyrå AB
(86) International application number: PCT/CN2021/140628
(87) International publication number: WO 2022/206055

(57) **Abstract**

The present disclosure relates to the technical field of organic materials, and provides an organic electroluminescent material, an electronic element and an electronic device. The organic electroluminescent material has a structure as represented by Formula I. The organic electroluminescent material of the present disclosure is used in an organic electroluminescent device, and can effectively improve the performance of the device.

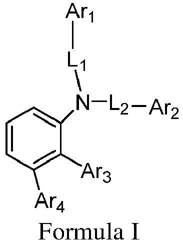

## Description

### CROSS REFERENCE TO RELATED APPLICATIONS

The present application claims priority to Chinese Patent Application No. 202110363862.0 filed on April 2, 2021 and Chinese Patent Application No. 202110770912.7 filed on July 7, 2021, the entire content of which is incorporated herein by reference.

### TECHNICAL FIELD

The present disclosure belongs to the technical field of organic materials, and in particular relates to an organic electroluminescent material, an electronic element and an electronic device.

### BACKGROUND

Organic electroluminescent devices, also known as organic light-emitting diodes, refer to devices in which organic light-emitting materials are excited by electric current to emit light under the action of electric fields. Compared with inorganic light-emitting materials, organic electroluminescent devices (OLEDs) have the advantages of active light emission, large optical path range, low driving voltage, high brightness, high efficiency, low energy consumption and simple fabrication process. Due to these advantages, organic electroluminescent materials and devices have become one of the most popular research topics in the scientific and industrial circles.

An organic electroluminescent device generally includes an anode, a hole transport layer, an organic electroluminescent layer as an energy conversion layer, an electron transport layer and a cathode which are stacked in sequence. When a voltage is applied to the cathode and the anode, the two electrodes generate an electric field. Under the action of the electric field, electrons on the cathode side move to the electroluminescent layer, holes on the anode side also move to the electroluminescent layer, the electrons and the holes combine in the electroluminescent layer to form excitons, and the excitons are in an excited state to release energy to the outside, such that the electroluminescent layer emits light to the outside.

In the prior art, organic electroluminescent materials for hole transport layers in organic electroluminescent devices have been disclosed. However, it is still necessary to continue to develop new materials to further improve the performance of electronic elements.

### SUMMARY

The objective of the present disclosure is to provide an organic electroluminescent material, an electronic element and an electronic device. The organic electroluminescent material can be used in an organic electroluminescent device to improve the performance of the organic electroluminescent device.

The first aspect of the present disclosure provides an organic electroluminescent material, having a structure as represented by Formula I: 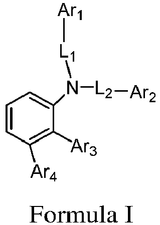
wherein Ar₁ and Ar₂ are the same or different, and are each independently selected from substituted or unsubstituted aryl with 6 to 40 carbon atoms, or substituted or unsubstituted heteroaryl with 3 to 30 carbon atoms;
L₁ and L₂ are the same or different, and are each independently selected from a single bond, substituted or unsubstituted arylene with 6 to 30 carbon atoms, or substituted or unsubstituted heteroarylene with 6 to 30 carbon atoms;
Ar₃ and Ar₄ are the same or different, and are each independently selected from substituted or unsubstituted aryl with 6 to 30 carbon atoms, or substituted or unsubstituted heteroaryl with 3 to 30 carbon atoms, and at least one of Ar₃ and Ar₄ is selected from 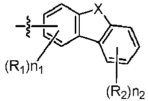 , wherein X is selected from C(R₃R₄), O, or S;
R₃ and R₄ are the same or different, and are each independently selected from alkyl with 1 to 5 carbon atoms, aryl with 6 to 12 carbon atoms, or heteroaryl with 3 to 12 carbon atoms; or, R₃ and R₄ form a saturated or unsaturated 5- to 13-membered ring;
R₁ and R₂ are the same or different, and are each independently selected from deuterium, a halogen group, cyano, alkyl with 1 to 5 carbon atoms, trialkylsilyl with 3 to 12 carbon atoms, aryl with 6 to 12 carbon atoms, or heteroaryl with 3 to 12 carbon atoms;
R₁ and R₂ are represented by Rᵢ, n₁ and n₂ are represented by nᵢ, nᵢ represents the number of Rᵢ, i is a variable and represents 1 and 2, and when i is 1 and 2, nᵢ is selected from 0, 1, 2, 3 or 4; when nᵢ is greater than 1, any two Rᵢs are the same or different; optionally, any two adjacent Rᵢs are connected to each other to form an unsaturated 6- to 10-membered ring;
the substituents in Ar₁, Ar₂, L₁, L₂, Ar₃ and Ar₄ are the same or different, and are each independently selected from deuterium, a halogen group, cyano, trialkylsilyl with 3 to 12 carbon atoms, triphenylsilyl, alkyl with 1 to 10 carbon atoms, aryl with 6 to 20 carbon atoms, heteroaryl with 3 to 20 carbon atoms, or cycloalkyl with 3 to 10 carbon atoms; optionally, any two adjacent substituents in Ar₁ and Ar₂ form a substituted or unsubstituted 3- to 15-membered ring, and the substituents in the 3- to 15-membered ring are independently selected from deuterium, a halogen group, cyano, trialkylsilyl with 3 to 6 carbon atoms, triphenylsilyl, or alkyl with 1 to 5 carbon atoms.

The second aspect of the present disclosure provides an electronic element, comprising an anode and a cathode which is arranged oppositely to the anode, and a functional layer arranged between the anode and the cathode; and the functional layer comprises the above-mentioned organic electroluminescent material.

The third aspect of the present disclosure provides an electronic device, comprising the above-mentioned electronic element.

In the molecular structure of the organic electroluminescent material of the present disclosure, three consecutive adjacent substitution positions of benzene are sequentially connected to an aromatic amine, the first aromatic group (Ar₃) and the second aromatic group (Ar₄), and at least one of the first aromatic group and the second aromatic group contains a specific dibenzo five-membered ring, which enhances the steric configuration ability of molecules, increases the glass transition temperature of the material, and at the same time enhances intermolecular conjugation, thereby forming high LUMO while forming deep HOMO. In addition, the N in the aromatic amine attached to the benzene can increase the molecular conjugation, thereby achieving high efficiency of a device. Even molecules with smaller molecular weights have high Tg, recrystallization can be prevented when the device is driven. The organic electroluminescent material of the present disclosure has relatively high stability, which is beneficial to the fabrication of electronic devices with longer life.

Other features and advantages of the present disclosure will be described in detail in the following specific embodiments.

### BRIEF DESCRIPTION OF THE DRAWINGS

The accompanying drawings are used to provide a further understanding of the present disclosure, constitute a part of the description, and are used for interpreting the present disclosure together with the following specific embodiments, rather than limiting the present disclosure.
Fig. 1 is a schematic structural diagram of an organic electroluminescent element according to an embodiment of the present disclosure.
Fig. 2 is a schematic structural diagram of a first electronic device according to an embodiment of the present disclosure.
Fig. 3 is a schematic structural diagram of a photoelectric conversion element according to an embodiment of the present disclosure.
Fig. 4 is a schematic structural diagram of a second electronic device according to an embodiment of the present disclosure.

**Reference numerals:**

| | | | |
|---|---|---|---|
| 100. Anode | 200. Cathode | 300. Functional layer | 310. Hole injection layer |
| 321. First hole transport layer | | 322. Second hole transport layer | |
| 330. Organic light-emitting layer | | 320. Hole transport layer | |
| 340. Electron transport layer | | 350. Electron injection layer | |
| 360. Photoelectric conversion layer | | 400. First electronic device | |
| 500. Second electronic device | | | |

### DETAILED DESCRIPTION

Example embodiments are now described more comprehensively with reference to the accompanying drawings. However, the example embodiments can be implemented in various forms, and should not be construed as being limited to the examples set forth herein. On the contrary, the provision of these embodiments makes the present disclosure more comprehensive and complete, and fully conveys the concept of the example embodiments to those skilled in the art. The features, structures or characteristics described may be combined in one or more embodiments in any suitable way. In the following description, many specific details are provided to give a sufficient understanding of the embodiments of the present disclosure.

The first aspect of the present disclosure provides an organic electroluminescent material, having a structure as represented by Formula I: 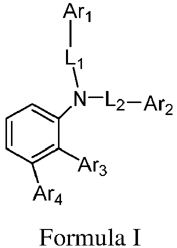
wherein Ar₁ and Ar₂ are the same or different, and are each independently selected from substituted or unsubstituted aryl with 6 to 40 carbon atoms, or substituted or unsubstituted heteroaryl with 3 to 30 carbon atoms;
L₁ and L₂ are the same or different, and are each independently selected from a single bond, substituted or unsubstituted arylene with 6 to 30 carbon atoms, or substituted or unsubstituted heteroarylene with 6 to 30 carbon atoms;
Ar₃ and Ar₄ are the same or different, and are each independently selected from substituted or unsubstituted aryl with 6 to 30 carbon atoms, or substituted or unsubstituted heteroaryl with 3 to 30 carbon atoms, and at least one of Ar₃ and Ar₄ is selected from 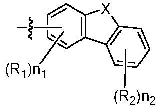 , wherein X is selected from C(R₃R₄), O, or S;
R₃ and R₄ are the same or different, and are each independently selected from alkyl with 1 to 5 carbon atoms, aryl with 6 to 12 carbon atoms, or heteroaryl with 3 to 12 carbon atoms; or, R₃ and R₄ form a saturated or unsaturated 5- to 13-membered ring;
R₁ and R₂ are the same or different, and are each independently selected from deuterium, a halogen group, cyano, alkyl with 1 to 5 carbon atoms, trialkylsilyl with 3 to 12 carbon atoms, aryl with 6 to 12 carbon atoms, or heteroaryl with 3 to 12 carbon atoms;
R₁ and R₂ are represented by Rᵢ, n₁ and n₂ are represented by nᵢ, nᵢ represents the number of Rᵢ, i is a variable and represents 1 and 2, and when i is 1 and 2, nᵢ is selected from 0, 1, 2, 3 or 4; when nᵢ is greater than 1, any two Rᵢs are the same or different; optionally, any two adjacent Rᵢs are connected to each other to form an unsaturated 6- to 10-membered ring;
the substituents in Ar₁, Ar₂, L₁, L₂, Ar₃ and Ar₄ are the same or different, and are each independently selected from deuterium, a halogen group, cyano, trialkylsilyl with 3 to 12 carbon atoms, triphenylsilyl, alkyl with 1 to 10 carbon atoms, aryl with 6 to 20 carbon atoms, heteroaryl with 3 to 20 carbon atoms, or cycloalkyl with 3 to 10 carbon atoms; optionally, any two adjacent substituents in Ar₁ and Ar₂ form a substituted or unsubstituted 3- to 15-membered ring, and the substituents in the 3- to 15-membered ring are independently selected from deuterium, a halogen group, cyano, trialkylsilyl with 3 to 6 carbon atoms, triphenylsilyl, or alkyl with 1 to 5 carbon atoms.

In the present disclosure, the terms "optional" and "optionally" mean that the subsequently described event or circumstance may but need not occur, and the description includes situations where the event or circumstance occurs or does not occur. For example, "optionally, two adjacent substituents XX form a ring" means that the two substituents may but does not have to form a ring, including: the situation where the two adjacent substituents form a ring and the situation where the two adjacent substituents do not form a ring. For another example, "optionally, any two adjacent substituents in Ar₁ and Ar₂ form a substituted or unsubstituted 3- to 15-membered ring" means that any two adjacent substituents in Ar₁ and Ar₂ may form a substituted or unsubstituted 3- to 15-membered ring, or may each independently exist.

In the present disclosure, in the expression "any two adjacent substituents form a ring", "any two adjacent" may include two substituents on the same atom, and may also include one substituent on each of two adjacent atoms. When there are two substituents on the same atom, the two substituents may form a saturated or unsaturated ring with the atom to which they are jointly connected. When there is one substituent on each of two adjacent atoms, the two substituents may be fused into a ring. For example, "any two adjacent Rᵢs are connected to each other to form an unsaturated 6- to 10-membered ring" includes the situation that any two adjacent R₁s are connected to each other to form an unsaturated 6- to 10-membered ring with the atom to which they are jointly connected, or any two adjacent R₂s are connected to each other to form an unsaturated 6- to 10-membered ring with the atom to which they are jointly connected. The ring may be, for example, an aromatic ring. Specific examples of the aromatic ring include a benzene ring 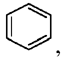 a naphthalene ring 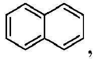 a phenanthrene ring 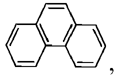 etc.

Optionally, one of Ar₃ and Ar₄ is selected from 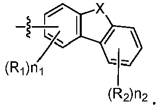 Ar₃ is 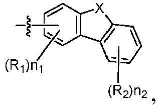 and Ar₄ is not 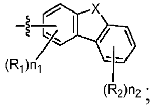 or Ar₄ is 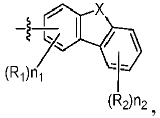 and Ar₃ is not

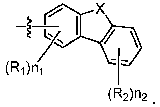

Optionally, both Ar₃ and Ar₄ are selected from

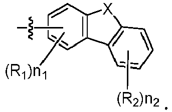

In the present disclosure, 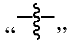 refers to a position bound to other substituents or binding positions.

In the present disclosure, the used descriptions "each independently selected from" and "independently selected from" can be interchanged, should be understood in a broad sense, and may indicate that the specific options expressed by the same symbols do not affect each other in different groups, or the specific options expressed by the same symbols do not affect each other in the same groups. For example, 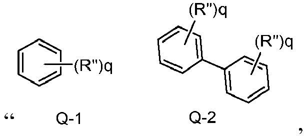 wherein each q is independently 0, 1, 2 or 3, and each R" is independently selected from hydrogen, deuterium, fluorine and chlorine" means: formula Q-1 represents that there are q substituents R" on the benzene ring, each R" may be the same or different, and the options of each R" do not affect each other; and formula Q-2 represents that each benzene ring of biphenyl has q substituents R", the numbers q of R" substituents on two benzene rings may be the same or different, each R" may be the same or different, and the options of each R" do not affect each other.

In the present disclosure, the term "substituted or unsubstituted" indicates that the functional group described behind the term may or may not have a substituent (hereinafter, for the convenience of description, the substituents are collectively referred to as Rc). For example, "substituted or unsubstituted aryl" indicates aryl with a substituent Rc or unsubstituted aryl. The above substituent, namely Rc, may be, for example, deuterium, a halogen group, cyano, trialkylsilyl, triphenylsilyl, alkyl, aryl, heteroaryl, or cycloalkyl.

In the present disclosure, the number of carbon atoms of a substituted or unsubstituted functional group refers to the number of all carbon atoms. For example, if L is selected from substituted arylene with 12 carbon atoms, the number of all carbon atoms in the arylene and substituents thereon is 12. For example: Ar is 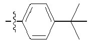 , the number of carbon atoms of which is 10; and L is 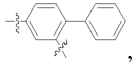 the number of carbon atoms of which is 12.

In the present disclosure, the aryl refers to an optional functional group or substituent derived from an aromatic carbon ring. The aryl may be monocyclic aryl (e.g., phenyl) or polycyclic aryl. In other words, the aryl may be monocyclic aryl, fused-ring aryl, two or more monocyclic aryls conjugated by carbon-carbon bonds, monocyclic aryl and fused-ring aryl that are conjugated by carbon-carbon bonds, or two or more fused-ring aryls conjugated by carbon-carbon bonds. That is, unless otherwise specified, two or more aromatic groups conjugated by carbon-carbon bonds may also be considered as aryl in the present disclosure. The fused-ring aryl may include, for example, bicyclic fused aryl (e.g., naphthyl), tricyclic fused aryl (e.g., phenanthrenyl, fluorenyl, anthracenyl), etc. The aryl does not contain heteroatoms such as B, N, O, S, P, Se and Si. For example, in the present disclosure, biphenyl, terphenyl, etc. are aryl. Examples of the aryl may include, but are not limited to, phenyl, naphthyl, fluorenyl, anthryl, phenanthryl, biphenyl, terphenyl, benzo[9,10]phenanthryl, pyrenyl, benzofluoranthenyl, chrysenyl, etc. In the present disclosure, the arylene referred to refers to a divalent group formed by the further loss of one hydrogen atom from the aryl.

In the present disclosure, the substituted aryl may indicate that one or two or more hydrogen atoms in the aryl are substituted by a group(s) such as a deuterium atom, a halogen group, -CN, aryl, heteroaryl, trialkylsilyl, alkyl, cycloalkyl, and alkoxy. Specific examples of the aryl substituted by heteroaryl include, but are not limited to, phenyl substituted by dibenzofuranyl, phenyl substituted by dibenzothienyl, phenyl substituted by pyridyl, etc. It should be understood that the number of carbon atoms in the substituted aryl refers to the total number of carbon atoms in the aryl and the substituents on the aryl, for example, the substituted aryl with 18 carbon atoms indicates that the total number of carbon atoms in the aryl and the substituents thereon is 18.

In the present disclosure, the heteroaryl refers to a monovalent aromatic ring containing at least one heteroatom or derivatives thereof, and the heteroatom may be at least one of B, O, N, P, Si, Se and S. The heteroaryl may be monocyclic heteroaryl or polycyclic heteroaryl. In other words, the heteroaryl may be a system of a single aromatic ring or a system of multiple aromatic rings conjugated by carbon-carbon bonds, and any aromatic ring system is an aromatic monocyclic ring or an aromatic fused ring. Illustratively, the heteroaryl may include thienyl, furyl, pyrrolyl, imidazolyl, thiazolyl, oxazolyl, oxadiazolyl, triazolyl, pyridyl, bipyridyl, pyrimidinyl, triazinyl, acridinyl, pyridazinyl, pyrazinyl, quinolinyl, quinazolinyl, quinoxalinyl, phenoxazinyl, phthalazinyl, pyridopyrimidinyl, pyridopyrazinyl, pyrazinopyrazinyl, isoquinolinyl, indolyl, carbazolyl, benzoxazolyl, benzimidazolyl, benzothiazolyl, benzocarbazolyl, benzothienyl, dibenzothienyl, thienothienyl, benzofuranyl, phenanthrolinyl, isoxazolyl, thiadiazolyl, benzothiazolyl, phenothiazinyl, silafluorenyl, dibenzofuranyl, N-phenylcarbazolyl, N-pyridylcarbazolyl, N-methylcarbazolyl, etc., but is not limited thereto. Among them, the thienyl, furanyl, phenanthrolinyl, etc. are heteroaryls of a single aromatic ring system type, and N-phenylcarbazolyl and N-pyridylcarbazolyl are heteroaryls of a polycyclic system type conjugated by carbon-carbon bonds. In the present disclosure, the heteroarylene referred to refers to a divalent group formed by the further loss of one hydrogen atom from the heteroaryl.

In the present disclosure, the substituted heteroaryl may indicate that one or two or more hydrogen atoms in the heteroaryl are substituted by a group(s) such as a deuterium atom, a halogen group, -CN, aryl, heteroaryl, trialkylsilyl, alkyl, cycloalkyl, and alkoxy. Specific examples of the heteroaryl substituted by aryl include, but are not limited to, dibenzofuranyl substituted by phenyl, dibenzothienyl substituted by phenyl, pyridyl substituted by phenyl, etc. It should be understood that the number of carbon atoms in the substituted heteroaryl refers to the total number of carbon atoms in the heteroaryl and the substituents on the heteroaryl.

In the present disclosure, the number of carbon atoms of the aryl as a substituent may be 6 to 20, for example, the number of carbon atoms may be 6, 7, 8, 9, 10, 11, 12, 13, 14, 15, 16, 17, 18, 19, or 20. Specific examples of the aryl as a substituent include, but are not limited to, phenyl, biphenyl, naphthyl, anthryl, phenanthryl, and chrysenyl.

In the present disclosure, the number of carbon atoms of the heteroaryl as a substituent may be 3 to 20, for example, the number of carbon atoms may be 3, 4, 5, 6, 7, 8, 9, 10, 11, 12, 13, 14, 15, 16, 17, 18, 19, or 20. Specific examples of the heteroaryl as a substituent include, but are not limited to, pyridyl, pyrimidinyl, carbazolyl, dibenzofuranyl, dibenzothienyl, quinolinyl, quinazolinyl, quinoxalinyl, and isoquinolinyl.

In the present disclosure, a non-positioned connection bond refers to a single bond 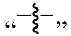 extending out from a ring system, which indicates that one end of the connection bond can be connected to any position in the ring system through which the bond penetrates, and the other end is connected to the rest of a compound molecule.

For example, as shown in the following formula (f), the naphthyl represented by formula (f) is connected to other positions of a molecule by two non-positioned connection bonds penetrating through double rings, which includes any possible connection shown in formulas (f-1) to (f-10):

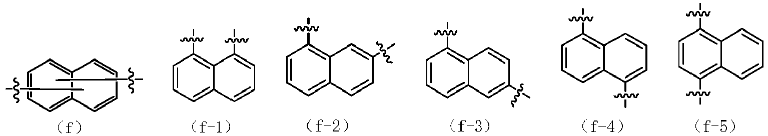

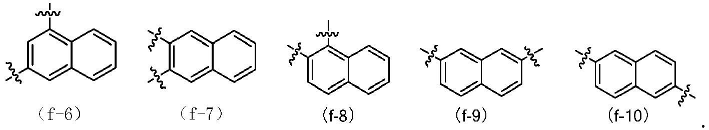

For another example, as shown in the following formula (X'), the dibenzofuranyl represented by formula (X') is connected to other positions of a molecule by a non-positioned connection bond extending out from the middle of a benzene ring on one side, which includes any possible connection shown in formulas (X'-1) to (X'-4):

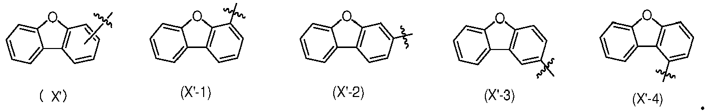

In the present disclosure, the alkyl with 1 to 10 carbon atoms may include linear alkyl with 1 to 10 carbon atoms and branched alkyl with 3 to 10 carbon atoms. The number of carbon atoms of the alkyl may be, for example, 1, 2, 3, 4, 5, 6, 7, 8, 9, or 10. Specific examples of the alkyl include, but are not limited to, methyl, ethyl, n-propyl, isopropyl, n-butyl, isobutyl, tert-butyl, n-pentyl, isopentyl, neopentyl, n-hexyl, n-heptyl, n-octyl, 2-ethylhexyl, nonyl, decyl, 3,7-dimethyloctyl, etc.

In the present disclosure, specific examples of the cycloalkyl include, but are not limited to, cyclopentyl, cyclohexyl, and adamantyl.

In the present disclosure, the halogen group may be, for example, fluorine, chlorine, bromine, or iodine.

In the present disclosure, specific examples of the trialkylsilyl include, but are not limited to, trimethylsilyl, triethylsilyl, etc.

In some embodiments, Ar₁ and Ar₂ are each independently selected from substituted or unsubstituted aryl with 6 to 33 carbon atoms, or substituted or unsubstituted heteroaryl with 5 to 18 carbon atoms. For example, Ar₁ and Ar₂ are each independently selected from substituted or unsubstituted aryl with 6, 7, 8, 9, 10, 11, 12, 13, 14, 15, 16, 17, 18, 19, 20, 21, 22, 23, 24, 25, 26, 27, 28, 29, 30, 31, 32 or 33 carbon atoms, or substituted or unsubstituted heteroaryl with 5, 6, 7, 8, 9, 10, 11, 12, 13, 14, 15, 16, 17 or 18 carbon atoms.

Preferably, the substituents in Ar₁ and Ar₂ are each independently selected from deuterium, fluorine, cyano, trimethylsilyl, triphenylsilyl, alkyl with 1 to 5 carbon atoms, aryl with 6 to 12 carbon atoms, heteroaryl with 5 to 12 carbon atoms, or cycloalkyl with 5 to 10 carbon atoms; and optionally, any two adjacent substituents in Ar₁ and Ar₂ form a substituted or unsubstituted 5- to 13-membered ring, and the substituents in the 5- to 13-membered ring are each independently selected from deuterium, fluorine, cyano, trimethylsilyl, triphenylsilyl, methyl, ethyl, isopropyl, or tert-butyl.

Optionally, Ar₁ and Ar₂ are each independently selected from substituted or unsubstituted phenyl, substituted or unsubstituted naphthyl, substituted or unsubstituted biphenyl, substituted or unsubstituted fluorenyl, substituted or unsubstituted dibenzofuranyl, substituted or unsubstituted dibenzothienyl, substituted or unsubstituted carbazolyl, substituted or unsubstituted phenanthryl, substituted or unsubstituted triphenylene, substituted or unsubstituted pyrenyl, or substituted or unsubstituted anthryl.

Preferably, the substituents in Ar₁ and Ar₂ are each independently selected from deuterium, fluorine, cyano, trimethylsilyl, triphenylsilyl, methyl, ethyl, isopropyl, tert-butyl, cyclopentyl, cyclohexyl, adamantyl, pyridyl, phenyl, naphthyl, or biphenyl; and optionally, any two adjacent substituents in Ar₁ and Ar₂ form adamantane 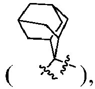 cyclopentane 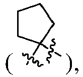 cyclohexane 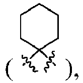 a fluorene ring 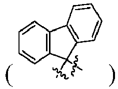 or a tert-butyl substituted fluorene ring

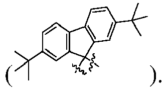

Optionally, Ar₁ and Ar₂ are each independently selected from a substituted or unsubstituted group W, and the unsubstituted group W is selected from the following groups: 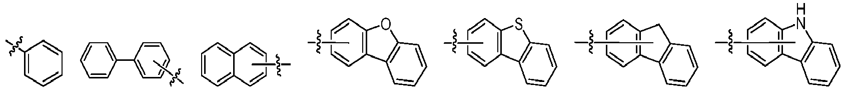 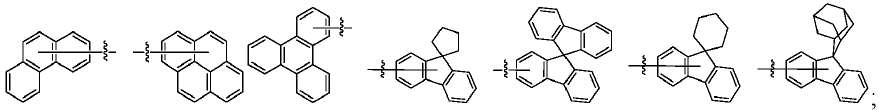 wherein the substituted group W has one or two or more substituents, the substituents are each independently selected from deuterium, fluorine, cyano, trimethylsilyl, triphenylsilyl, methyl, ethyl, isopropyl, tert-butyl, cyclopentyl, cyclohexyl, adamantyl, pyridyl, phenyl, naphthyl, or biphenyl, and when the number of substituents is greater than 1, the substituents are the same or different.

Optionally, Ar₁ and Ar₂ are each independently selected from the following groups:

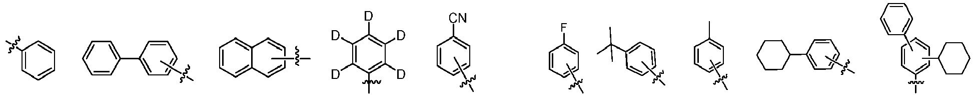

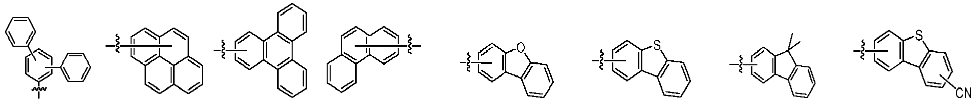

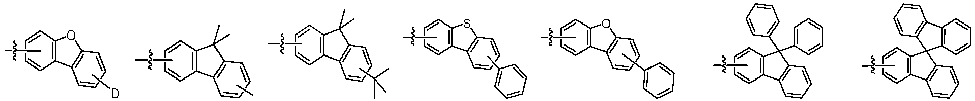

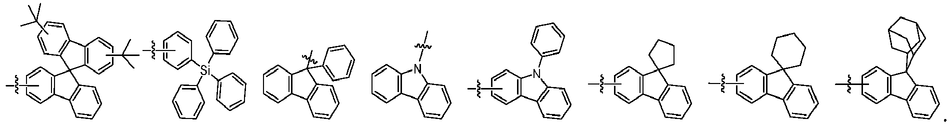

Further optionally, Ar₁ and Ar₂ are each independently selected from the following groups:

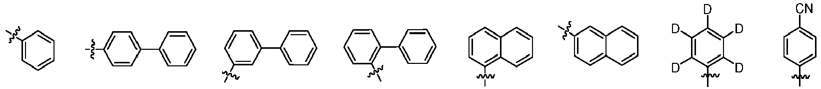

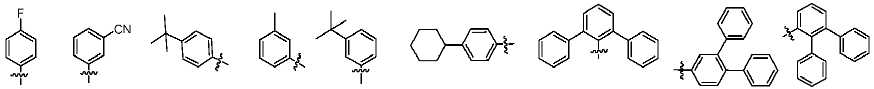

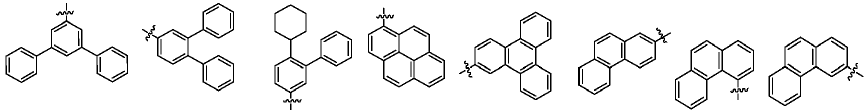

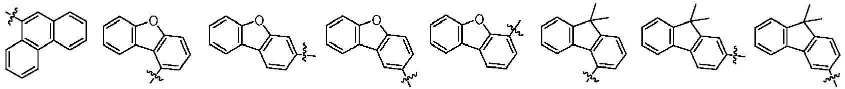

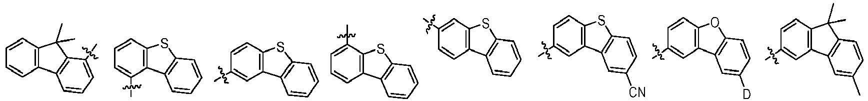

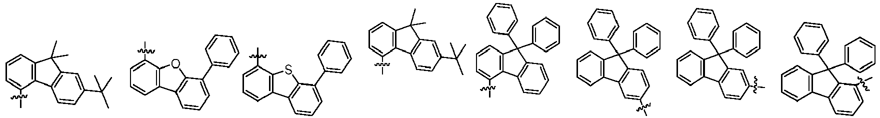

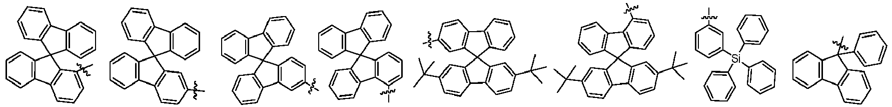

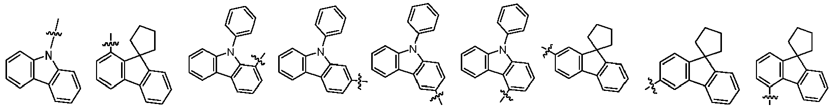

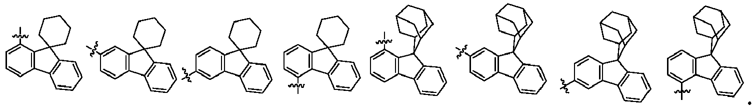

In some embodiments, L₁ and L₂ are each independently selected from a single bond, or substituted or unsubstituted arylene with 6 to 20 carbon atoms. For example, L₁ and L₂ are each independently selected from a single bond, or substituted or unsubstituted arylene with 6, 7, 8, 9, 10, 11, 12, 13, 14, 15, 16, 17, 18, 19, or 20 carbon atoms.

Preferably, the substituents in L₁ and L₂ are each independently selected from deuterium, fluorine, cyano, alkyl with 1 to 5 carbon atoms, aryl with 6 to 12 carbon atoms, or trimethylsilyl.

Optionally, L₁ and L₂ are each independently selected from a single bond, or substituted or unsubstituted arylene with 6 to 12 carbon atoms.

Preferably, the substituents in L₁ and L₂ are each independently selected from deuterium, fluorine, cyano, methyl, ethyl, isopropyl, tert-butyl, phenyl, or trimethylsilyl.

Optionally, L₁ and L₂ are each independently selected from a single bond, substituted or unsubstituted phenylene, substituted or unsubstituted naphthylene, or substituted or unsubstituted biphenylene.

Preferably, the substituents in L₁ and L₂ are each independently selected from deuterium, fluorine, cyano, methyl, ethyl, isopropyl, tert-butyl, phenyl, or trimethylsilyl.

In some embodiments, L₁ and L₂ are each independently selected from a single bond, or a substituted or unsubstituted group V, and the unsubstituted group V is selected from the following groups: 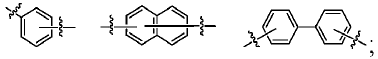 wherein the substituted group V has one or two or more substituents, the substituents are each independently selected from deuterium, fluorine, cyano, methyl, ethyl, isopropyl, tert-butyl, phenyl, naphthyl, or trimethylsilyl, and when the number of substituents is greater than 1, the substituents are the same or different.

Optionally, L₁ and L₂ are each independently selected from a single bond or the following groups:

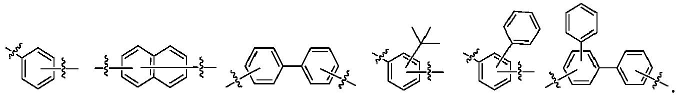

Optionally, L₁ and L₂ are each independently selected from a single bond or the following groups:

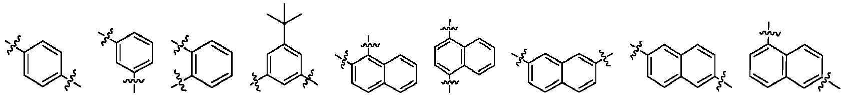

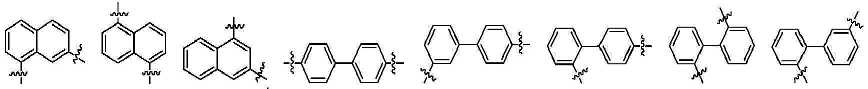

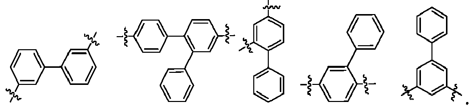

In an embodiment of the present disclosure, Ar₃ and Ar₄ are each independently selected from substituted or unsubstituted aryl with 6 to 20 carbon atoms, or substituted or unsubstituted heteroaryl with 12 to 16 carbon atoms; and at least one of Ar₃ and Ar₄ is selected from 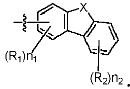 For example, Ar₃ and Ar₄ are each independently selected from substituted or unsubstituted aryl with 6, 7, 8, 9, 10, 11, 12, 13, 14, 15, 16, 17, 18, 19 or 20 carbon atoms, or substituted or unsubstituted heteroaryl with 12, 13, 14, 15 or 16 carbon atoms.

Preferably, the substituents in Ar₃ and Ar₄ are each independently selected from deuterium, fluorine, cyano, trimethylsilyl, triphenylsilyl, alkyl with 1 to 5 carbon atoms, aryl with 6 to 12 carbon atoms, or heteroaryl with 3 to 12 carbon atoms.

Optionally, Ar₃ and Ar₄ are each independently selected from a substituted or unsubstituted group Q, and the unsubstituted group Q is selected from the following groups: 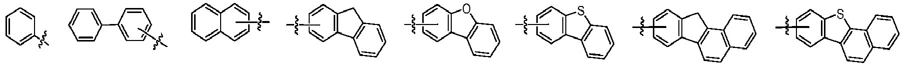 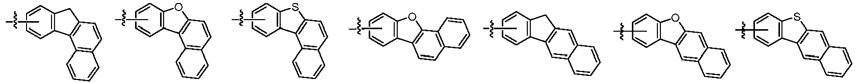 wherein the substituted group Q has one or two or more substituents, the substituents are each independently selected from deuterium, fluorine, cyano, trimethylsilyl, triphenylsilyl, methyl, ethyl, isopropyl, tert-butyl, phenyl, naphthyl, or biphenyl, and when the number of substituents is greater than 1, the substituents are the same or different.

Further optionally, Ar₃ and Ar₄ are each independently selected from the following groups:

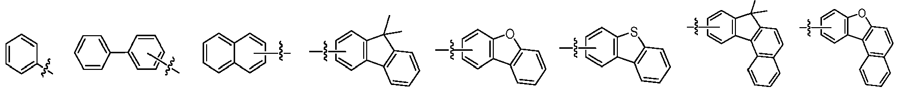

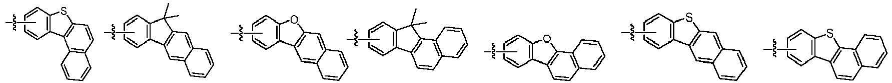

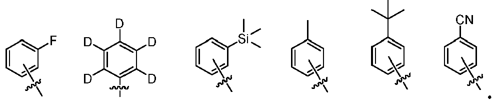

More optionally, Ar₃ and Ar₄ are each independently selected from the following groups:

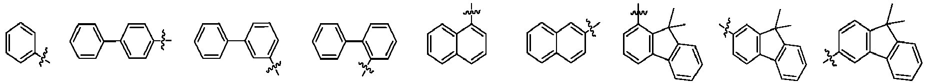

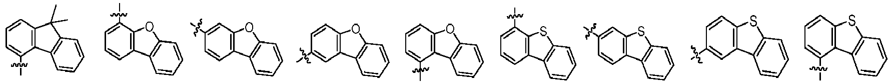

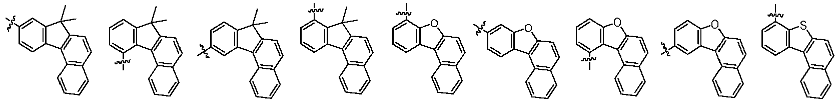

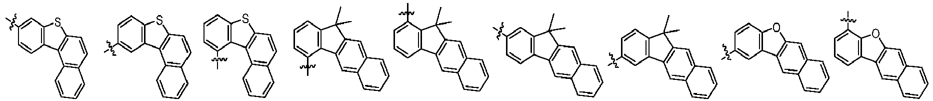

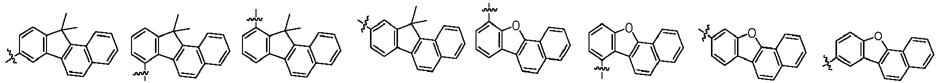

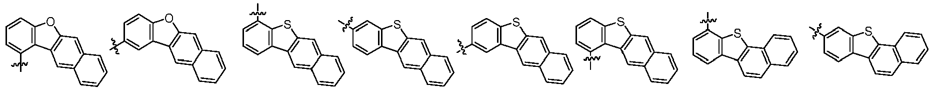

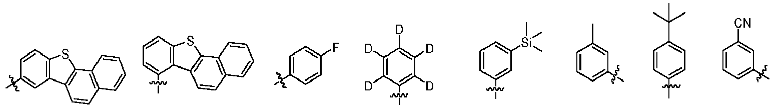

Preferably, Ar₃ is selected from

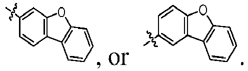

In some embodiments, R₃ and R₄ are each independently selected from methyl, ethyl, isopropyl, tert-butyl, phenyl, naphthyl, biphenyl, pyridyl, pyrimidinyl, quinolyl, isoquinolyl, dibenzofuranyl, dibenzothienyl, or carbazolyl; alternatively, R₃ and R₄ form cyclopentane, cyclohexane, or a fluorene ring.

In some embodiments, R₁ and R₂ are each independently selected from deuterium, fluorine, cyano, methyl, ethyl, isopropyl, tert-butyl, trimethylsilyl, phenyl, naphthyl, biphenyl, pyridyl, pyrimidinyl, quinolyl, isoquinolyl, dibenzofuranyl, dibenzothienyl, or carbazolyl.

Optionally, the organic electroluminescent material is selected from a group consisting of the following compounds:

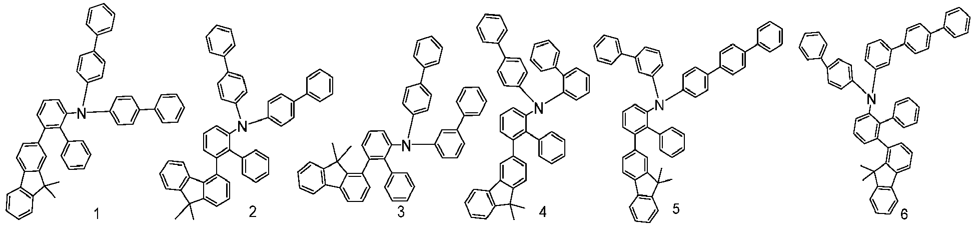

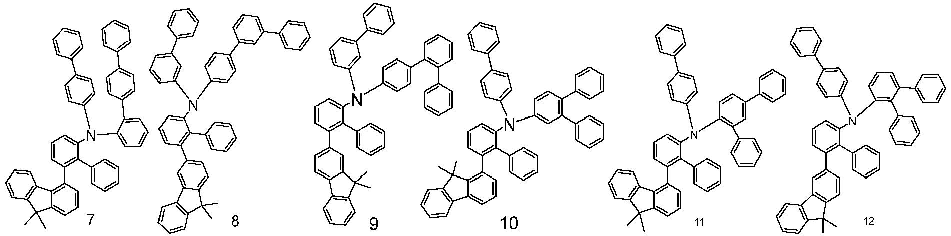

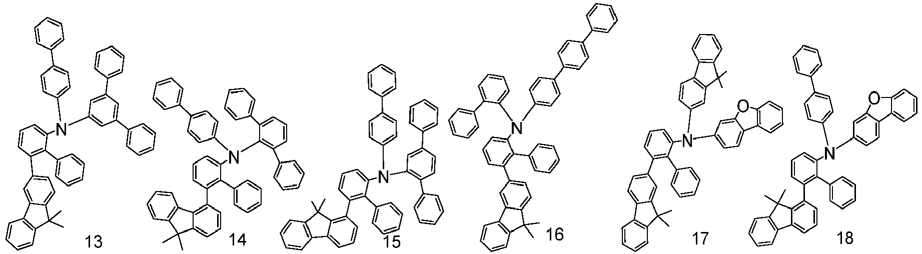

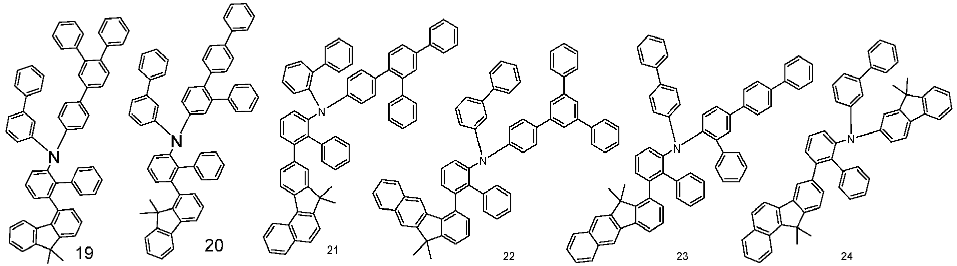

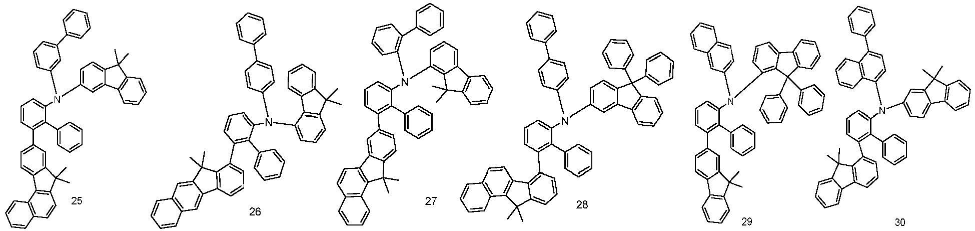

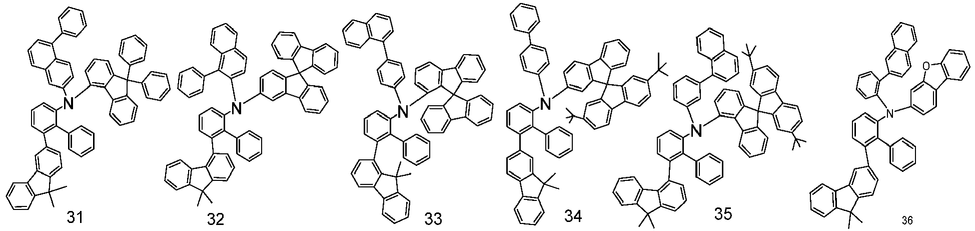

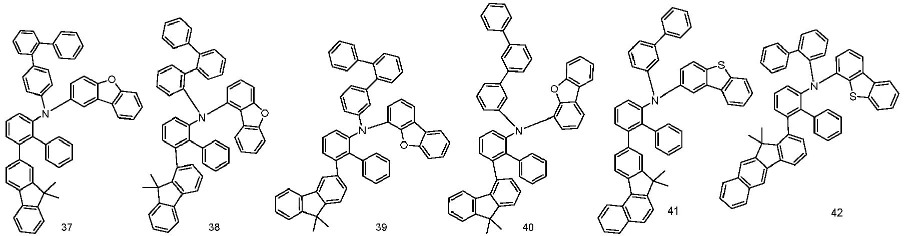

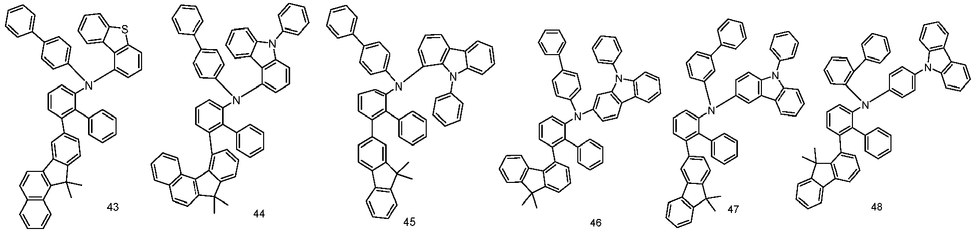

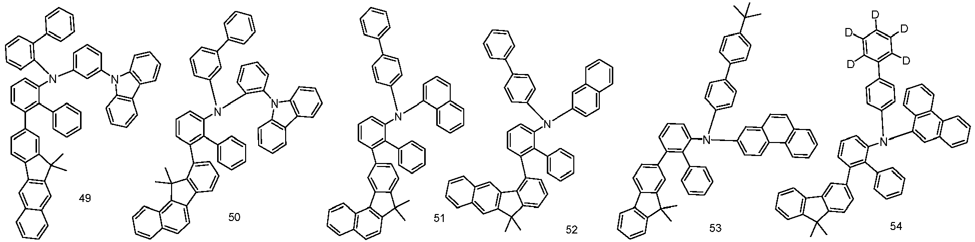

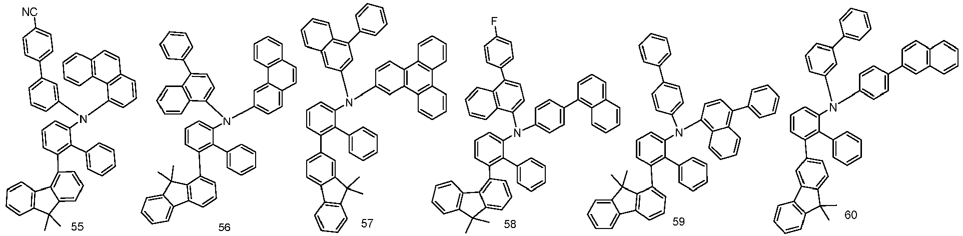

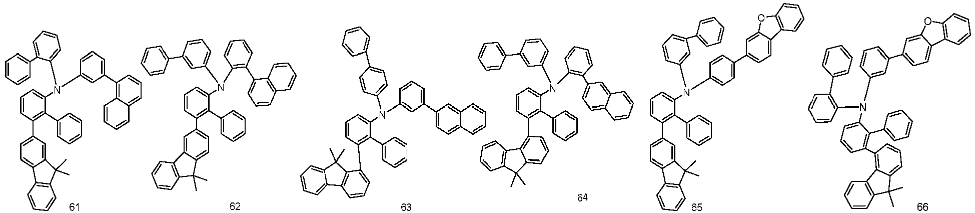

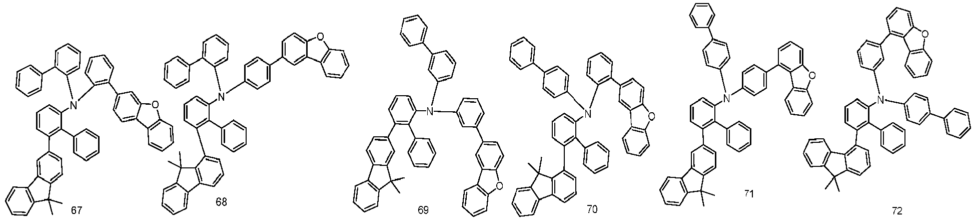

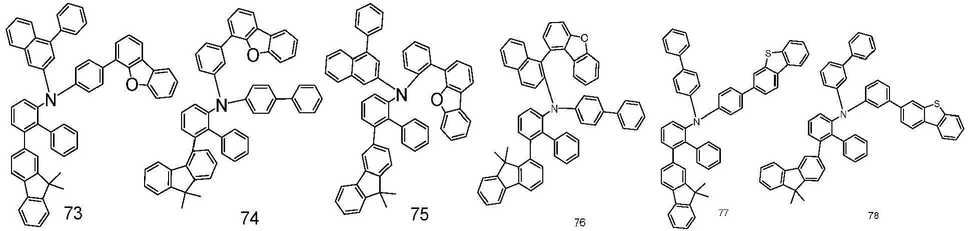

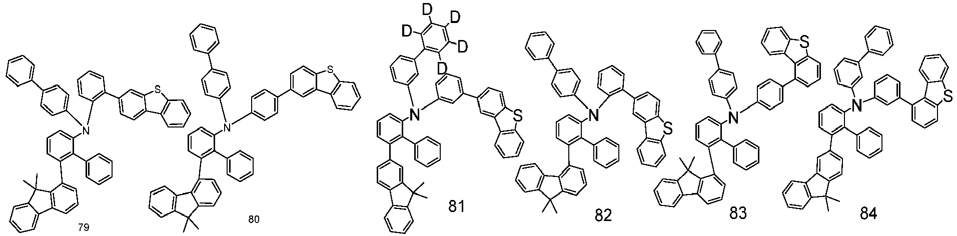

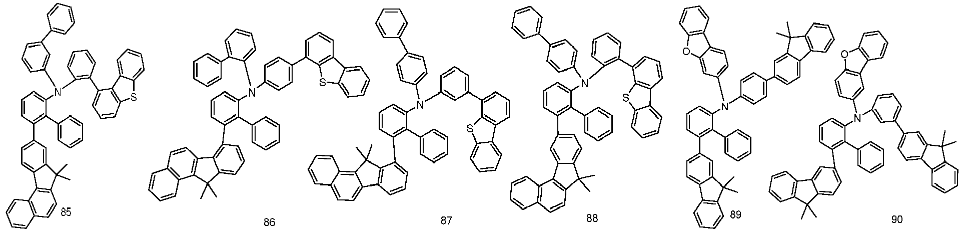

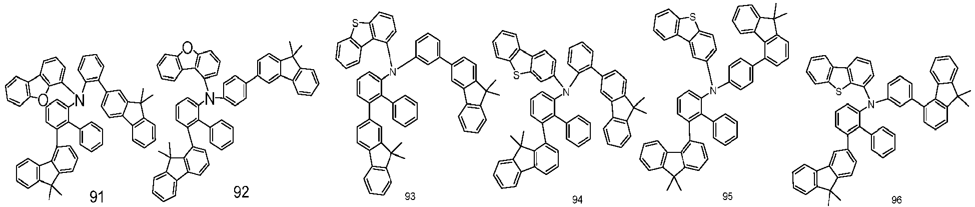

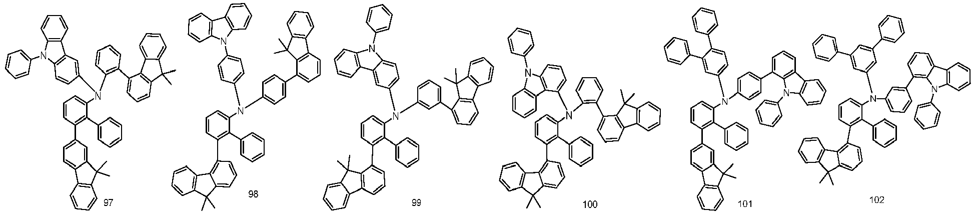

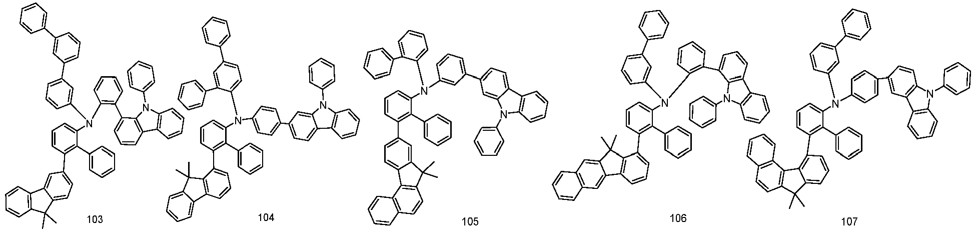

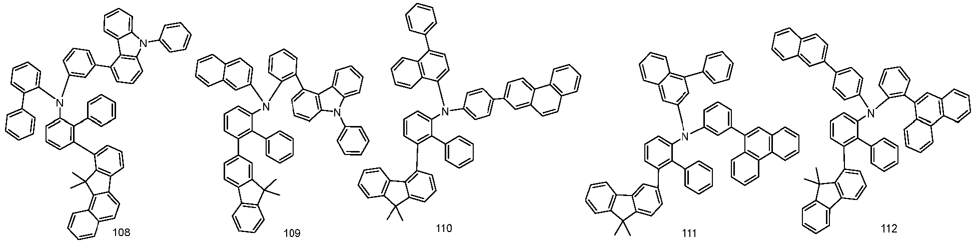

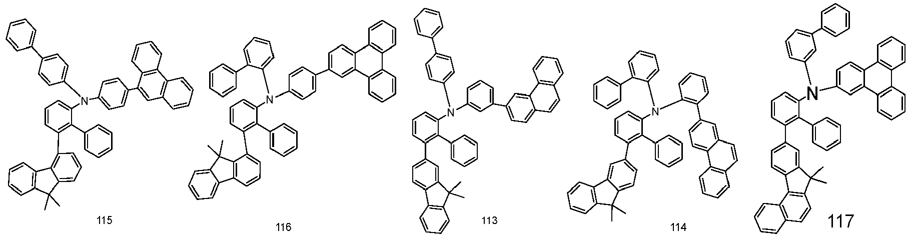

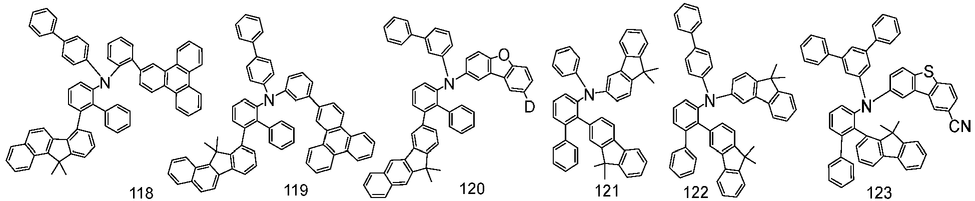

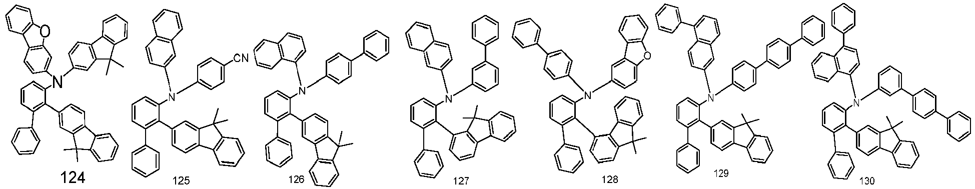

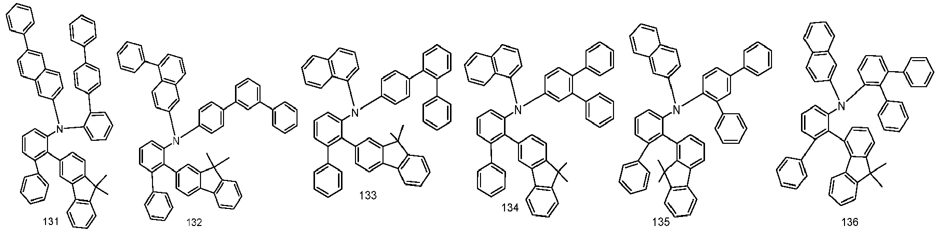

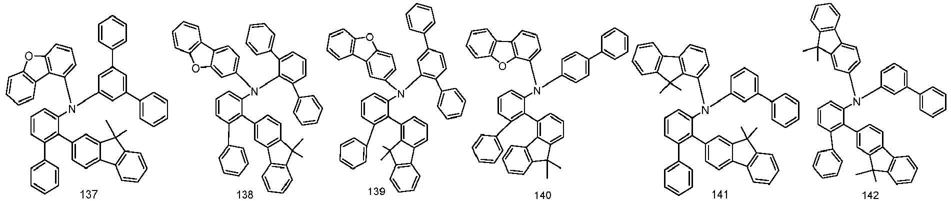

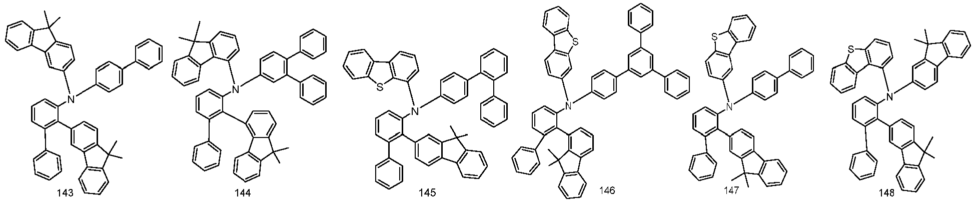

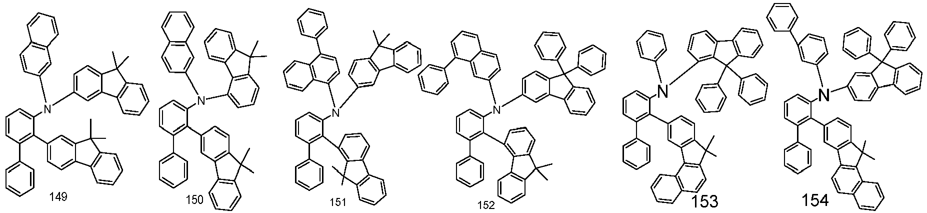

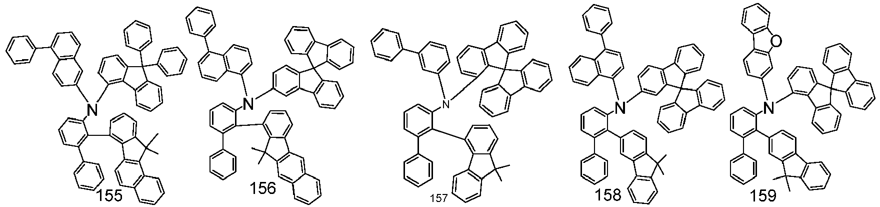

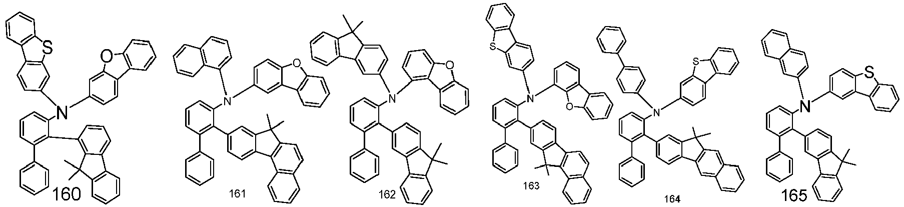

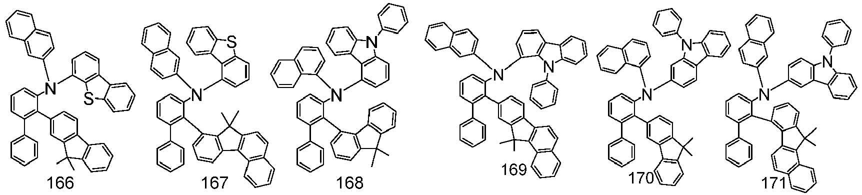

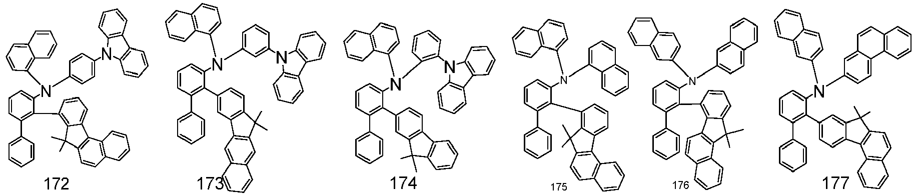

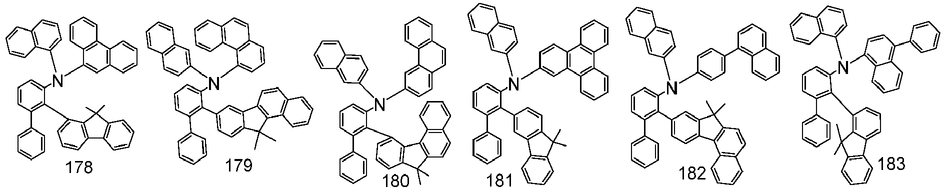

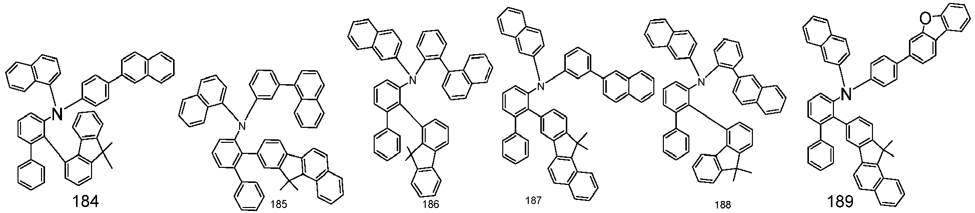

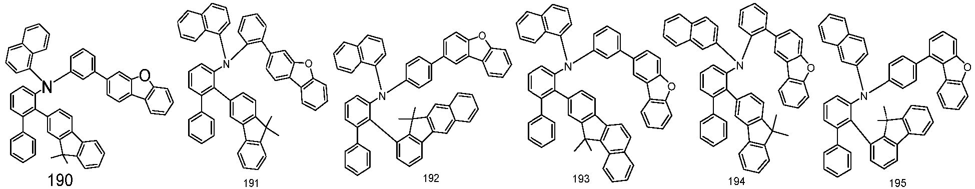

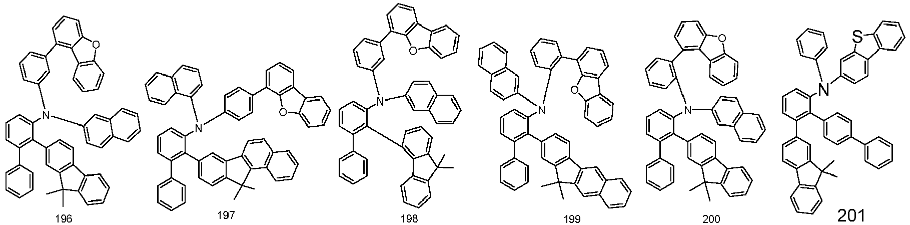

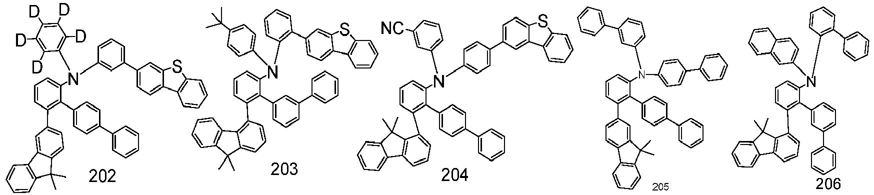

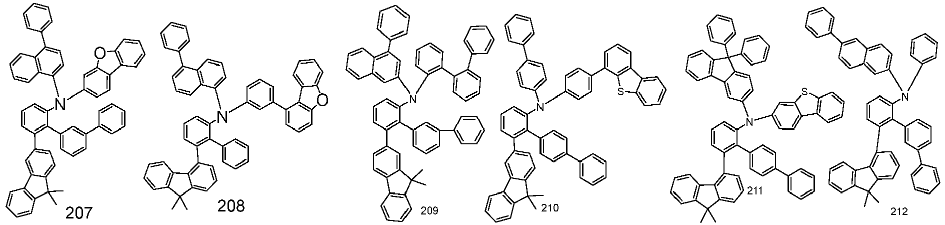

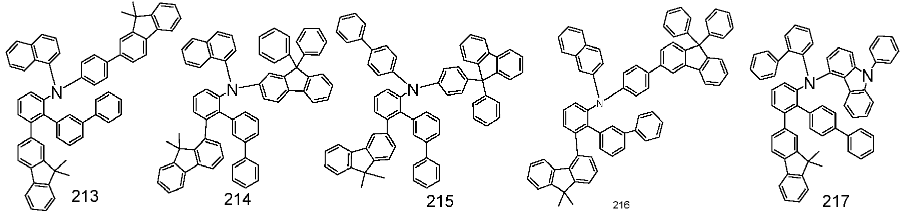

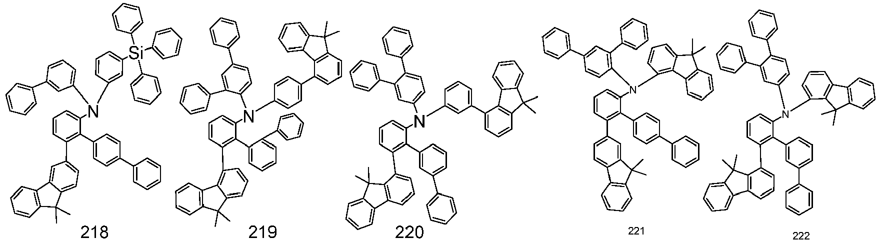

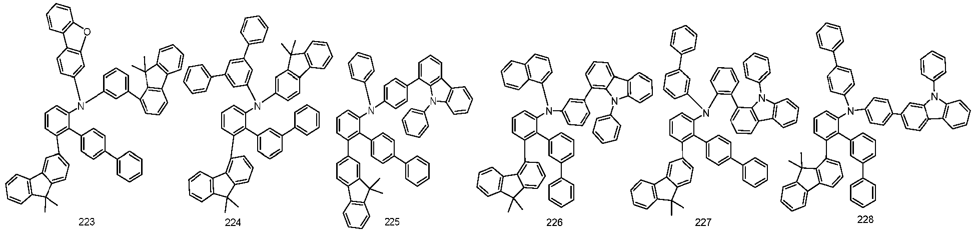

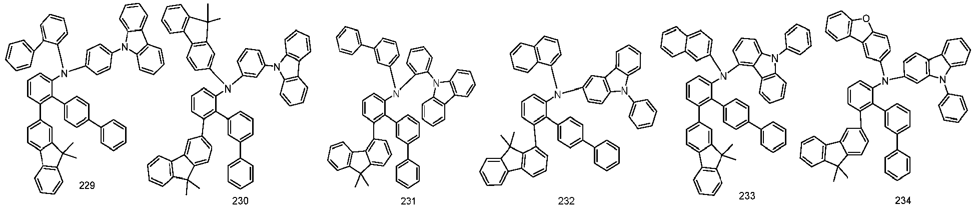

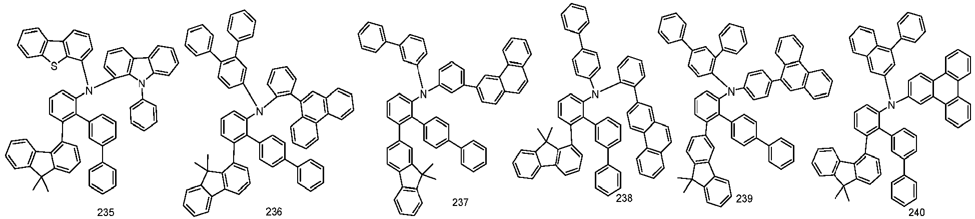

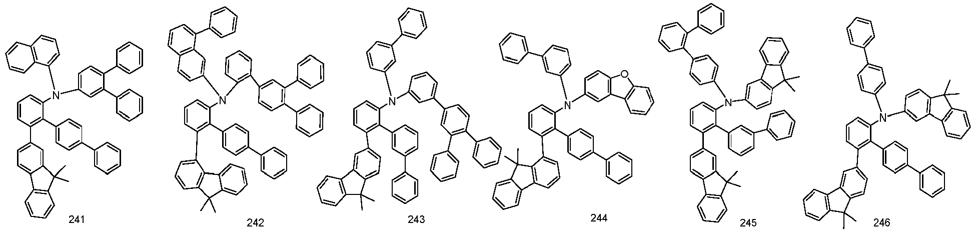

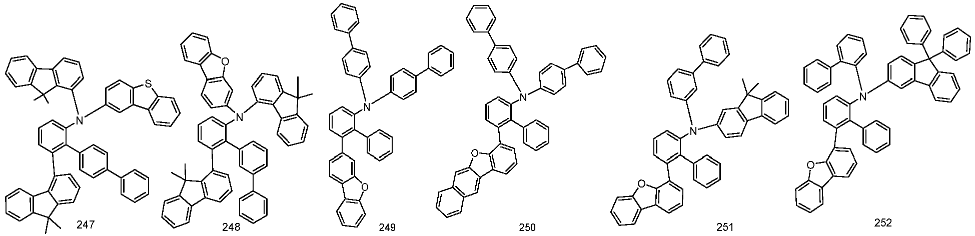

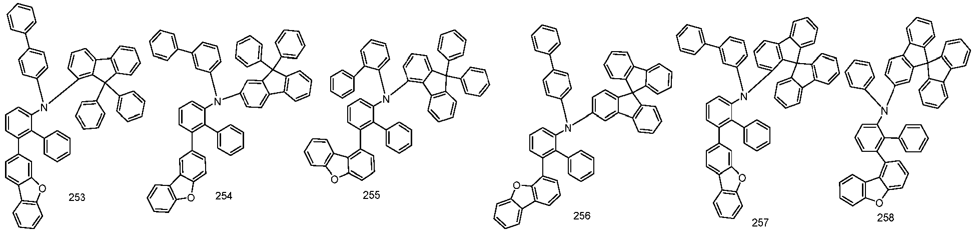

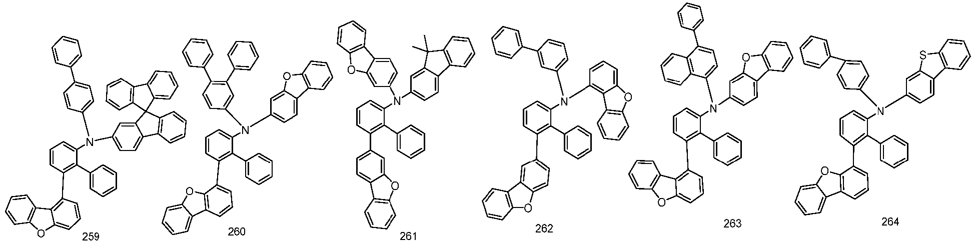

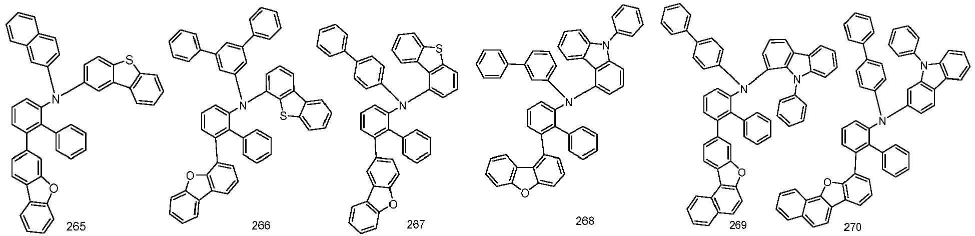

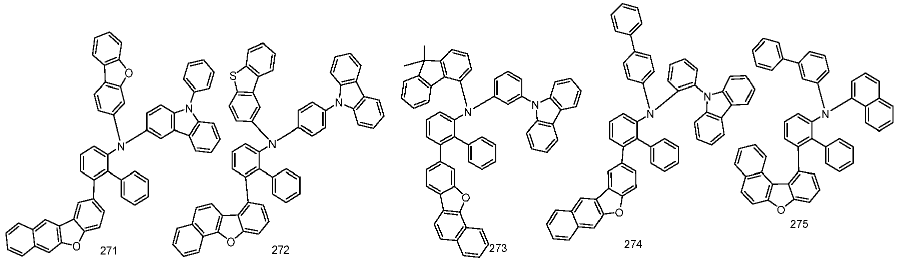

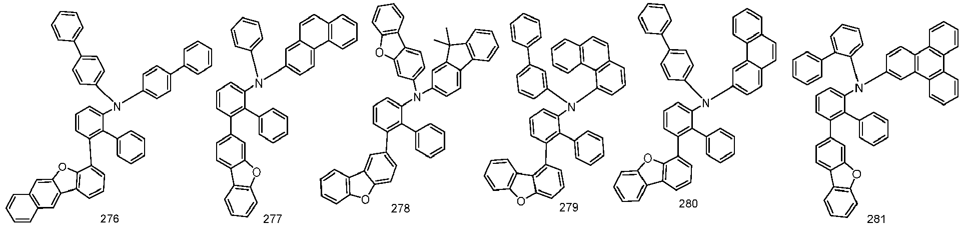

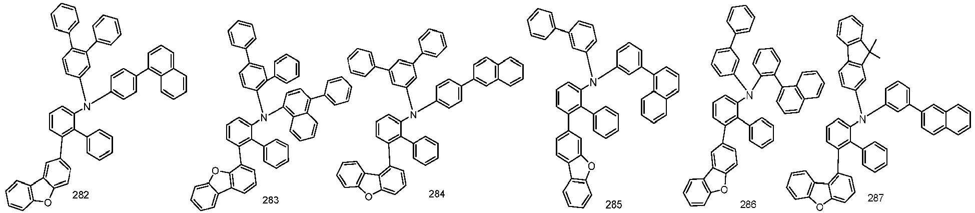

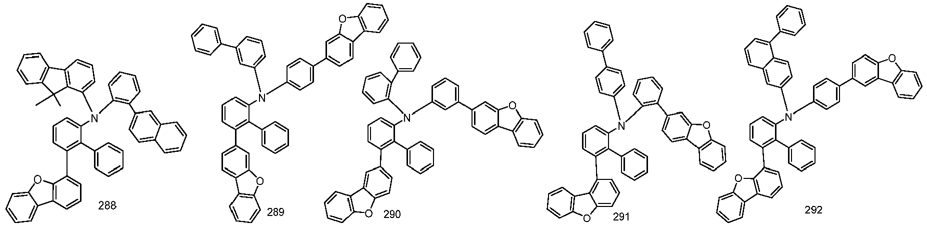

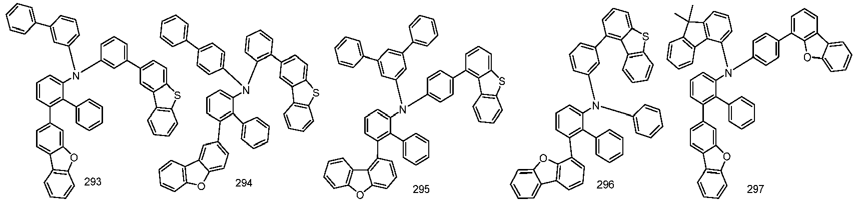

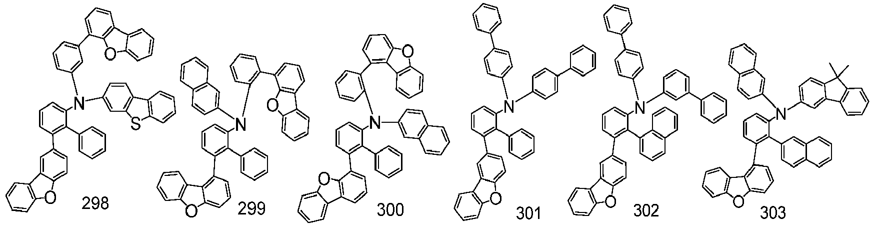

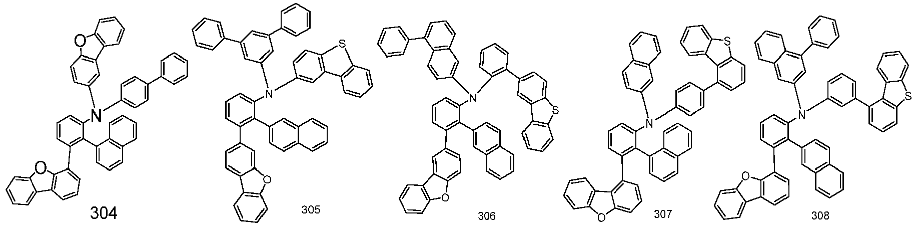

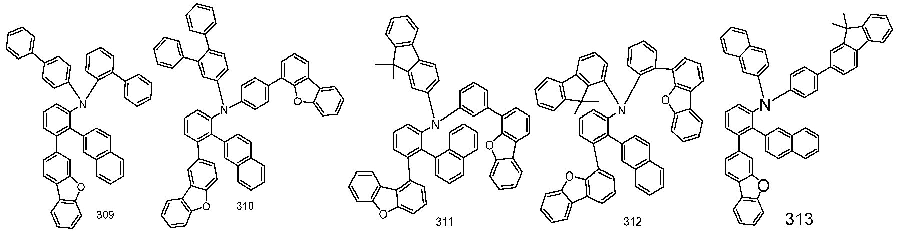

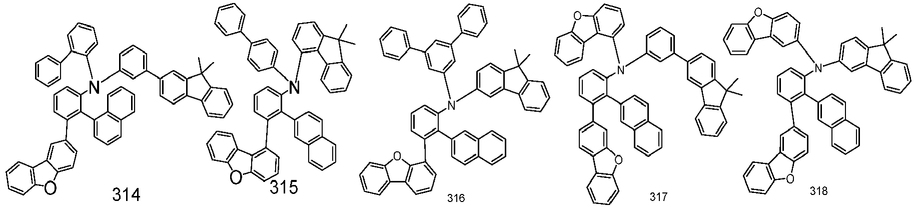

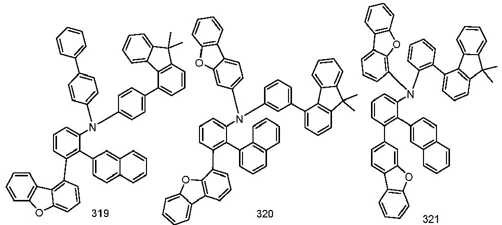

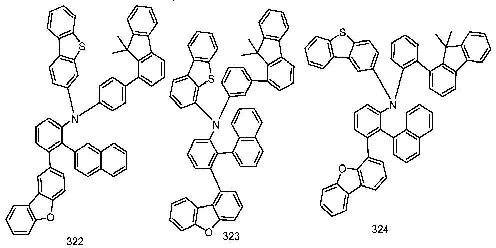

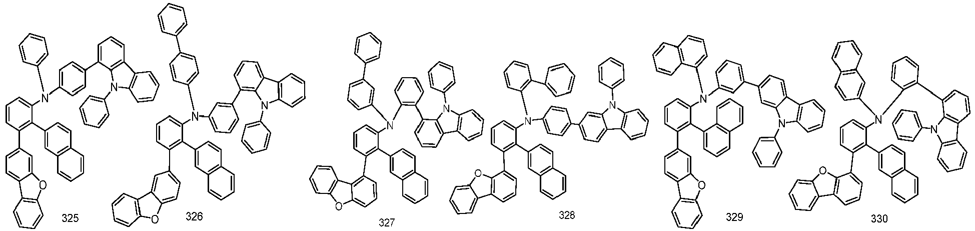

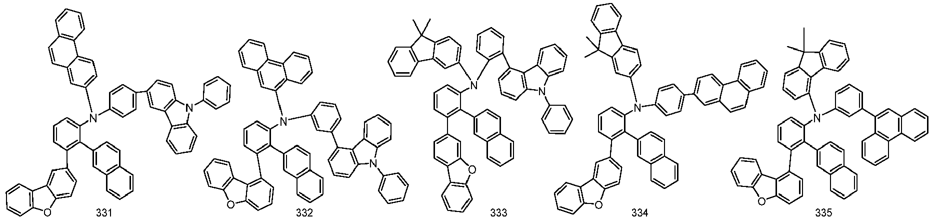

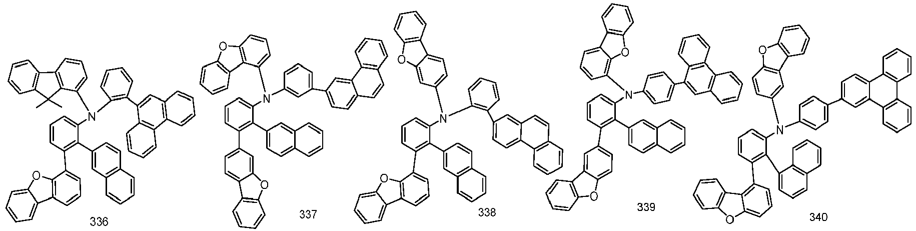

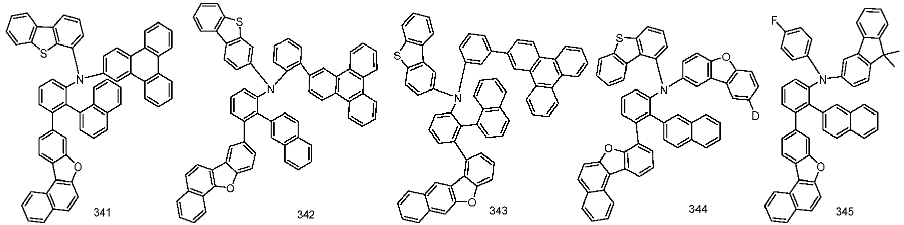

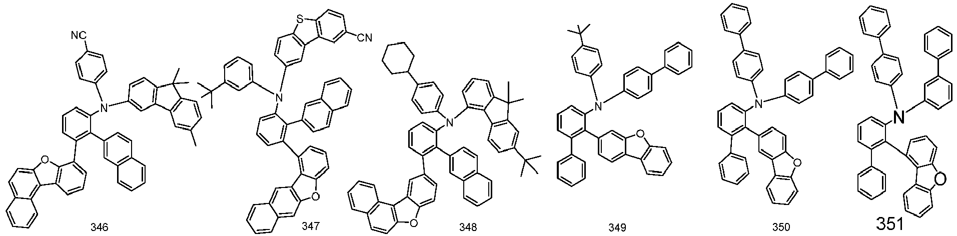

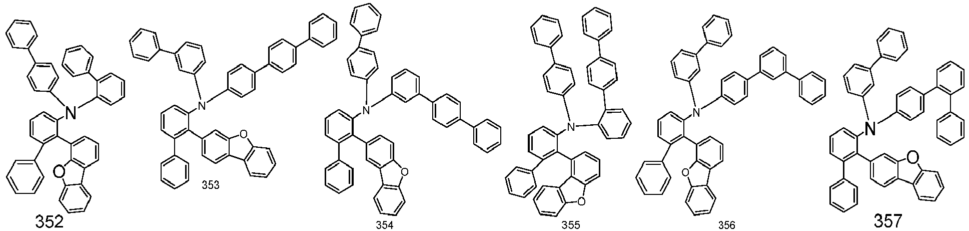

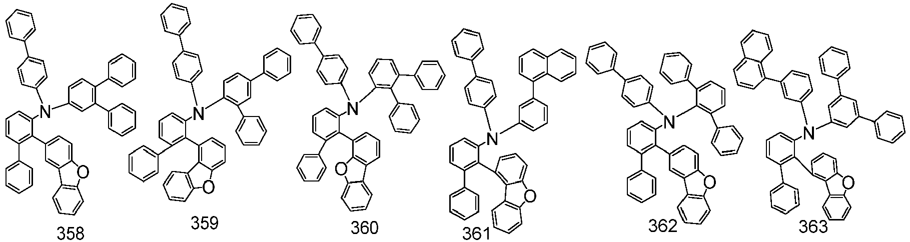

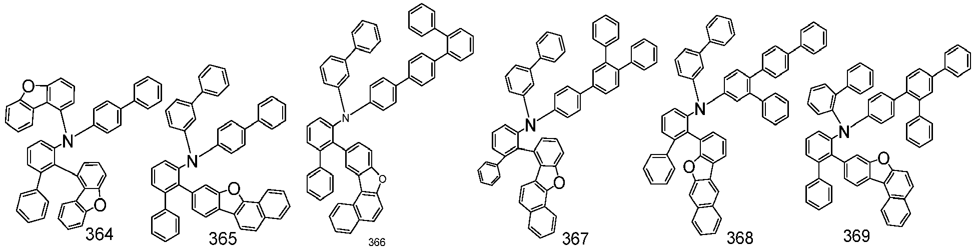

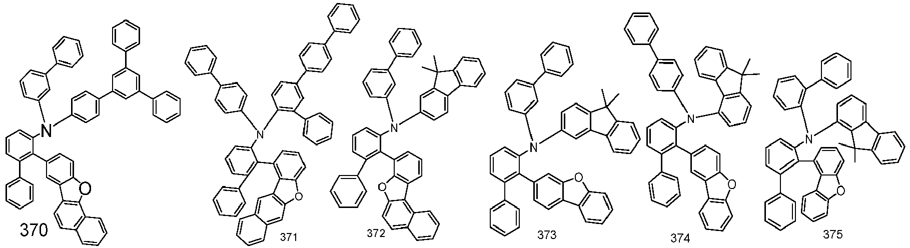

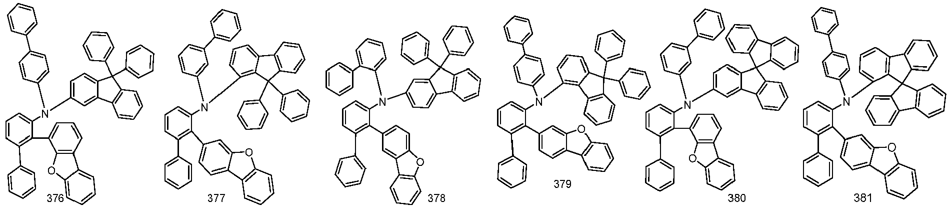

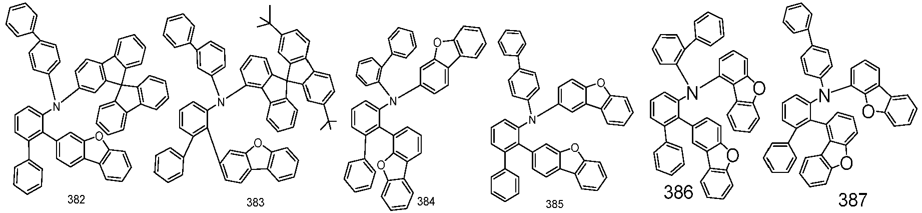

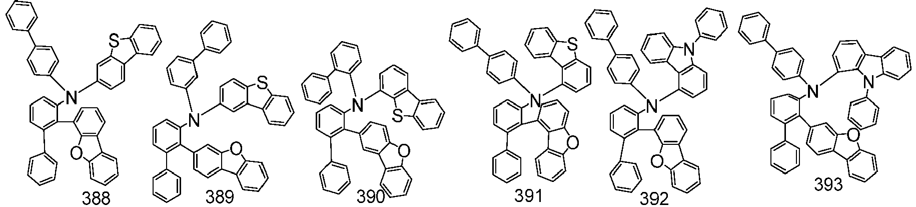

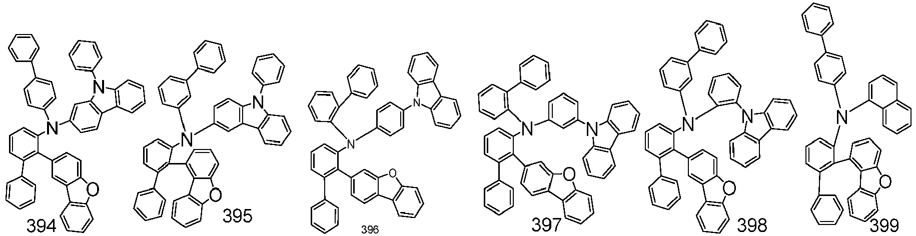

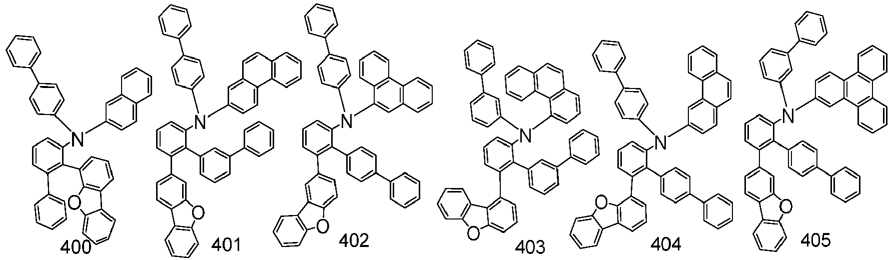

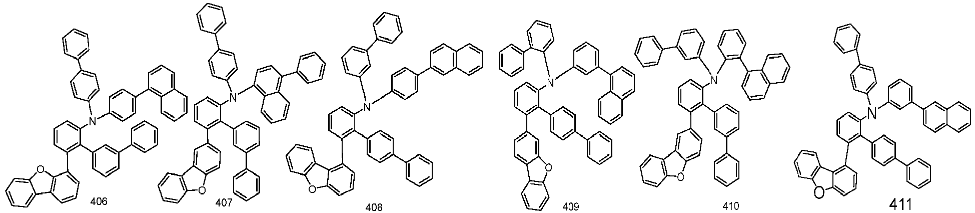

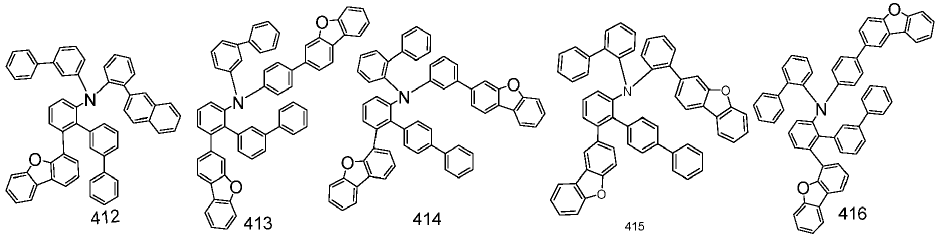

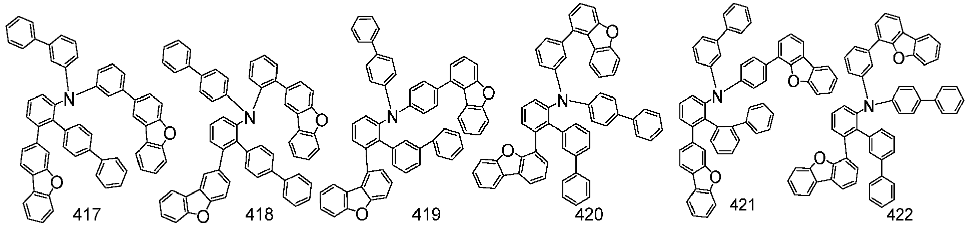

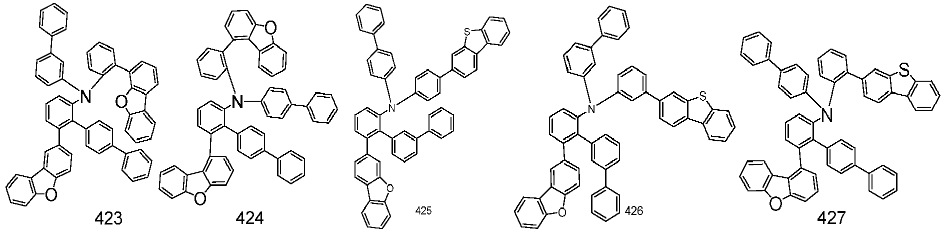

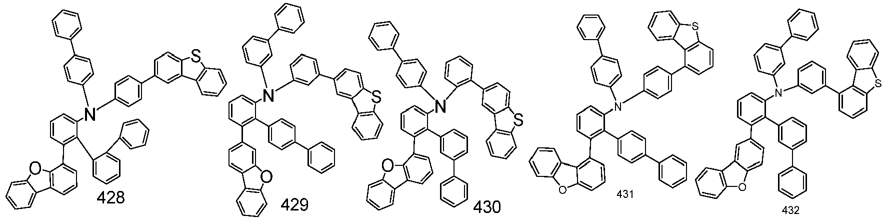

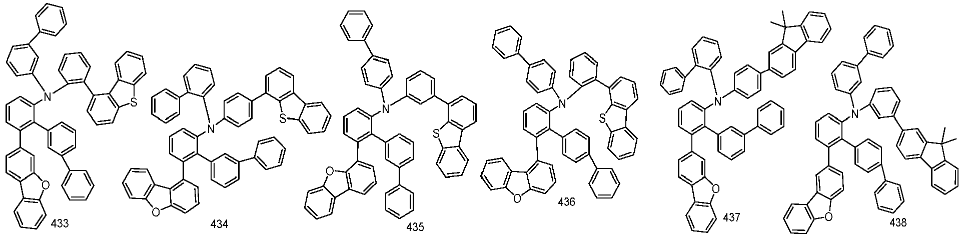

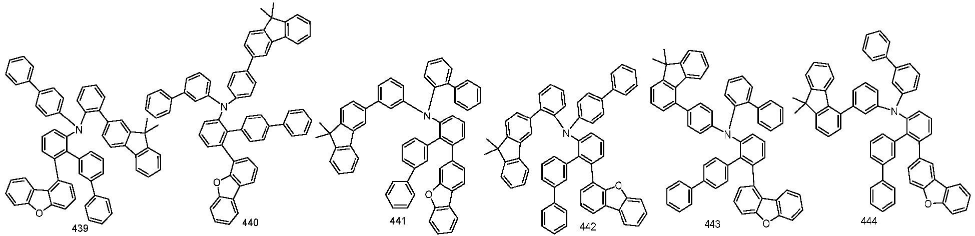

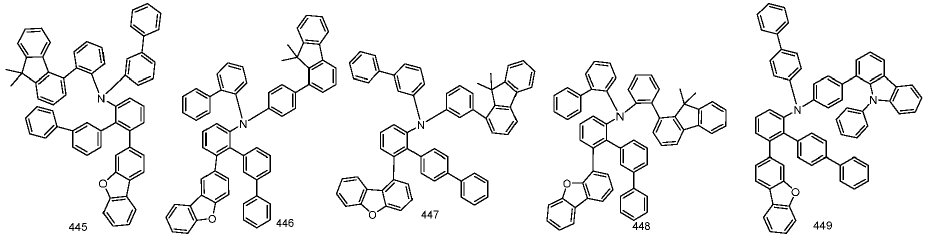

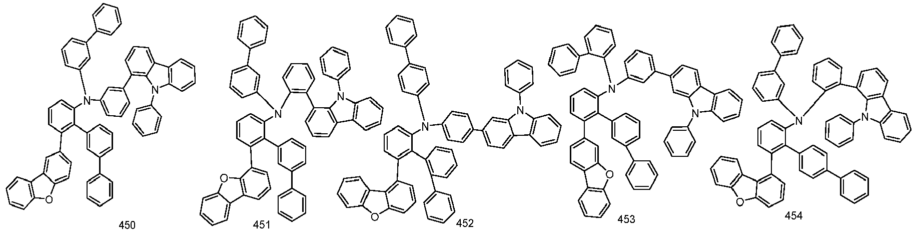

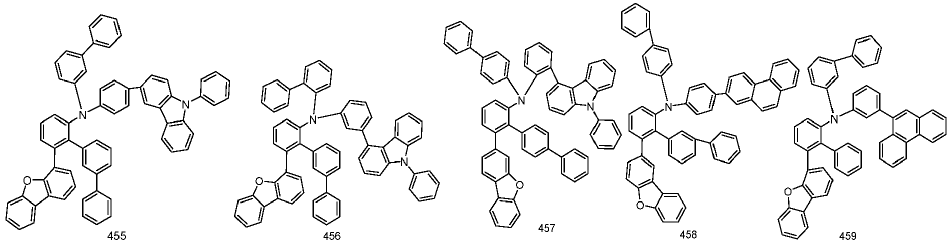

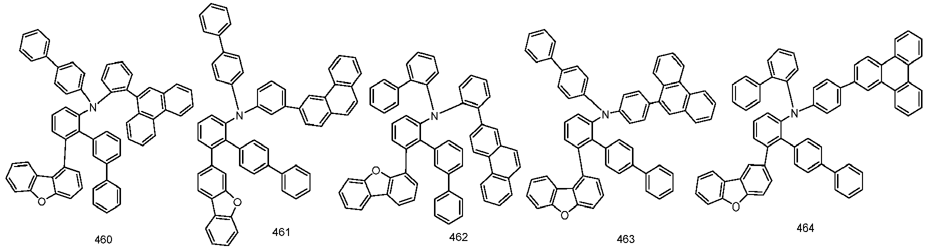

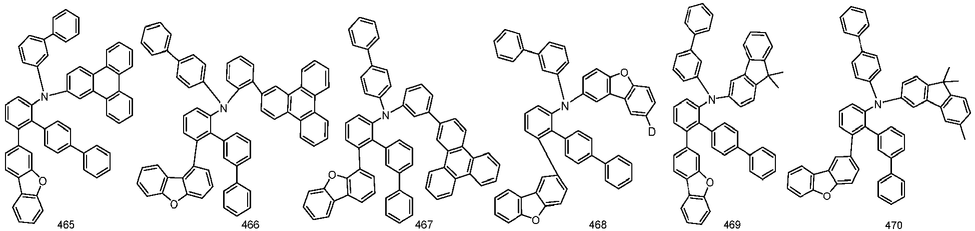

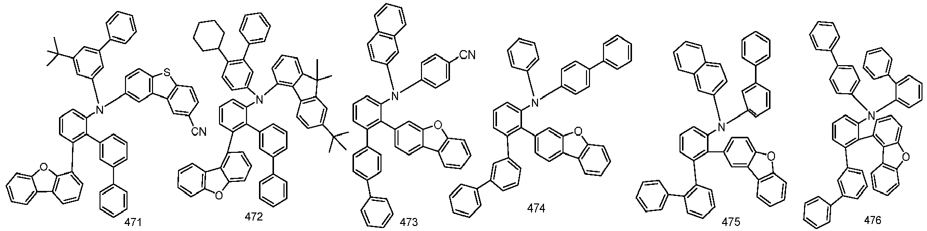

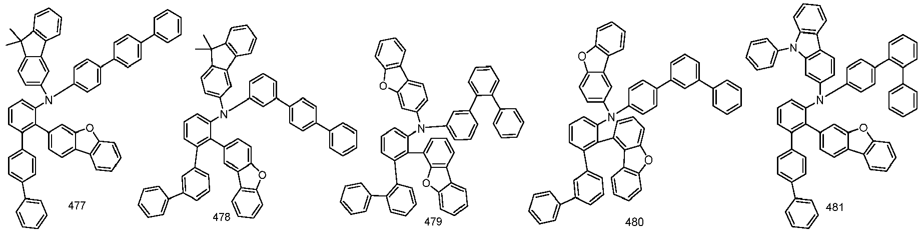

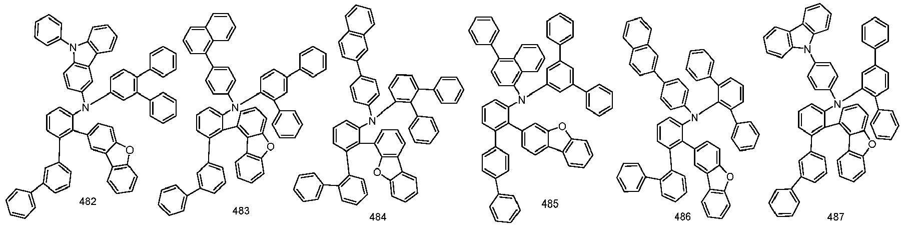

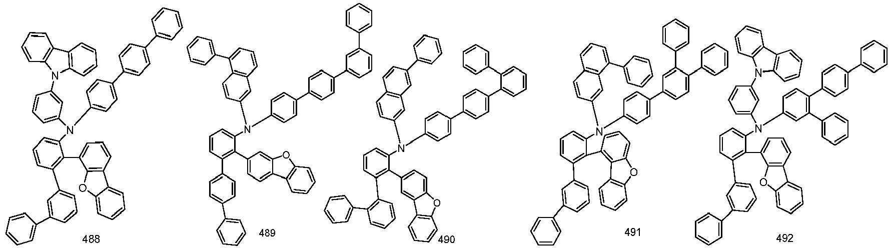

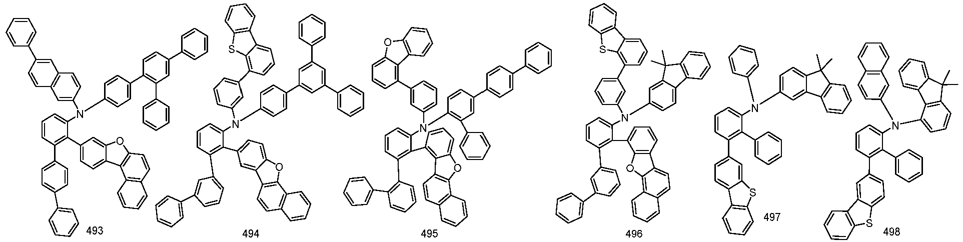

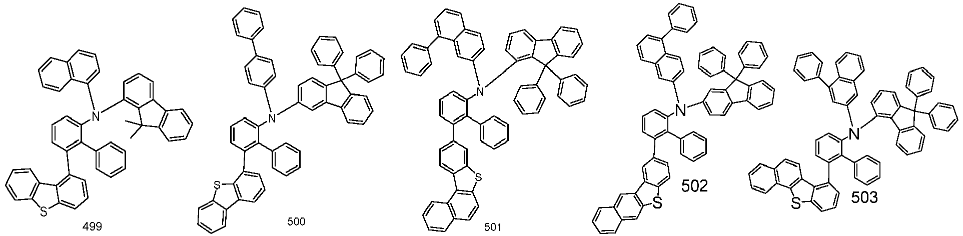

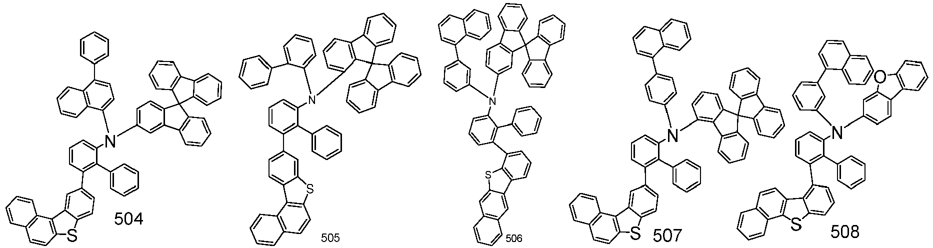

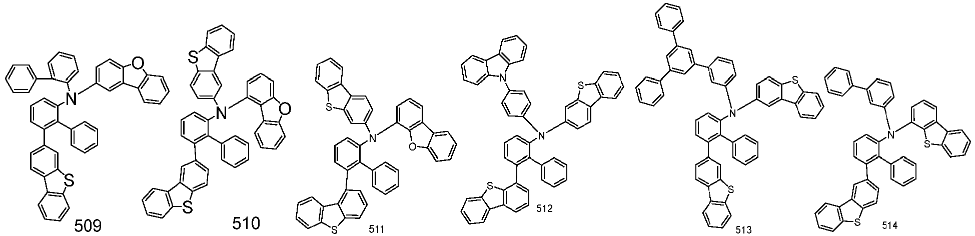

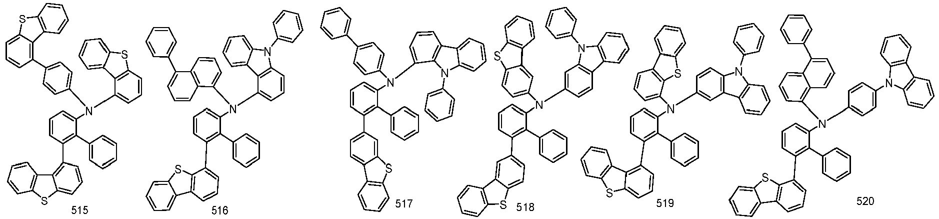

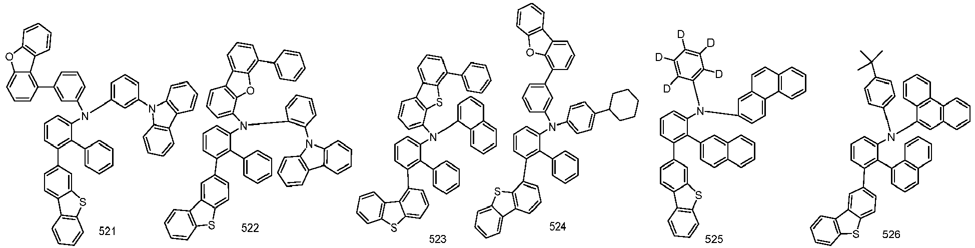

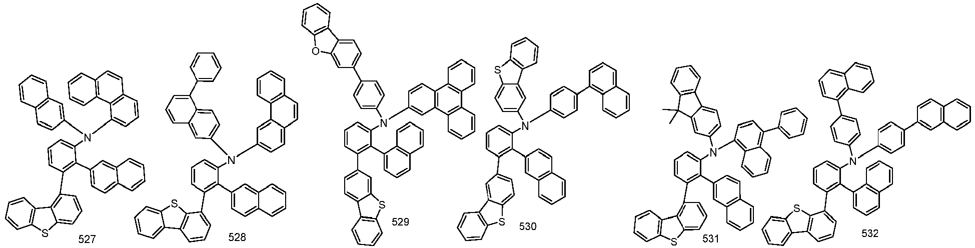

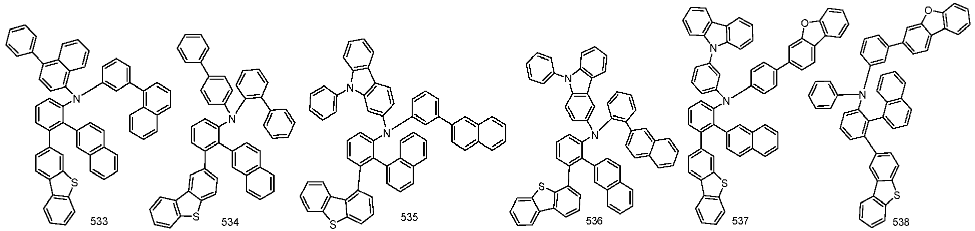

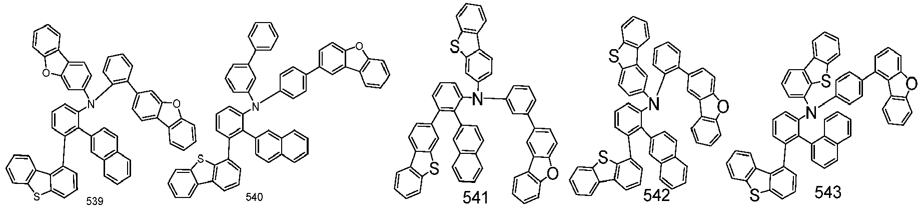

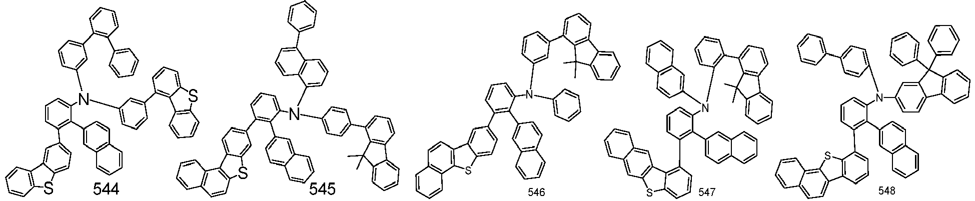

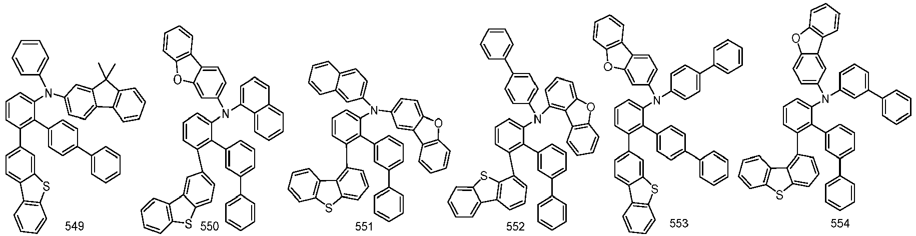

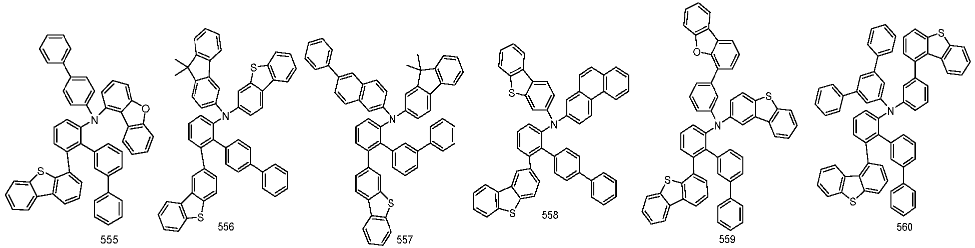

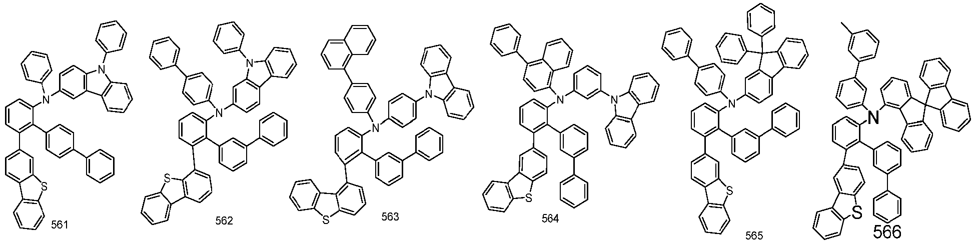

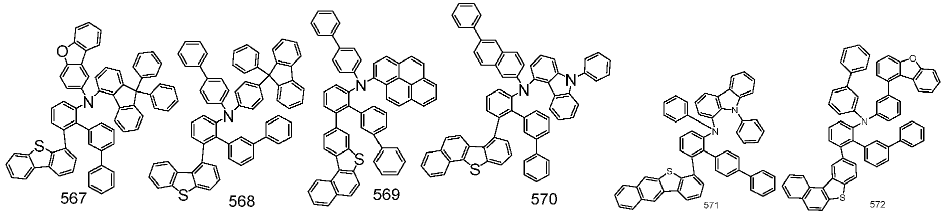

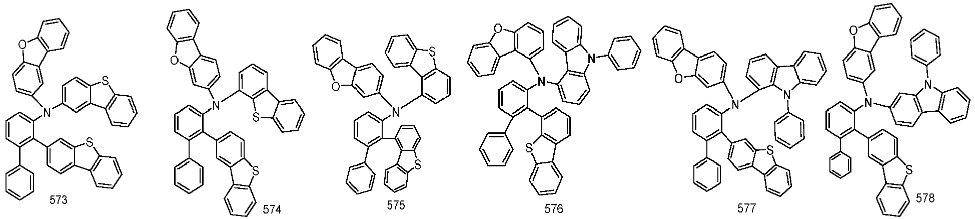

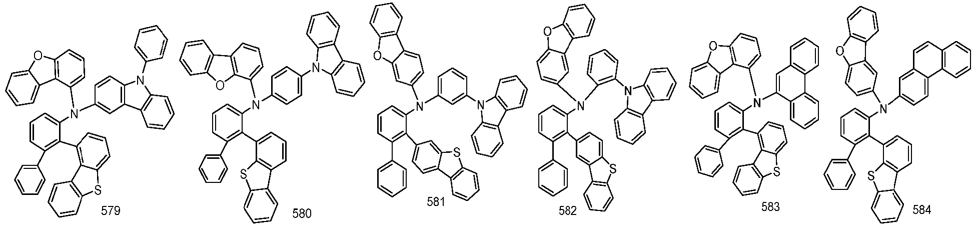

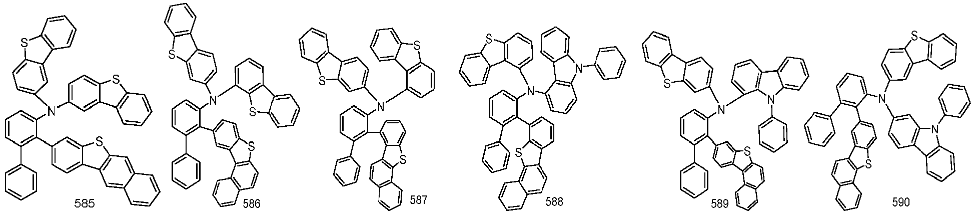

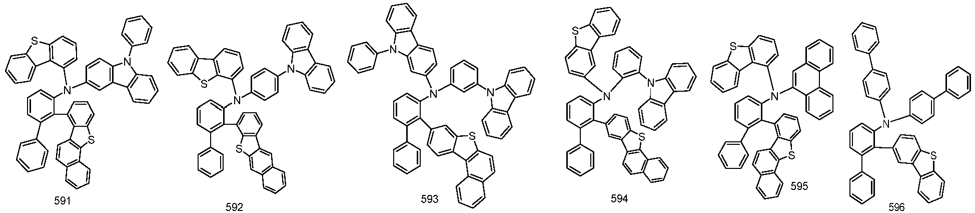

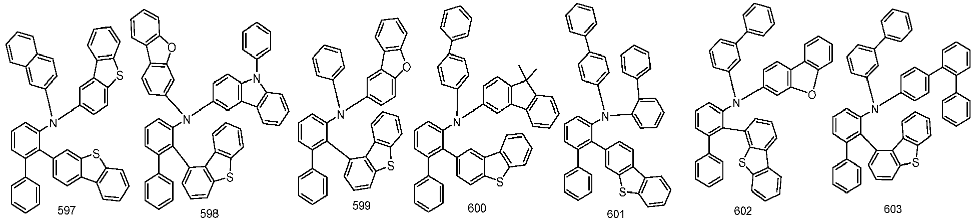

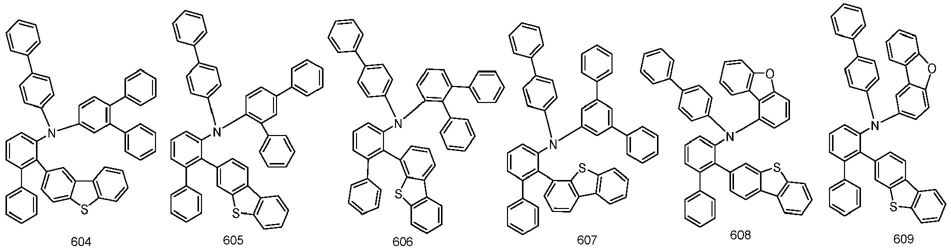

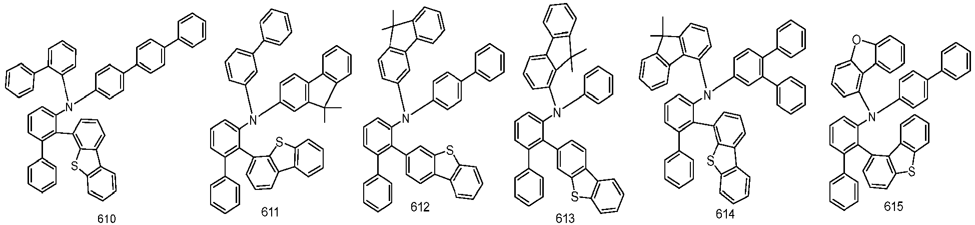

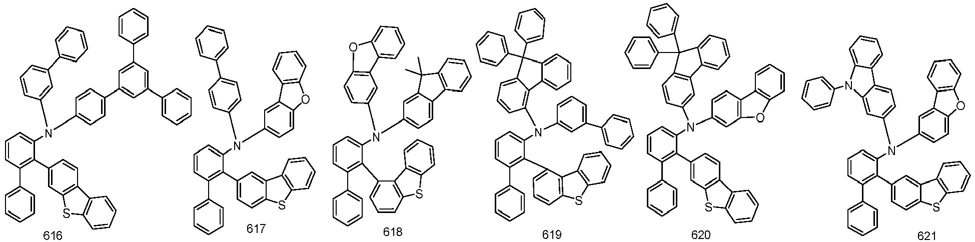

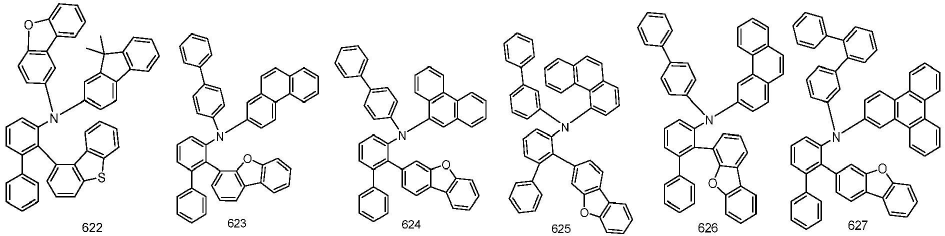

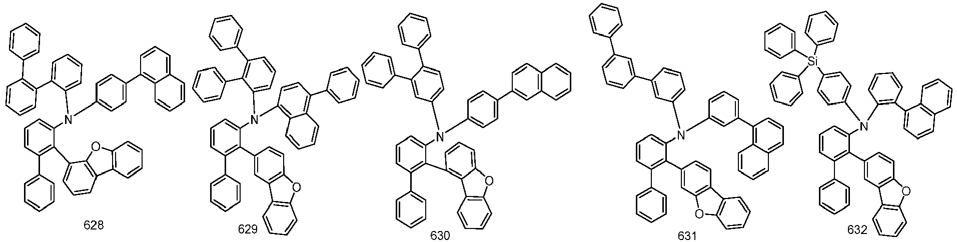

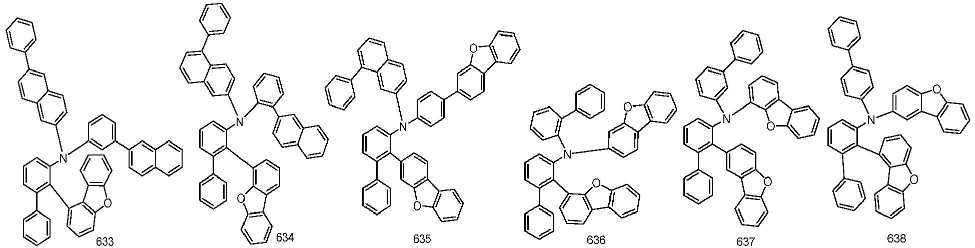

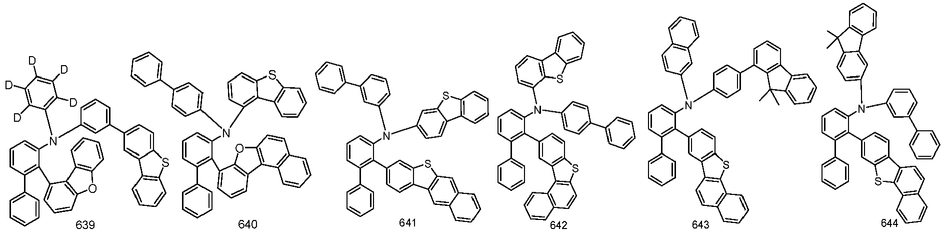

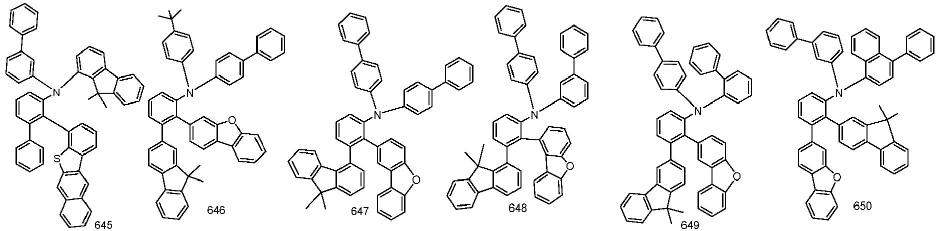

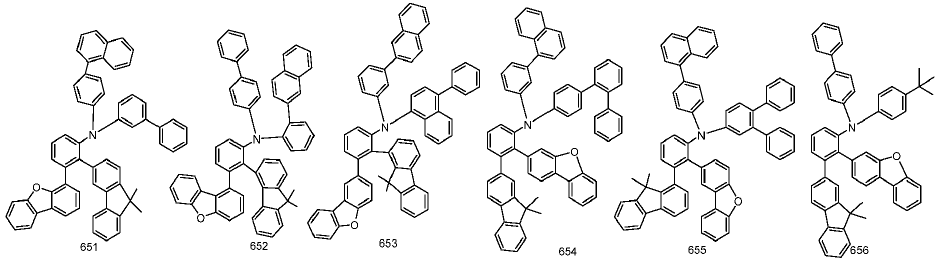

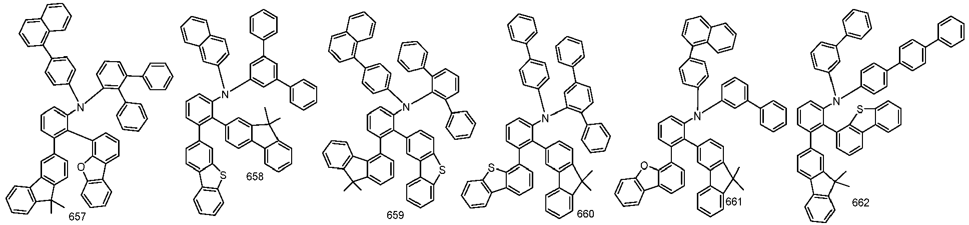

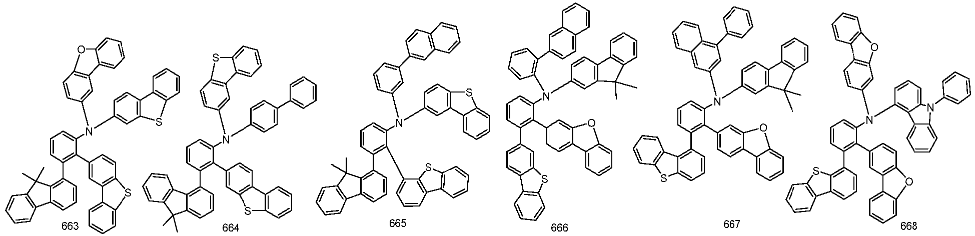

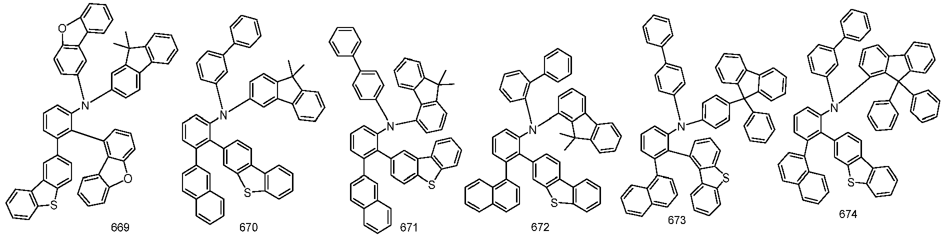

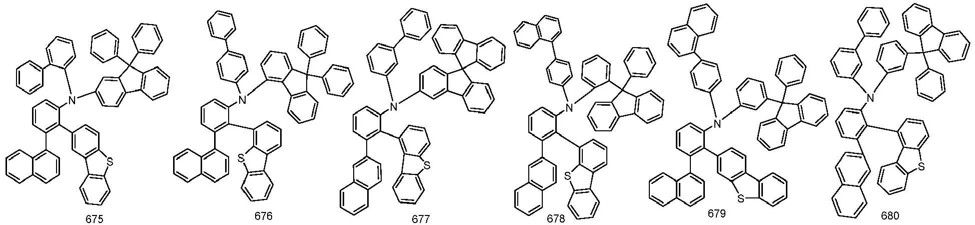

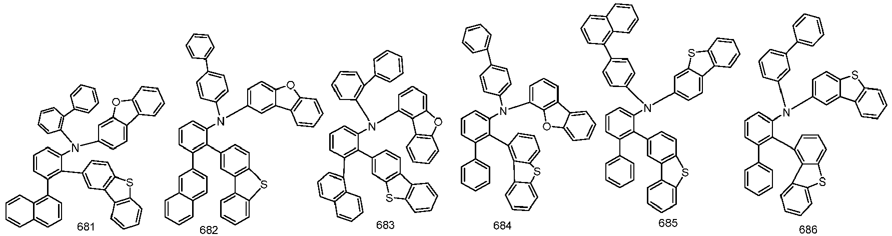

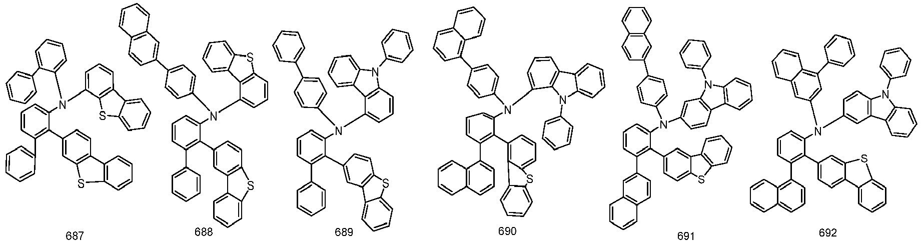

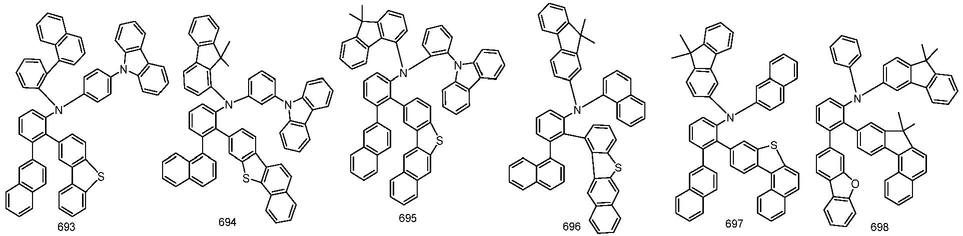

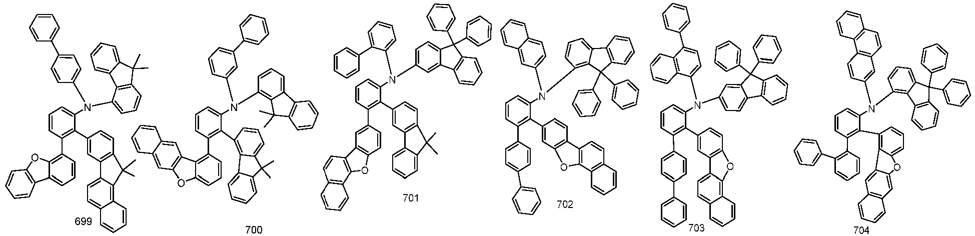

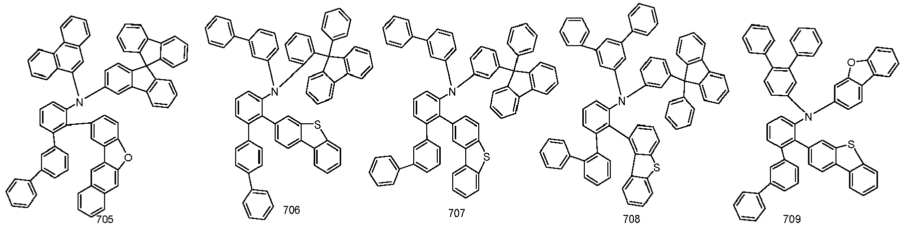

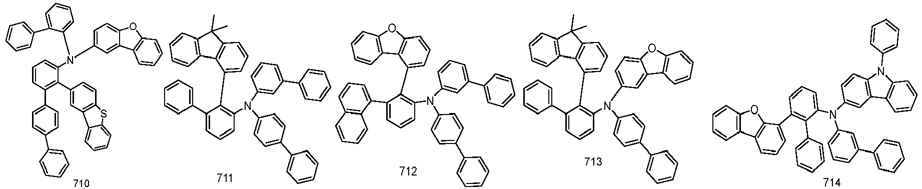

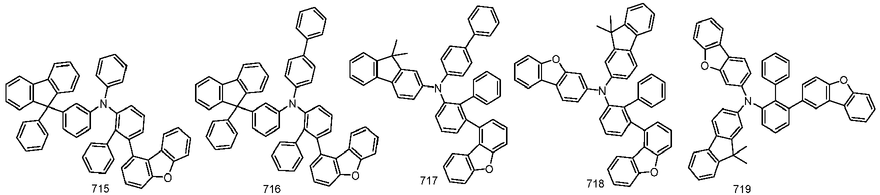

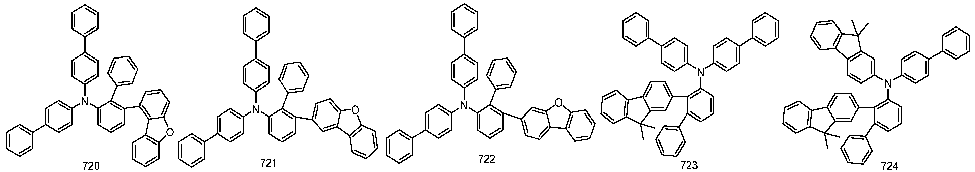

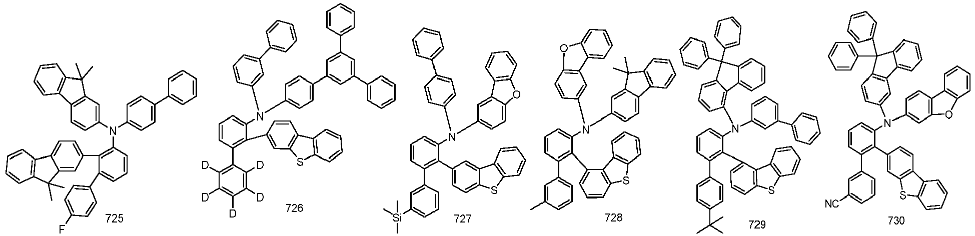

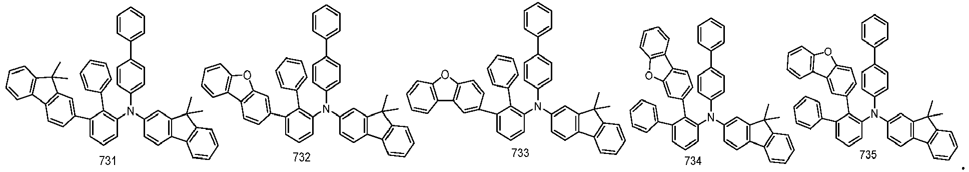

The second aspect of the present disclosure provides an electronic element, comprising an anode and a cathode which is arranged oppositely to the anode, and a functional layer arranged between the anode and the cathode; and the functional layer comprises the organic electroluminescent material of the present disclosure.

Optionally, the functional layer comprises a hole transport layer, and the hole transport layer comprises the organic electroluminescent material of the present disclosure.

Optionally, the electronic element is an organic electroluminescent device or a photoelectric conversion device.

Preferably, the electronic element is an organic electroluminescent device.

More preferably, the hole transport layer comprises the first hole transport layer and the second hole transport layer, and the first hole transport layer is closer to the anode than the second hole transport layer, wherein the second hole transport layer comprises the organic electroluminescent material of the present disclosure.

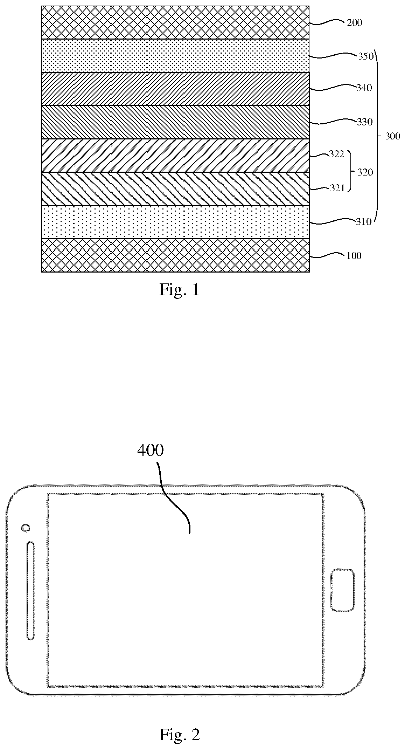

In an embodiment, the electronic element may be an organic electroluminescent device. As shown in Fig. 1, the organic electroluminescent device may comprise an anode 100, a first hole transport layer 321, a second hole transport layer 322, an organic light-emitting layer 330, an electron transport layer 340 and a cathode 200 which are stacked in sequence.

Optionally, the anode 100 comprises the following anode material, which is preferably a material with a large work function that facilitates hole injection into the functional layer. Specific examples of the anode material include: metals such as nickel, platinum, vanadium, chromium, copper, zinc and gold or alloys thereof; metal oxides such as zinc oxide, indium oxide, indium tin oxide (ITO) and indium zinc oxide (IZO); combined metals and oxides such as ZnO:Al or SnO₂:Sb; or conducting polymers such as poly(3-methylthiophene), poly[3,4-(ethylidene-1,2-dioxy)thiophene] (PEDT), polypyrrole and polyaniline, but not limited thereto. A transparent electrode containing indium tin oxide (ITO) is preferably comprised as the anode.

Optionally, the hole transport layer may comprise one or more hole transport materials, and the hole transport materials may be selected from carbazole polymers, carbazole-linked triarylamine compounds or other types of compounds, which are not particularly limited in the present disclosure. For example, the hole transport layer comprises a first hole transport layer 321 and a second hole transport layer 322.

Optionally, the first hole transport layer 321 is not limited, for example, it may be composed of a compound NPB.

Optionally, the second hole transport layer 322 comprises the organic electroluminescent material of the present disclosure.

Optionally, the organic light-emitting layer 330 may be composed of a single light-emitting layer material, or may comprise a host material and a dopant material. Optionally, the organic light-emitting layer 330 is composed of a host material and a dopant material, holes injected into the organic light-emitting layer 330 and electrons injected into the organic light-emitting layer 330 can recombine in the organic light-emitting layer 330 to form excitons, the excitons transfer energy to the host material, and the host material transfers energy to the dopant material, such that the dopant material can emit light.

The host material of the organic light-emitting layer 330 may be a metal chelate compound, a bis-styryl derivative, an aromatic amine derivative, a dibenzofuran derivative or other type of material, which is not particularly limited in the present disclosure. In an embodiment of the present disclosure, the host material of the organic light-emitting layer 330 may be RH-1 or GH-1.

The dopant material of the organic light-emitting layer 330 may be a compound having a condensed aryl ring or a derivative thereof, a compound having a heteroaryl ring or a derivative thereof, an aromatic amine derivative or other materials, which are not particularly limited in the present disclosure. In an embodiment of the present disclosure, the dopant material of the organic light-emitting layer 330 may be Ir(dmpq)₂(acac) or Ir(ppy)₃.

The electron transport layer 340 may be of a single-layer structure or a multi-layer structure, which may comprise one or more electron transport materials, and the electron transport materials may be selected from, but are not limited to, ET-1, ET-2, TPBi, LiQ, benzimidazole derivatives, oxadiazole derivatives, quinoxaline derivatives or other electron transport materials.

Optionally, a hole barrier layer (HBL) may also be provided between the organic light-emitting layer and the electron transport layer. The material of the hole barrier layer may be selected with reference to the prior art, which is not limited in the present disclosure, for example, the material may be HB-1.

In the present disclosure, the cathode 200 may comprise a cathode material, which is a material with a small work function that facilitates the injection of electrons into the functional layer. Specific examples of the cathode material include, but are not limited to, metals such as magnesium, calcium, sodium, potassium, titanium, indium, yttrium, lithium, gadolinium, aluminum, silver, tin and lead or alloys thereof; or multi-layer materials such as LiF/Al, Liq/Al, LiO₂/Al, LiF/Ca, LiF/Al and BaF₂/Ca. A metal electrode containing magnesium and silver is preferably comprised as the cathode.

Optionally, as shown in Fig. 1, a hole injection layer 310 may be further provided between the anode 100 and the first hole transport layer 321 to enhance the capability of injecting holes into the first hole transport layer 321. The hole injection layer 310 may be selected from benzidine derivatives, starburst arylamine compounds, phthalocyanine derivatives or other materials, which are not particularly limited in the present disclosure. For example, the hole injection layer 310 may be composed of HAT-CN or F4-TCNQ.

Optionally, as shown in Fig. 1, an electron injection layer 350 may be further provided between the cathode 200 and the electron transport layer 340 to enhance the capability of injecting electrons into the electron transport layer 340. The electron injection layer 350 may comprise an inorganic material such as an alkali metal sulfide and an alkali metal halide, or may comprise a complex compound of an alkali metal and an organic matter. For example, the electron injection layer 350 may comprise Yb.

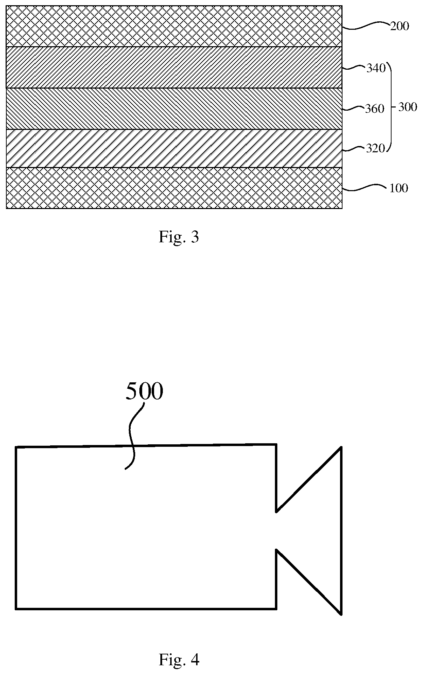

According to another embodiment, the electronic element may be a photoelectric conversion device. As shown in Fig. 3, the photoelectric conversion device may comprise an anode 100 and a cathode 200 that are arranged oppositely, and a functional layer 300 arranged between the anode 100 and the cathode 200; and the functional layer 300 comprises the organic electroluminescent material provided in the present disclosure.

According to a specific embodiment, as shown in Fig. 3, the photoelectric conversion device may comprise an anode 100, a hole transport layer 320, a photoelectric conversion layer 360, an electron transport layer 340 and a cathode 200 which are stacked in sequence.

Optionally, the photoelectric conversion device may be a solar cell, especially an organic thin film solar cell. For example, in an embodiment of the present disclosure, the solar cell may comprise an anode, a hole transport layer, an organic light-emitting layer, an electron transport layer and a cathode which are stacked in sequence, wherein the hole transport layer includes the organic electroluminescent material of the present disclosure.

The third aspect of the present disclosure provides an electronic device, comprising the electronic element provided in the second aspect of the present disclosure.

According to an embodiment, as shown in Fig. 2, the electronic device is a first electronic device 400 including the above-mentioned organic electroluminescent device. The first electronic device 400 may be, for example, a display apparatus, a lighting apparatus, an optical communication apparatus or other types of electronic devices, such as but not limited to a computer screen, a mobile phone screen, a television, electronic paper, an emergency lamp, and an optical module.

According to another embodiment, as shown in Fig. 4, the electronic device is a second electronic device 500 including the above-mentioned photoelectric conversion device. The second electronic device 500 may be, for example, a solar power generation device, a photodetector, a fingerprint identification device, an optical module, a CCD camera, or other types of electronic devices.

The synthesis method of the organic electroluminescent material of the present disclosure will be specifically described below with reference to examples of synthesis, but the present disclosure is not limited thereby.

The compounds in the synthetic methods that are not mentioned in the present disclosure are all raw materials obtained through commercial channels.

### Examples of synthesis

### 1. Synthesis of intermediates

### (1) Synthesis of intermediate IM a-1:

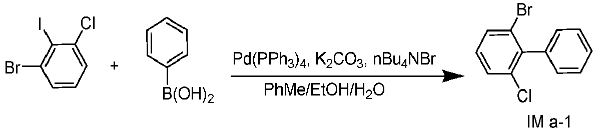

Nitrogen (0.100 L/min) was introduced into a three-necked flask equipped with a mechanical stirrer, a thermometer and a spherical condenser for displacement 15 min, 1-bromo-2-iodo-3-chlorobenzene (80.0 g, 252 mmol), phenylboronic acid (31.04 g, 254.52 mmol), tetrakis(triphenylphosphine)palladium (6.9 g, 1.3 mmol), potassium carbonate (86.97 g, 630.22 mmol) and tetrabutylammonium bromide (8.1 g, 25.2 mmol) were added successively, and a mixed solvent of toluene (800 mL), ethanol (150 mL) and water (150 mL) was added. Stirring was initiated, followed by heating to 78 to 80°C, reacting for 72 h, and then cooling to room temperature. An organic phase was separated out after the reaction solution was washed with water, the organic phase was dried with anhydrous magnesium sulfate, and after filtration, the filtrate was distilled under reduced pressure to remove the solvent. The crude product was purified by silica gel column chromatography using a dichloromethane/n-heptane system to obtain a white solid IM a-1 (37.78 g, yield 56%).

The IM x-1 listed in Table 1 was synthesized with reference to the method for the IM a-1, except that raw material 1 was used instead of the phenylboronic acid. The main raw materials used, the synthesized intermediates and the yields thereof were as shown in Table 1.

**Table 1**

| Raw material 1 | IM x-1 | Yield/% |
|---|---|---|
| 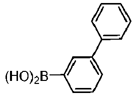 | 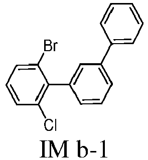 | 60 |
| 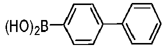 | 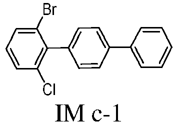 | 65 |
| 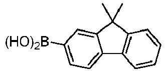 | 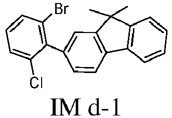 | 55 |
| 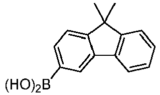 | 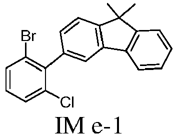 | 53 |
| 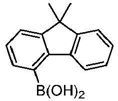 | 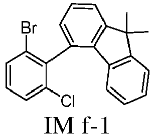 | 60 |
| 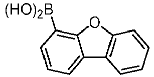 | 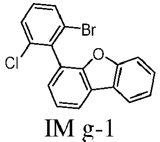 | 59 |
| 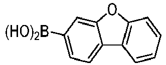 | 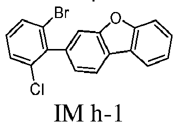 | 53 |
| 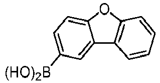 | 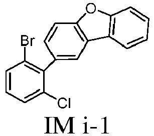 | 58 |
| 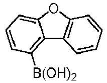 | 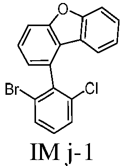 | 57 |
| 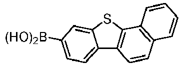 | 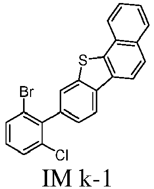 | 55 |
| 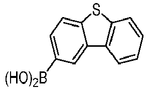 | 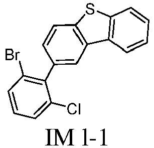 | 52 |
| 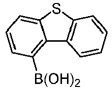 | 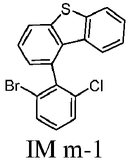 | 51 |
| 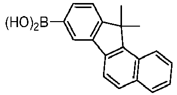 | 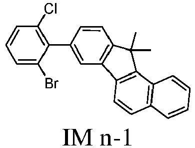 | 54 |
| 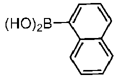 | 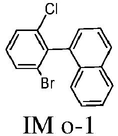 | 43 |
| 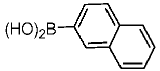 | 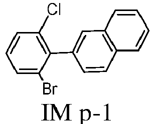 | 45 |

### (2) Synthesis of intermediate IM a-2

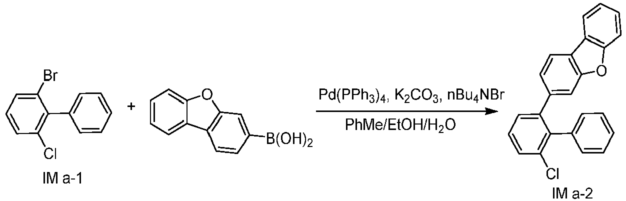

Nitrogen (0.100 L/min) was introduced into a three-necked flask equipped with a mechanical stirrer, a thermometer and a constant pressure drop funnel for displacement 15 min, the IM a-1 (20.0 g, 74.75 mmol), dibenzofuran-3-boronic acid (15.85 g, 74.75 mmol), tetrakis(triphenylphosphine)palladium (0.44 g, 0.37 mmol), potassium carbonate (25.79 g, 186.9 mmol) and tetrabutylammonium bromide (2.4 g, 7.5 mmol) were added successively, and a mixed solvent of toluene (200 mL), ethanol (40 mL) and water (40 mL) was added. Stirring was initiated, followed by heating to 78 to 80°C, reacting for 63 h, and then cooling to room temperature. An organic phase was separated after the reaction solution was washed with water, the organic phase was dried with anhydrous magnesium sulfate, and after filtration, the filtrate was distilled under reduced pressure to remove the solvent. The crude product was purified by silica gel column chromatography using a dichloromethane/n-heptane system to obtain a white solid IM a-2 (15.91 g, yield 60%).

The intermediates listed in Table 2 were synthesized with reference to the method for the IM a-2, except that IM x-1 in Table 1 was used instead of IM a-1, and raw material 2 was used instead of dibenzofuran-3-boronic acid. The main raw materials used, the synthesized intermediates and the yields thereof were as shown in Table 2.

**Table 2**

| Intermediate IM x-1 | Raw material 2 | Synthesized intermediate | Yield/% |
|---|---|---|---|
| 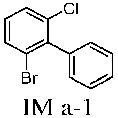 | 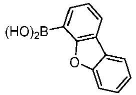 | 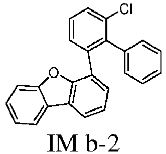 | 55 |
| | 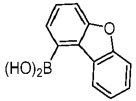 | 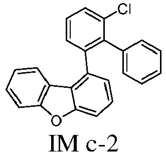 | 59 |
| | 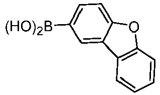 | 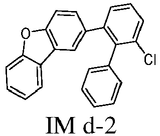 | 56 |
| | 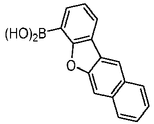 | 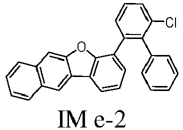 | 51 |
| | 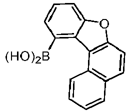 | 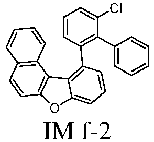 | 48 |
| | 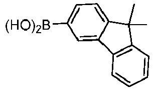 | 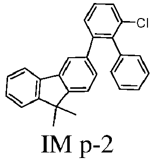 | 50 |
| | 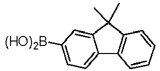 | 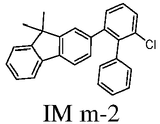 | 62 |
| | 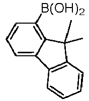 | 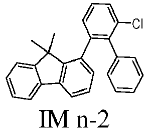 | 61 |
| | 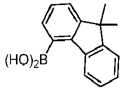 | 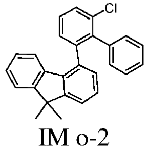 | 63 |
| | 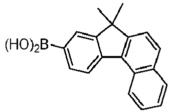 | 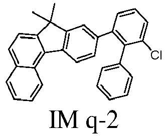 | 58 |
| | 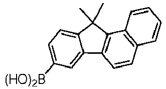 | 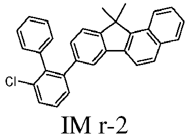 | 54 |
| | 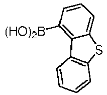 | 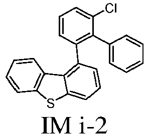 | 62 |
| | 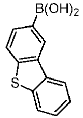 | 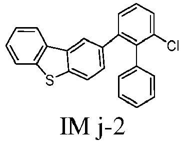 | 53 |
| | 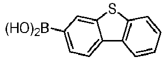 | 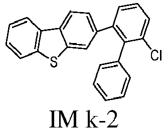 | 57 |
| | 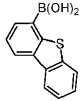 | 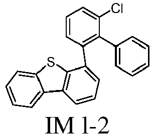 | 55 |
| 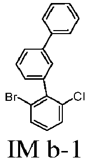 | 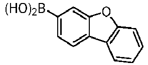 | 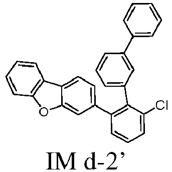 | 58 |
| 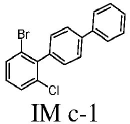 | 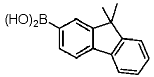 | 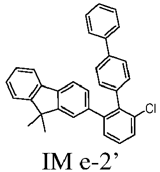 | 56 |
| | 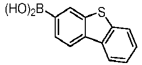 | 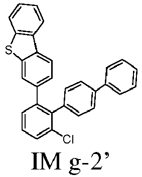 | 50 |
| 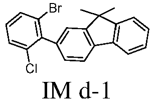 | 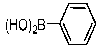 | 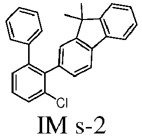 | 58 |
| 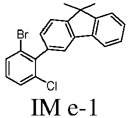 | 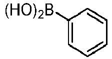 | 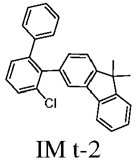 | 52 |
| | 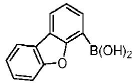 | 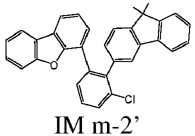 | 51 |
| 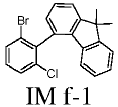 | 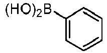 | 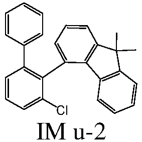 | 50 |
| 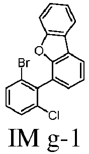 | 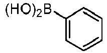 | 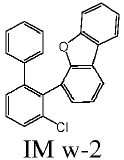 | 41 |
| 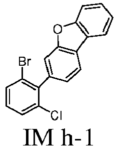 | 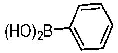 | 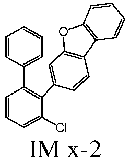 | 58 |
| | 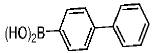 | 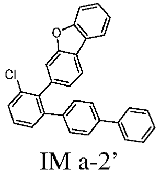 | 55 |
| | 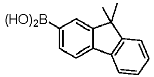 | 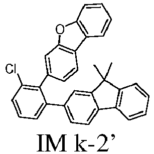 | 45 |
| 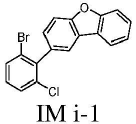 | 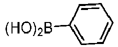 | 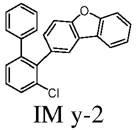 | 51 |
| | 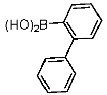 | 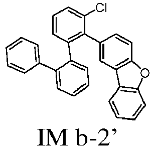 | 50 |
| 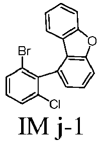 | 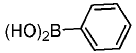 | 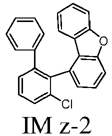 | 46 |
| | 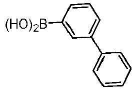 | 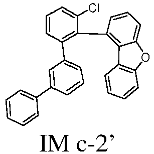 | 43 |
| 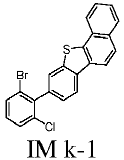 | 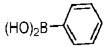 | 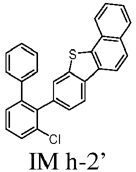 | 53 |
| 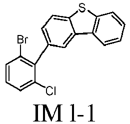 | 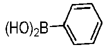 | 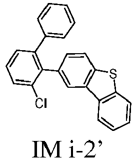 | 51 |
| 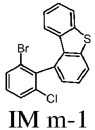 | 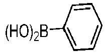 | 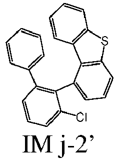 | 43 |
| 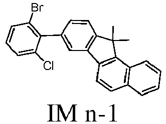 | 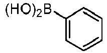 | 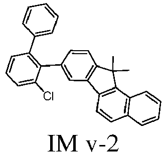 | 52 |
| 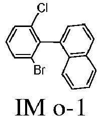 | 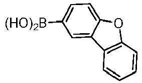 | 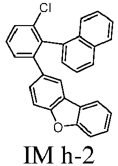 | 40 |
| 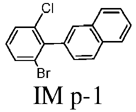 | 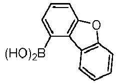 | 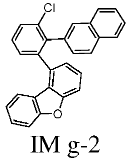 | 46 |
| | 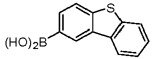 | 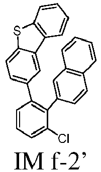 | 46 |

### 2. Synthesis of compounds

### (1) Synthesis of intermediate IM 1-1

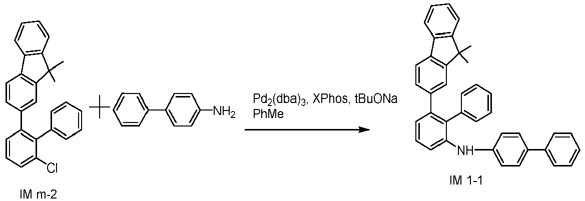

Nitrogen (0.200 L/min) was introduced into a three-necked flask equipped with a mechanical stirrer, a thermometer and a spherical condenser for displacement 15 min, and the IM m-2 (15 g, 39.37 mmol), 4-aminobiphenyl (6.66 g, 39.37 mmol), tris(dibenzalacetone)dipalladium (0.18 g, 0.2 mmol), 2-dicyclohexylphosphine-2',4',6'-triisopropylbiphenyl (0.2 g, 0.4 mmol), sodium tert-butoxide (5.64 g, 58.76 mmol) and toluene (110 mL) were added successively, followed by reflux reaction at 105 to 110°C for 4 h and then cooled to room temperature. After the organic phase was washed with water, anhydrous magnesium sulfate was added to dry the organic phase. After filtration, the filtrate was distilled under reduced pressure to remove the solvent. The crude product was purified by silica gel column chromatography using a dichloromethane/n-heptane system to obtain a white solid IM 1-1 (15.17 g, yield 75%).

### (2) Synthesis of compound 1

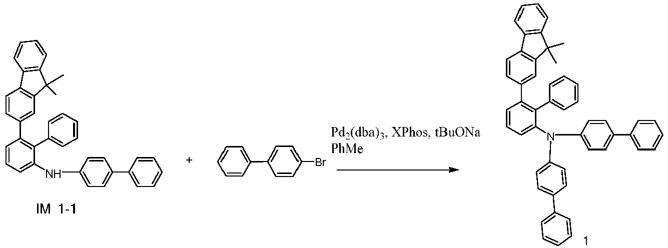

Nitrogen (0.100 L/min) was introduced into a three-necked flask equipped with a mechanical stirrer, a thermometer and a spherical condenser for displacement 15 min, and the IM 1-1 (10 g, 19.47 mmol), 4-bromobiphenyl (4.54 g, 19.47 mmol), tris(dibenzalacetone)dipalladium (0.36 g, 0.39 mmol), 2-dicyclohexylphosphine-2',4',6'-triisopropylbiphenyl (0.32 g, 0.78 mmol), sodium tert-butoxide (4.11 g, 42.83 mmol) and toluene (100 mL) were added successively, followed by reflux reaction at 105 to 110°C for 30 h and then cooed to room temperature. After the organic phase was washed with water, the solution was separated, and anhydrous magnesium sulfate was added to dry the organic phase. After filtration, the filtrate was distilled under reduced pressure to remove the solvent. The crude product was purified by silica gel column chromatography using a dichloromethane/n-heptane system to obtain a white solid compound 1 (4.54 g, yield 35%). Mass spectrum (m/z) = 666.3 [M+H]⁺.

The compounds listed in Table 3 were synthesized with reference to the method for the compound 1, except that raw material 3 was used instead of IM m-2, raw material 4 was used instead of 4-aminobiphenyl, and raw material 5 was used instead of 4-bromobiphenyl. The main raw materials used, synthesized compounds and their final step yields and mass spectra were shown in Table 3:

**Table 3**

| Raw material 3 | Raw material 4 | Raw material 5 | Compound | Yield/ % | Mass spectrum (m/z)/ [M+H]⁺ |
|---|---|---|---|---|---|
| 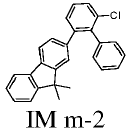 | 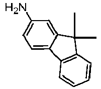 | 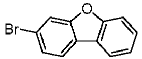 | 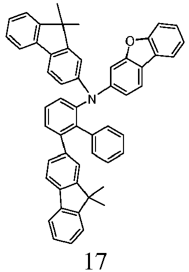 | 38 | 720.3 |
| | 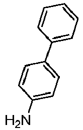 | 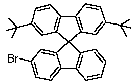 | 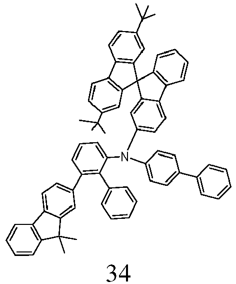 | 40 | 940.5 |
| | 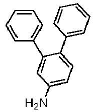 | 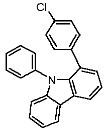 | 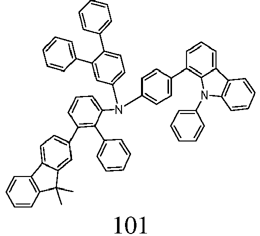 | 36 | 907.4 |
| 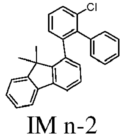 | 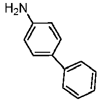 | 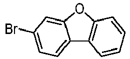 | 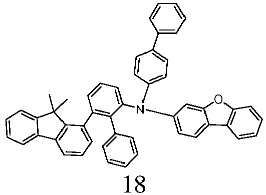 | 43 | 680.3 |
| 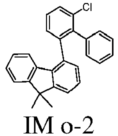 | 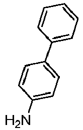 | 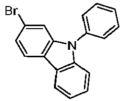 | 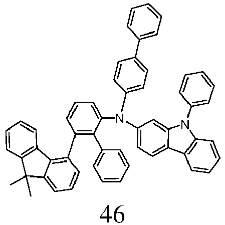 | 43 | 755.3 |
| | 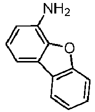 | 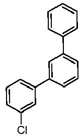 | 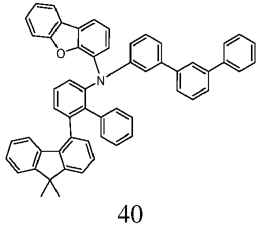 | 42 | 756.3 |
| 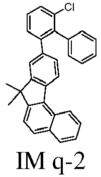 | 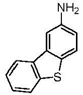 | 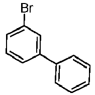 | 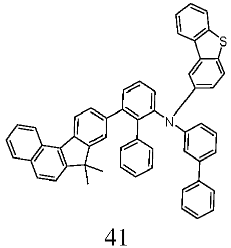 | 46 | 746.3 |
| 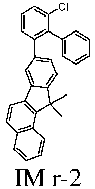 | 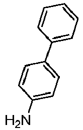 | 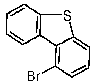 | 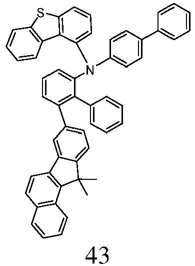 | 49 | 746.3 |
| 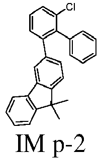 | 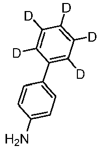 | 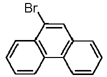 | 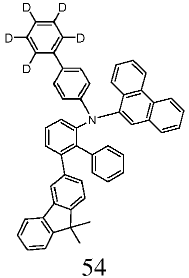 | 38 | 695.3 |
| 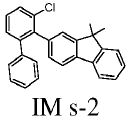 | 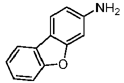 | 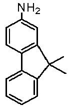 | 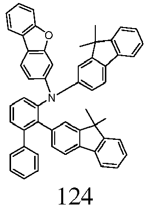 | 35 | 720.3 |
| 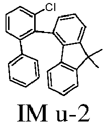 | 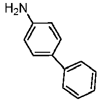 | 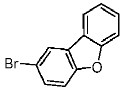 | 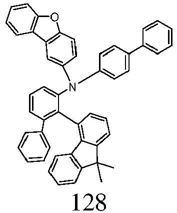 | 36 | 680.3 |
| 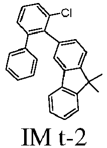 | 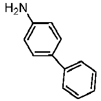 | 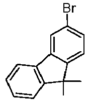 | 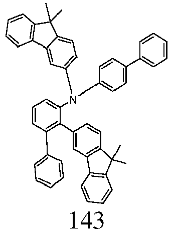 | 30 | 706.3 |
| | 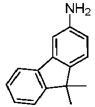 | 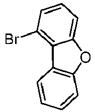 | 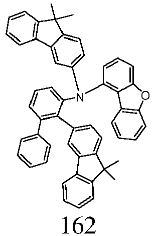 | 48 | 720.3 |
| 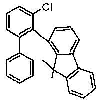 | 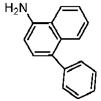 | 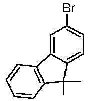 | 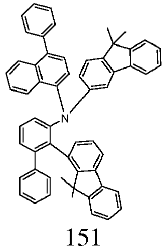 | 31 | 756.4 |
| 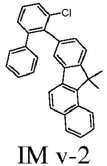 | 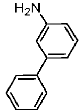 | 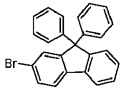 | 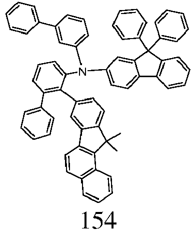 | 46 | 880.4 |
| 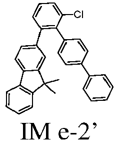 | 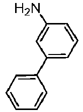 | 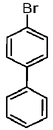 | 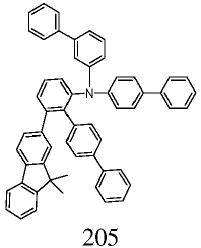 | 41 | 742.3 |
| 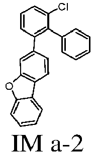 | 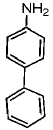 | 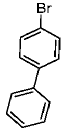 | 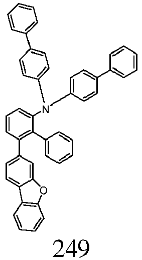 | 45 | 640.3 |
| | 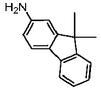 | 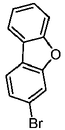 | 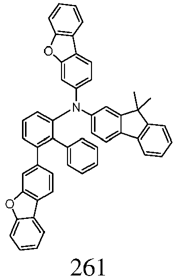 | 45 | 694.3 |
| 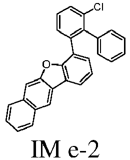 | 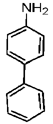 | 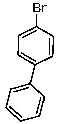 | 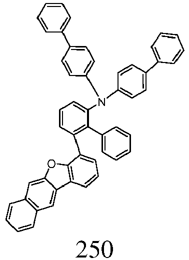 | 42 | 690.3 |
| | 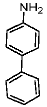 | 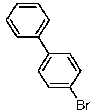 | 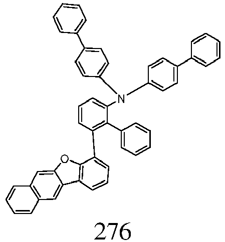 | 43 | 690.3 |
| 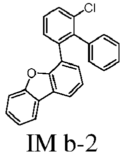 | 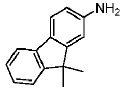 | 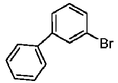 | 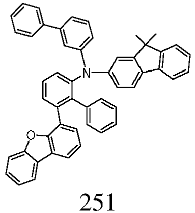 | 45 | 680.3 |
| | 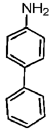 | 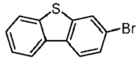 | 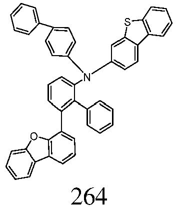 | 48 | 670.2 |
| 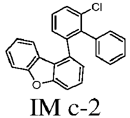 | 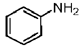 | 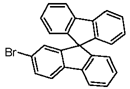 | 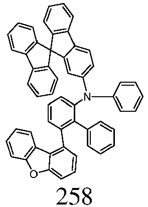 | 38 | 726.3 |
| | 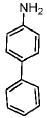 | 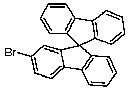 | 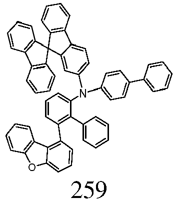 | 41 | 802.3 |
| | 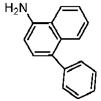 | 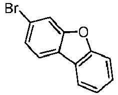 | 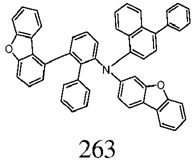 | 45 | 704.2 |
| | 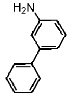 | 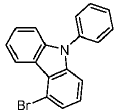 | 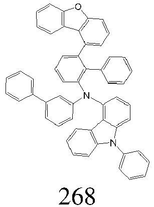 | 41 | 729.3 |
| 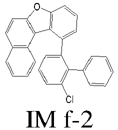 | 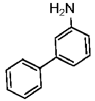 | 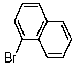 | 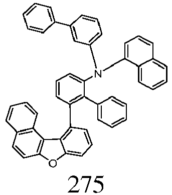 | 52 | 664.2 |
| 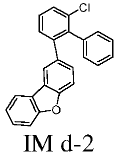 | 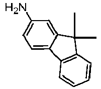 | 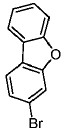 | 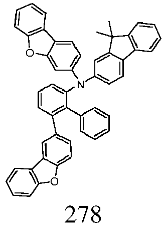 | 47 | 694.3 |
| | 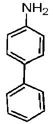 | 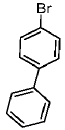 | 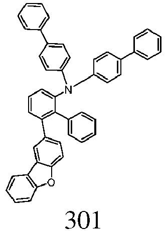 | 46 | 640.3 |
| 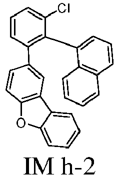 | 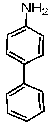 | 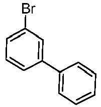 | 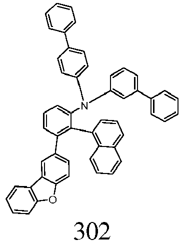 | 43 | 690.3 |
| 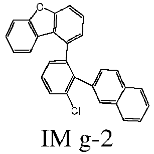 | 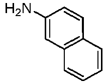 | 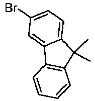 | 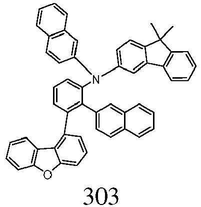 | 50 | 704.3 |
| 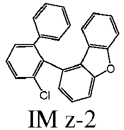 | 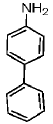 | 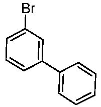 | 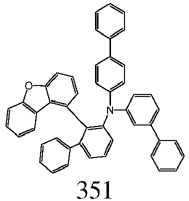 | 48 | 640.3 |
| | 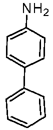 | 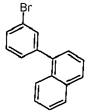 | 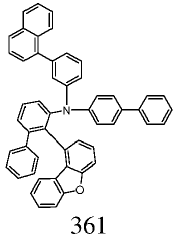 | 43 | 690.3 |
| | 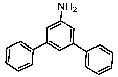 | 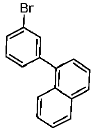 | 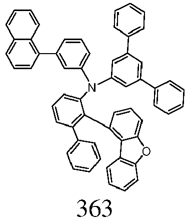 | 39 | 766.3 |
| | 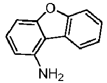 | 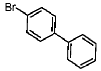 | 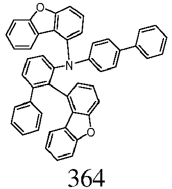 | 42 | 654.2 |
| 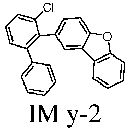 | 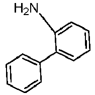 | 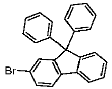 | 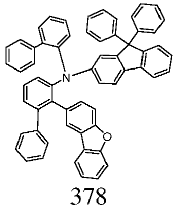 | 42 | 804.3 |
| 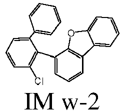 | 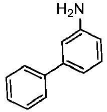 | 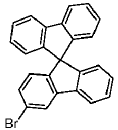 | 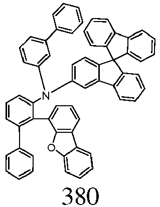 | 48 | 802.3 |
| 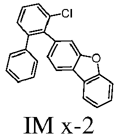 | 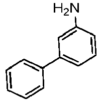 | 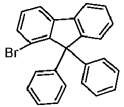 | 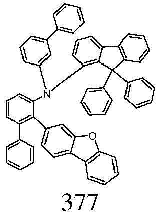 | 38 | 804.3 |
| | 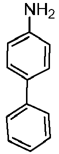 | 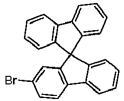 | 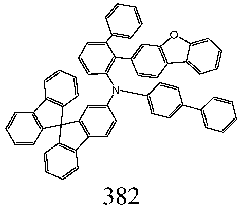 | 46 | 802.3 |
| | 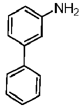 | 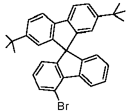 | 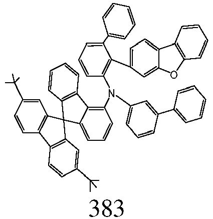 | 43 | 914.4 |
| 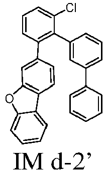 | 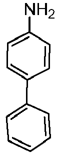 | 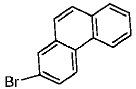 | 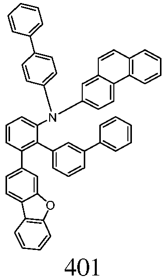 | 41 | 740.3 |
| 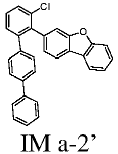 | 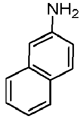 | 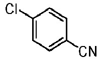 | 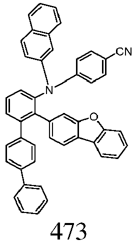 | 46 | 639.2 |
| 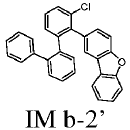 | 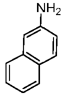 | 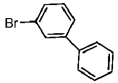 | 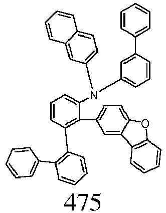 | 45 | 690.3 |
| 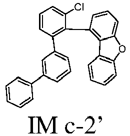 | 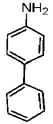 | 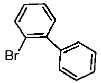 | 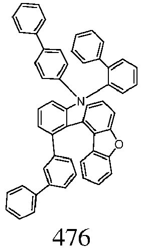 | 23 | 716.3 |
| 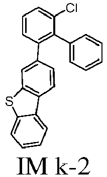 | 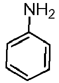 | 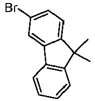 | 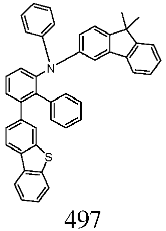 | 45 | 620.2 |
| 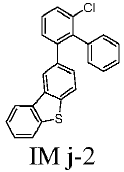 | 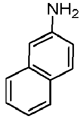 | 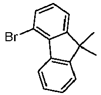 | 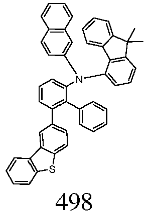 | 43 | 670.2 |
| 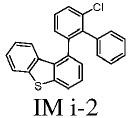 | 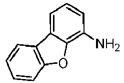 | 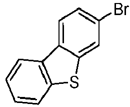 | 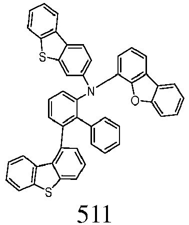 | 38 | 700.2 |
| 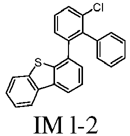 | 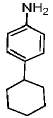 | 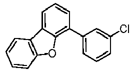 | 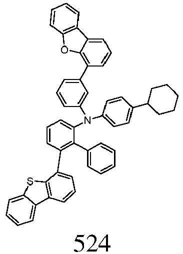 | 41 | 752.3 |
| 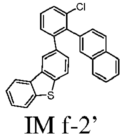 | 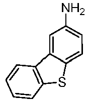 | 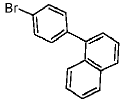 | 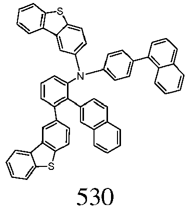 | 39 | 786.2 |
| 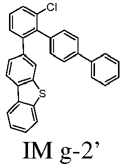 | 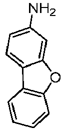 | 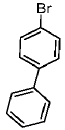 | 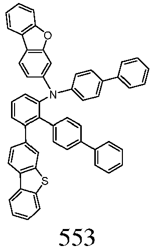 | 48 | 746.2 |
| 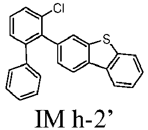 | 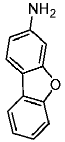 | 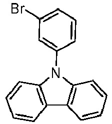 | 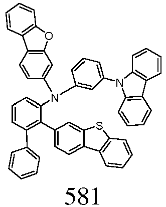 | 46 | 759.2 |
| 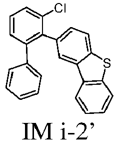 | 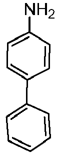 | 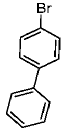 | 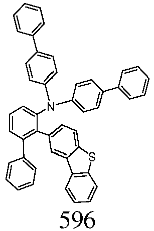 | 43 | 656.2 |
| 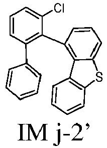 | 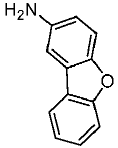 | 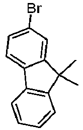 | 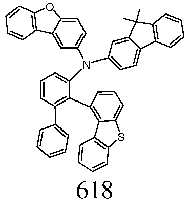 | 45 | 710.2 |
| 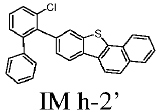 | 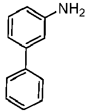 | 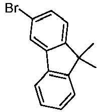 | 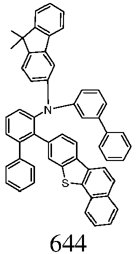 | 41 | 746.3 |
| 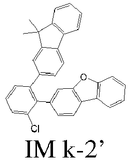 | 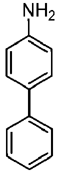 | 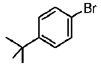 | 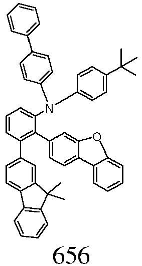 | 50 | 736.3 |
| 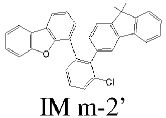 | 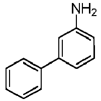 | 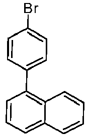 | 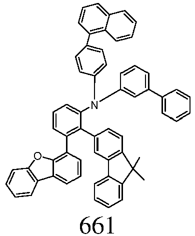 | 49 | 806.3 |
| 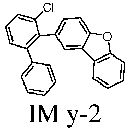 | 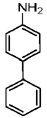 | 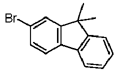 | 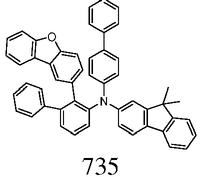 | 51 | 680.29 |

NMR of compound 259
¹H-NMR(CD₂Cl₂, 400MHz): 7.82(d, 1H), 7.67(d, 2H), 7.59-7.07(m, 22H), 7.07-6.71(m, 7H), 6.71-6.58(m, 4H), 6.57-5.6.01(m, 3H).
NMR of compound 17
¹H-NMR(CD₂Cl₂, 400MHz): 7.83(d, 1H), 7.78-7.41(m, 10H), 7.41-7.16(m, 9H), 7.15-6.77(m, 9H), 1.35-1.15(m, 12H).

### Fabrication and evaluation of organic electroluminescent devices

### Example 1

### Red organic electroluminescent device

An anode was fabricated by the following process: an ITO substrate with a thickness of 1300 Å was cut into a size of 40 mm (length) × 40 mm (width) × 0.7 mm (thickness) and prepared by photolithography into an experimental substrate with a cathode, an anode and an insulating layer pattern, surface treatment was performed with ultraviolet ozone and O₂:N₂ plasma to increase the work function of the anode (experimental substrate), and the surface of the substrate was cleaned with an organic solvent to remove foreign matters and oil from the surface of the substrate.

HAT-CN was vacuum-evaporated on the experimental substrate (anode) to form a hole injection layer (HIL) with a thickness of 100 Å, and then NPB was vacuum-evaporated on the hole injection layer to form the first hole transport layer with a thickness of 1050 Å.

The compound 1 was vacuum-evaporated on the first hole transport layer to form the second hole transport layer with a thickness of 810 Å.

On the second hole transport layer, the compound RH-1 and Ir(dmpq)₂(acac) were co-evaporated at a weight ratio of 95%: 5% to form a red light-emitting layer (EML) with a thickness of 320 Å.

The compound HB-1 was vacuum-evaporated on the red light-emitting layer to form a hole barrier layer with a thickness of 50 Å. Then the compound ET-1 and LiQ were mixed at a weight ratio of 1:1 and evaporated on the hole barrier layer to form a 300 Å thick electron transport layer (ETL), Yb was evaporated on the electron transport layer to form an electron injection layer (EIL) with a thickness of 15 Å, and then magnesium (Mg) and silver (Ag) were mixed at a 1:9 evaporation rate and vacuum-evaporated on the electron injection layer to form a cathode with a thickness of 130 Å.

In addition, CP-1 with a thickness of 680 Å was vacuum-evaporated on the above cathode to form an organic capping layer (CPL), thereby completing the fabrication of an organic electroluminescent device.

### Examples 2 to 52

Organic electroluminescent devices were fabricated by the same method as in Example 1, except that compounds in Table 5 were respectively used instead of the compound 1 used in Example 1 when the second hole transport layer was formed.

### Comparative Examples 1 to 3

Organic electroluminescent devices were fabricated by the same method as in Example 1, except that compound A, compound B, and compound C were respectively used instead of the compound 1 used in Example 1 when the second hole transport layer was formed.

In the examples and comparative examples, the structural formulas of the main materials used were shown in Table 4.

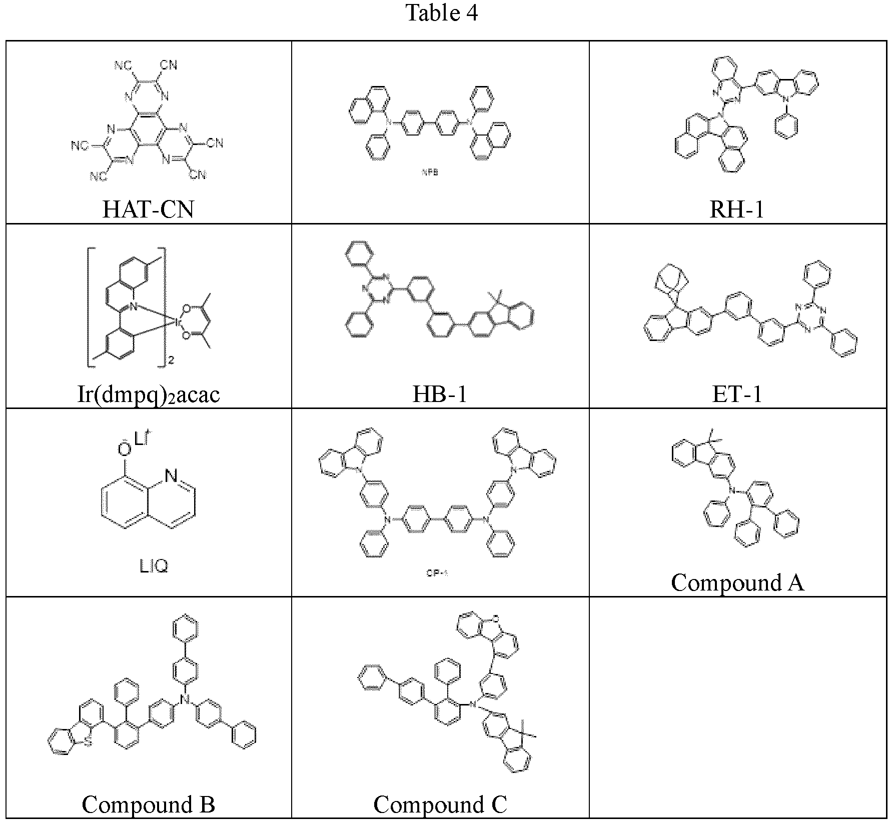

The performances of the red organic electroluminescent devices fabricated in Examples 1 to 52 and Comparative Examples 1 to 3 were tested. Specifically, the IVL performance of the devices was tested under the condition of 10 mA/cm², and the T95 device life was tested under the condition of 20 mA/cm². The test results were shown in Table 5.

**Table 5**

| Example number | Compound of second hole transport layer | Working voltage Volt (V) | Light-emit ting efficiency (Cd/A) | External quantum efficiency EQE (%) | T95 device life (h) | Color coordinates CIEx, CIEy |
|---|---|---|---|---|---|---|
| Example 1 | 1 | 3.86 | 43.2 | 29.8 | 390 | 0.682, 0.322 |
| Example 2 | 17 | 3.87 | 42.0 | 29.0 | 387 | 0.683, 0.322 |
| Example 3 | 18 | 3.86 | 42.2 | 29.1 | 391 | 0.684, 0.323 |
| Example 4 | 34 | 3.86 | 42.7 | 29.5 | 383 | 0.682, 0.322 |
| Example 5 | 40 | 3.87 | 41.9 | 28.9 | 393 | 0.683, 0.322 |
| Example 6 | 41 | 3.87 | 41.7 | 28.8 | 388 | 0.684, 0.321 |
| Example 7 | 46 | 3.83 | 42.3 | 29.2 | 381 | 0.683, 0.321 |
| Example 8 | 54 | 3.85 | 43.4 | 29.9 | 395 | 0.684, 0.323 |
| Example 9 | 101 | 3.88 | 41.9 | 28.9 | 394 | 0.682, 0.322 |
| Example 10 | 124 | 3.88 | 42.7 | 29.5 | 382 | 0.683, 0.321 |
| Example 11 | 128 | 3.83 | 42.4 | 29.3 | 390 | 0.684, 0.323 |
| Example 12 | 143 | 3.85 | 42.2 | 29.1 | 386 | 0.683, 0.322 |
| Example 13 | 151 | 3.87 | 42.2 | 29.1 | 387 | 0.683, 0.323 |
| Example 14 | 154 | 3.87 | 43.0 | 29.6 | 386 | 0.683, 0.322 |
| Example 15 | 162 | 3.83 | 42.9 | 29.6 | 394 | 0.682, 0.323 |
| Example 16 | 205 | 3.85 | 43.2 | 29.8 | 383 | 0.684, 0.323 |
| Example 17 | 249 | 3.84 | 42.0 | 29.0 | 384 | 0.683, 0.323 |
| Example 18 | 250 | 3.86 | 43.3 | 29.9 | 389 | 0.683, 0.321 |
| Example 19 | 251 | 3.88 | 42.9 | 29.6 | 388 | 0.682, 0.321 |
| Example 20 | 258 | 3.85 | 42.5 | 29.3 | 396 | 0.683, 0.323 |
| Example 21 | 263 | 3.83 | 42.8 | 29.6 | 395 | 0.684, 0.321 |
| Example 22 | 264 | 3.84 | 42.7 | 29.5 | 381 | 0.682, 0.322 |
| Example 23 | 268 | 3.84 | 42.5 | 29.3 | 388 | 0.684, 0.322 |
| Example 24 | 275 | 3.84 | 42.5 | 29.3 | 387 | 0.683, 0.322 |
| Example 25 | 276 | 3.84 | 42.2 | 29.1 | 389 | 0.684, 0.322 |
| Example 26 | 301 | 3.83 | 43.4 | 30.0 | 388 | 0.684, 0.323 |
| Example 27 | 302 | 3.88 | 42.4 | 29.2 | 381 | 0.682, 0.323 |
| Example 28 | 303 | 3.84 | 43.1 | 29.7 | 390 | 0.682, 0.321 |
| Example 29 | 351 | 3.85 | 43.5 | 30.0 | 396 | 0.682, 0.323 |
| Example 30 | 361 | 3.86 | 42.8 | 29.5 | 387 | 0.682, 0.322 |
| Example 31 | 363 | 3.86 | 41.8 | 28.8 | 388 | 0.684, 0.322 |
| Example 32 | 377 | 3.86 | 43.4 | 30.0 | 393 | 0.683, 0.322 |
| Example 33 | 378 | 3.85 | 41.8 | 28.9 | 383 | 0.682, 0.323 |
| Example 34 | 380 | 3.84 | 42.3 | 29.2 | 388 | 0.682, 0.321 |
| Example 35 | 382 | 3.85 | 42.6 | 29.4 | 384 | 0.682, 0.322 |
| Example 36 | 383 | 3.86 | 43.0 | 29.7 | 387 | 0.684, 0.322 |
| Example 37 | 401 | 3.87 | 43.1 | 29.7 | 382 | 0.682, 0.321 |
| Example 38 | 473 | 3.87 | 42.6 | 29.4 | 382 | 0.683, 0.323 |
| Example 39 | 475 | 3.86 | 42.8 | 29.6 | 381 | 0.683, 0.323 |
| Example 40 | 476 | 3.86 | 42.6 | 29.4 | 387 | 0.682, 0.322 |
| Example 41 | 497 | 3.90 | 40.7 | 28.0 | 375 | 0.683, 0.324 |
| Example 42 | 498 | 3.86 | 41.7 | 28.7 | 370 | 0.684, 0.324 |
| Example 43 | 511 | 3.90 | 42.3 | 29.2 | 374 | 0.683, 0.324 |
| Example 44 | 524 | 3.89 | 39.9 | 27.5 | 370 | 0.684, 0.324 |
| Example 45 | 530 | 3.89 | 42.1 | 29.1 | 375 | 0.683, 0.322 |
| Example 46 | 553 | 3.88 | 40.9 | 28.2 | 367 | 0.684, 0.324 |
| Example 47 | 581 | 3.86 | 40.9 | 28.2 | 367 | 0.683, 0.322 |
| Example 48 | 596 | 3.91 | 40.4 | 27.8 | 368 | 0.683, 0.322 |
| Example 49 | 618 | 3.86 | 42.1 | 29.0 | 370 | 0.682, 0.323 |
| Example 50 | 644 | 3.91 | 42.2 | 29.1 | 369 | 0.684, 0.322 |
| Example 51 | 656 | 3.87 | 40.7 | 28.1 | 372 | 0.683, 0.323 |
| Example 52 | 661 | 3.87 | 40.6 | 28.0 | 366 | 0.684, 0.324 |
| Comparative Example 1 | Compound A | 4.07 | 30.3 | 19.9 | 260 | 0.683, 0.322 |
| Comparative Example 2 | Compound B | 4.08 | 33.7 | 22.1 | 317 | 0.683, 0.323 |
| Comparative Example 3 | Compound C | 4.04 | 35.1 | 24.5 | 326 | 0.684, 0.323 |

With reference to Table 5, it can be seen that the red organic electroluminescent devices fabricated using the compounds of the present disclosure as the materials of the second hole transport layers in Examples 1 to 52 had the characteristics of high efficiency and long life. Specifically, comparing Examples 1 to 52 with Comparative Examples 1 to 3, the light-emitting efficiency was increased by at least 13.7%, the external quantum efficiency was increased by at least 12.2%, and the life was prolonged by at least 12.3%.

### Application Example 1:

### Green organic electroluminescent device

An anode was fabricated by the following process: an ITO substrate with a thickness of 1200 Å was cut into a size of 40 mm (length) × 40 mm (width) × 0.7 mm (thickness) and prepared by photolithography into an experimental substrate with a cathode, an anode and an insulating layer pattern, surface treatment was performed with ultraviolet ozone and O₂:N₂ plasma to increase the work function of the anode (experimental substrate), and the surface of the substrate was cleaned with an organic solvent to remove foreign matters and oil from the surface of the substrate.

HAT-CN was vacuum-evaporated on the experimental substrate (anode) to form a hole injection layer (HIL) with a thickness of 100 Å, and then NPB was vacuum-evaporated on the hole injection layer to form a first hole transport layer with a thickness of 1000 Å.

The compound 251 was vacuum-evaporated on the first hole transport layer to form a second hole transport layer with a thickness of 360 Å.

On the second hole transport layer, the compound GH-1 and Ir(ppy)₃ were co-evaporated at a weight ratio of 95%: 5% to form a green light-emitting layer (EML) with a thickness of 400 Å.

The compound ET-2 and LiQ were mixed at a weight ratio of 1:1 and evaporated on the light-emitting layer to form a 320 Å thick electron transport layer (ETL), Yb was evaporated on the electron transport layer to form an electron injection layer (EIL) with a thickness of 15 Å, and then magnesium (Mg) and silver (Ag) were mixed at a 1:9 evaporation rate and vacuum-evaporated on the electron injection layer to form a cathode with a thickness of 130 Å.

In addition, CP-1 with a thickness of 600 Å was vacuum-evaporated on the above cathode to form an organic capping layer (CPL), thereby completing the fabrication of an organic electroluminescent device.

### Application Examples 2 to 17

Organic electroluminescent devices were fabricated by the same method as in Application Example 1, except that compounds in Table 7 were respectively used instead of the compound 251 used in Application Example 1 when the second hole transport layer was formed.

### Comparative Examples 4 to 7

Organic electroluminescent devices were fabricated by the same method as in Application Example 1, except that compound A, compound B, compound D, and compound E were respectively used instead of the compound 251 used in Application Example 1 when the second hole transport layer was formed.

In the application examples and comparative examples, the structural formula of each material used was shown in Table 6.

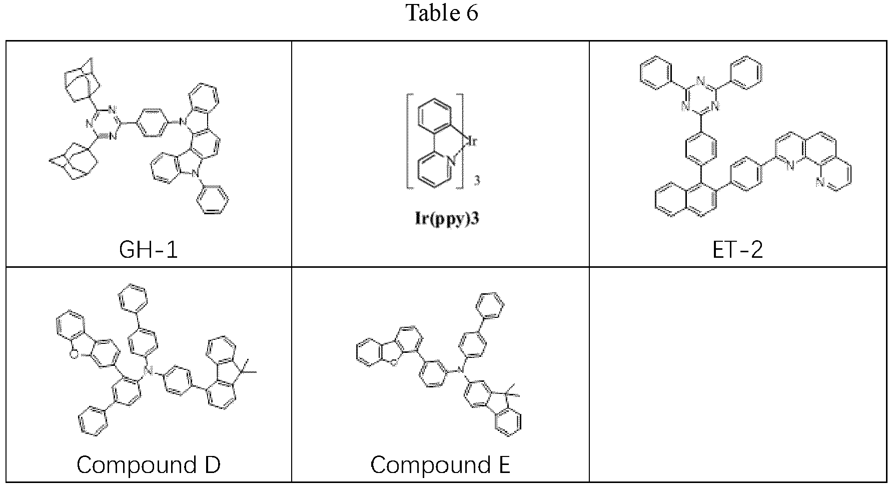

The performances of the green organic electroluminescent devices fabricated in Application Examples 1 to 17 and Comparative Examples 4 to 7 were tested. Specifically, the performances of the devices were tested under the condition of 10 mA/cm². The test results were shown in Table 7.

**Table 7**

| Application example number | Compound of second hole transport layer | Working voltage Volt (V) | Light-emit ting efficiency (Cd/A) | External quantum efficiency EQE (%) | T95 device life (h) | Color coordinates CIEx, CIEy |
|---|---|---|---|---|---|---|
| Application Example 1 | Compound 251 | 3.95 | 82.81 | 27.31 | 220 | 0.264, 0.704 |
| Application Example 2 | Compound 258 | 3.84 | 81.84 | 27.57 | 214 | 0.266, 0.708 |
| Application Example 3 | Compound 259 | 3.9 | 81.73 | 27.63 | 218 | 0.263, 0.706 |
| Application Example 4 | Compound 261 | 3.89 | 84.91 | 28.01 | 200 | 0.264, 0.704 |
| Application Example 5 | Compound 263 | 3.86 | 86.57 | 28.53 | 207 | 0.263, 0.704 |
| Application Example 6 | Compound 264 | 3.94 | 81.93 | 27.67 | 211 | 0.263, 0.705 |
| Application Example 7 | Compound 301 | 4.02 | 83.19 | 27.49 | 203 | 0.265, 0.708 |
| Application Example 8 | Compound 303 | 3.92 | 82.56 | 27.28 | 216 | 0.266, 0.706 |
| Application Example 9 | Compound 351 | 3.88 | 81.45 | 26.93 | 260 | 0.265, 0.707 |
| Application Example 10 | Compound 361 | 4.03 | 81.83 | 27.68 | 255 | 0.265, 0.705 |
| Application Example 11 | Compound 363 | 3.87 | 82.17 | 27.19 | 253 | 0.264, 0.708 |
| Application Example 12 | Compound 364 | 4.01 | 86.49 | 28.57 | 247 | 0.264, 0.706 |
| Application Example 13 | Compound 377 | 3.93 | 83.25 | 27.54 | 300 | 0.263, 0.707 |
| Application Example 14 | Compound 382 | 3.98 | 81.54 | 26.92 | 297 | 0.264, 0.705 |
| Application Example 15 | Compound 383 | 3.97 | 82.73 | 27.38 | 290 | 0.263, 0.705 |
| Application Example 16 | Compound 473 | 3.96 | 83.18 | 27.48 | 288 | 0.264, 0.708 |
| Application Example 17 | Compound 735 | 3.85 | 83.69 | 27.86 | 310 | 0.264, 0.709 |
| Comparative Example 4 | Compound A | 4.18 | 72.41 | 22.91 | 179 | 0.266, 0.707 |
| Comparative Example 5 | Compound B | 4.11 | 68.90 | 21.43 | 168 | 0.264, 0.708 |
| Comparative Example 6 | Compound D | 4.21 | 69.72 | 19.37 | 174 | 0.266, 0.708 |
| Comparative Example 7 | Compound E | 4.16 | 70.13 | 20.28 | 175 | 0.266, 0.707 |

With reference to Table 7, it can be seen that the green organic electroluminescent devices fabricated using the compounds of the present disclosure as the materials of the second hole transport layers in Application Examples 1 to 17 had the characteristics of high efficiency and long life. Specifically, comparing Application Examples 1 to 17 with Comparative Examples 4 to 7, the light-emitting efficiency was increased by at least 12.5%, the external quantum efficiency was increased by at least 17.6%, and the life was prolonged by at least 11.7%.

Those of ordinary skilled in the art can understand that the above embodiments are specific examples for implementing the present invention, and in practical applications, various changes in form and details can be made without departing from the spirit and scope of the present disclosure.

## Claims

1. An organic electroluminescent material, having a structure as represented by Formula I: 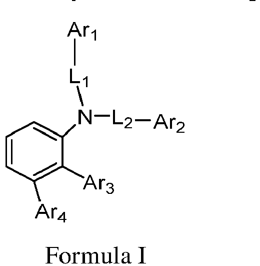 wherein Ar₁ and Ar₂ are the same or different, and are each independently selected from substituted or unsubstituted aryl with 6 to 40 carbon atoms, or substituted or unsubstituted heteroaryl with 3 to 30 carbon atoms;
L₁ and L₂ are the same or different, and are each independently selected from a single bond, substituted or unsubstituted arylene with 6 to 30 carbon atoms, or substituted or unsubstituted heteroarylene with 6 to 30 carbon atoms;
Ar₃ and Ar₄ are the same or different, and are each independently selected from substituted or unsubstituted aryl with 6 to 30 carbon atoms, or substituted or unsubstituted heteroaryl with 3 to 30 carbon atoms, and at least one of Ar₃ and Ar₄ is selected from 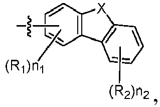 wherein X is selected from C(R₃R₄), O, or S;
R₃ and R₄ are the same or different, and are each independently selected from alkyl with 1 to 5 carbon atoms, aryl with 6 to 12 carbon atoms, or heteroaryl with 3 to 12 carbon atoms; or, R₃ and R₄ form a saturated or unsaturated 5- to 13-membered ring;
R₁ and R₂ are the same or different, and are each independently selected from deuterium, a halogen group, cyano, alkyl with 1 to 5 carbon atoms, trialkylsilyl with 3 to 12 carbon atoms, aryl with 6 to 12 carbon atoms, or heteroaryl with 3 to 12 carbon atoms;
R₁ and R₂ are represented by Rᵢ, n₁ and n₂ are represented by nᵢ, nᵢ represents the number of Rᵢ, i is a variable and represents 1 and 2, and when i is 1 and 2, nᵢ is selected from 0, 1, 2, 3 or 4; when nᵢ is greater than 1, any two Rᵢs are the same or different; optionally, any two adjacent Rᵢs are connected to each other to form an unsaturated 6- to 10-membered ring; and
the substituents in Ar₁, Ar₂, L₁, L₂, Ar₃ and Ar₄ are the same or different, and are each independently selected from deuterium, a halogen group, cyano, trialkylsilyl with 3 to 12 carbon atoms, triphenylsilyl, alkyl with 1 to 10 carbon atoms, aryl with 6 to 20 carbon atoms, heteroaryl with 3 to 20 carbon atoms, or cycloalkyl with 3 to 10 carbon atoms; optionally, any two adjacent substituents in Ar₁ and Ar₂ form a substituted or unsubstituted 3- to 15-membered ring, and the substituents in the 3- to 15-membered ring are independently selected from deuterium, a halogen group, cyano, trialkylsilyl with 3 to 6 carbon atoms, triphenylsilyl, or alkyl with 1 to 5 carbon atoms.

2. The organic electroluminescent material of claim 1, wherein Ar₁ and Ar₂ are each independently selected from substituted or unsubstituted aryl with 6 to 33 carbon atoms, or substituted or unsubstituted heteroaryl with 5 to 18 carbon atoms; and
preferably, the substituents in Ar₁ and Ar₂ are each independently selected from deuterium, fluorine, cyano, trimethylsilyl, triphenylsilyl, alkyl with 1 to 5 carbon atoms, aryl with 6 to 12 carbon atoms, heteroaryl with 5 to 12 carbon atoms, or cycloalkyl with 5 to 10 carbon atoms; and optionally, any two adjacent substituents in Ar₁ and Ar₂ form a substituted or unsubstituted 5- to 13-membered ring, and the substituents in the 5- to 13-membered ring are each independently selected from deuterium, fluorine, cyano, trimethylsilyl, triphenylsilyl, methyl, ethyl, isopropyl, or tert-butyl.

3. The organic electroluminescent material of claim 1, wherein Ar₁ and Ar₂ are each independently selected from substituted or unsubstituted phenyl, substituted or unsubstituted naphthyl, substituted or unsubstituted biphenyl, substituted or unsubstituted fluorenyl, substituted or unsubstituted dibenzofuranyl, substituted or unsubstituted dibenzothienyl, substituted or unsubstituted carbazolyl, substituted or unsubstituted phenanthryl, substituted or unsubstituted triphenylene, substituted or unsubstituted pyrenyl, or substituted or unsubstituted anthryl; and
preferably, the substituents in Ar₁ and Ar₂ are each independently selected from deuterium, fluorine, cyano, trimethylsilyl, triphenylsilyl, methyl, ethyl, isopropyl, tert-butyl, cyclopentyl, cyclohexyl, adamantyl, pyridyl, phenyl, naphthyl, or biphenyl; and optionally, any two adjacent substituents in Ar₁ and Ar₂ form adamantane, cyclopentane, cyclohexane, a fluorene ring or a tert-butyl substituted fluorene ring.

4. The organic electroluminescent material of claim 1, wherein Ar₁ and Ar₂ are each independently selected from a substituted or unsubstituted group W, and the unsubstituted group W is selected from the following groups: 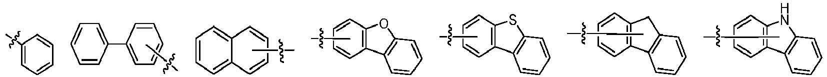 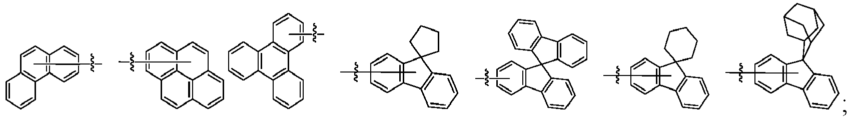 wherein the substituted group W has one or two or more substituents, the substituents are each independently selected from deuterium, fluorine, cyano, trimethylsilyl, triphenylsilyl, methyl, ethyl, isopropyl, tert-butyl, cyclopentyl, cyclohexyl, adamantyl, pyridyl, phenyl, naphthyl, or biphenyl, and when the number of substituents is greater than 1, the substituents are the same or different.

5. The organic electroluminescent material of claim 1, wherein Ar₁ and Ar₂ are each independently selected from the following groups:

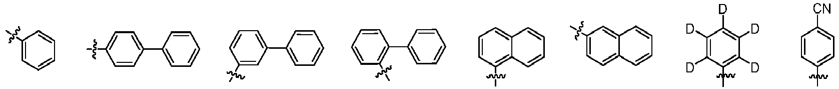

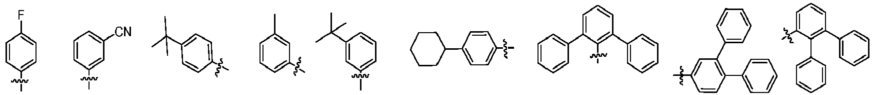

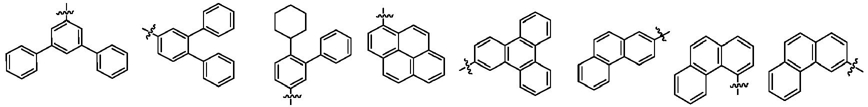

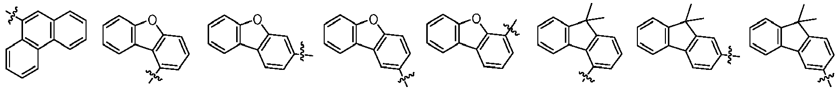

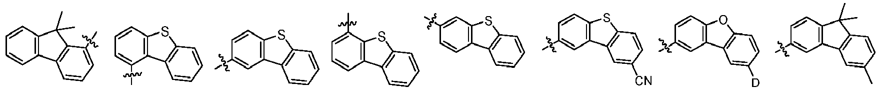

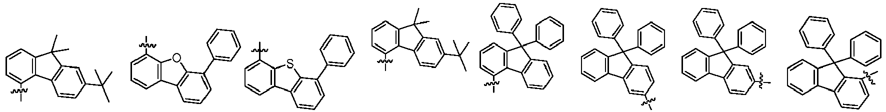

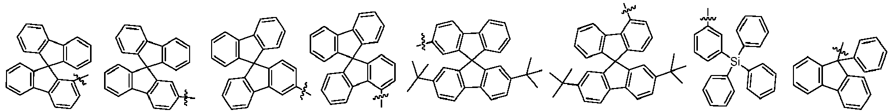

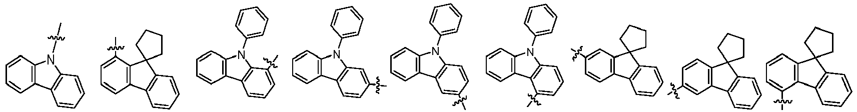

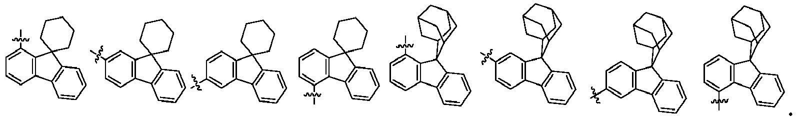

6. The organic electroluminescent material of claim 1, wherein L₁ and L₂ are each independently selected from a single bond, or substituted or unsubstituted arylene with 6 to 20 carbon atoms; and
preferably, the substituents in L₁ and L₂ are each independently selected from deuterium, fluorine, cyano, alkyl with 1 to 5 carbon atoms, aryl with 6 to 12 carbon atoms, or trimethylsilyl.

7. The organic electroluminescent material of claim 1, wherein L₁ and L₂ are each independently selected from a single bond, or substituted or unsubstituted arylene with 6 to 12 carbon atoms; and
preferably, the substituents in L₁ and L₂ are each independently selected from deuterium, fluorine, cyano, methyl, ethyl, isopropyl, tert-butyl, phenyl, or trimethylsilyl.

8. The organic electroluminescent material of claim 1, wherein L₁ and L₂ are each independently selected from a single bond, substituted or unsubstituted phenylene, substituted or unsubstituted naphthylene, or substituted or unsubstituted biphenylene; and
preferably, the substituents in L₁ and L₂ are each independently selected from deuterium, fluorine, cyano, methyl, ethyl, isopropyl, tert-butyl, phenyl, or trimethylsilyl.

9. The organic electroluminescent material of claim 1, wherein L₁ and L₂ are each independently selected from a single bond or the following groups:

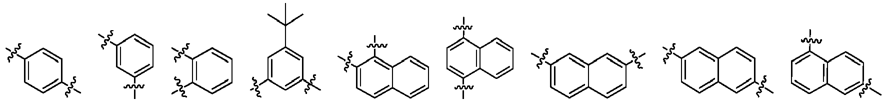

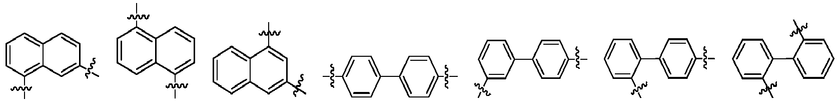

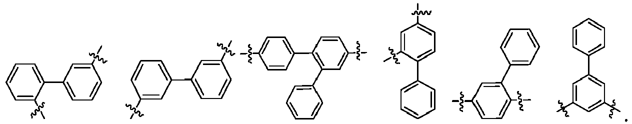

10. The organic electroluminescent material of claim 1, wherein Ar₃ and Ar₄ are each independently selected from substituted or unsubstituted aryl with 6 to 20 carbon atoms, or substituted or unsubstituted heteroaryl with 12 to 16 carbon atoms; and at least one of Ar₃ and Ar₄ is selected from 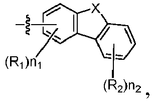 preferably, the substituents in Ar₃ and Ar₄ are each independently selected from deuterium, fluorine, cyano, trimethylsilyl, triphenylsilyl, alkyl with 1 to 5 carbon atoms, aryl with 6 to 12 carbon atoms, or heteroaryl with 3 to 12 carbon atoms.

11. The organic electroluminescent material of claim 1, wherein Ar₃ and Ar₄ are each independently selected from a substituted or unsubstituted group Q, and the unsubstituted group Q is selected from the following groups: 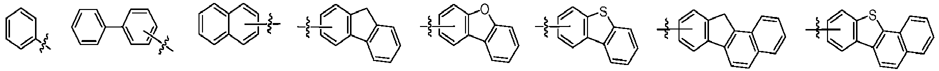 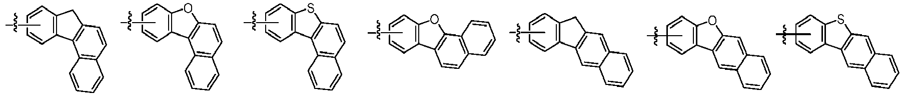 wherein the substituted group Q has one or two or more substituents, the substituents are each independently selected from deuterium, fluorine, cyano, trimethylsilyl, triphenylsilyl, methyl, ethyl, isopropyl, tert-butyl, phenyl, naphthyl, or biphenyl, and when the number of substituents is greater than 1, the substituents are the same or different.

12. The organic electroluminescent material of claim 1, wherein Ar₃ and Ar₄ are each independently selected from the following groups:

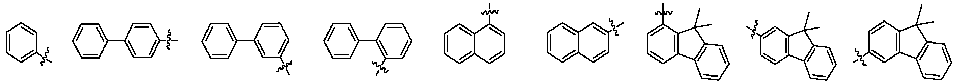

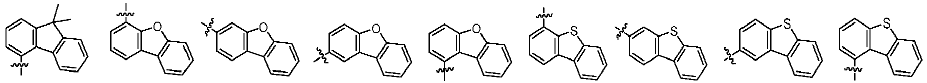

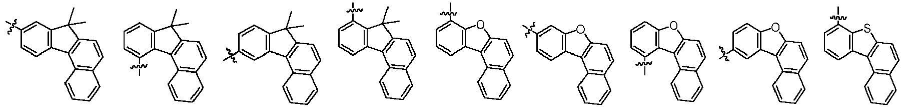

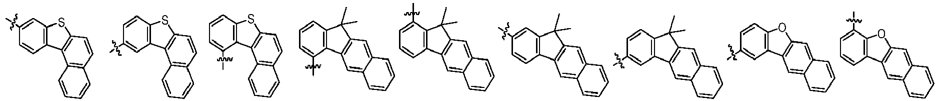

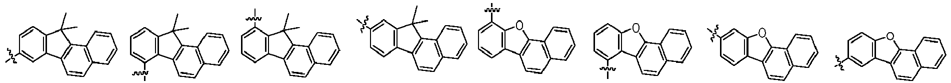

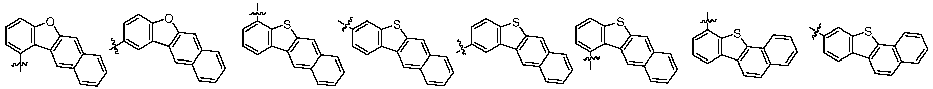

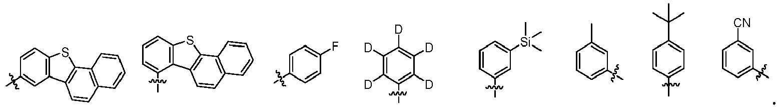

13. The organic electroluminescent material of claim 1, wherein R₃ and R₄ are each independently selected from methyl, ethyl, isopropyl, tert-butyl, phenyl, naphthyl, biphenyl, pyridyl, pyrimidinyl, quinolyl, isoquinolyl, dibenzofuranyl, dibenzothienyl, or carbazolyl; alternatively, R₃ and R₄ form cyclopentane, cyclohexane, or a fluorene ring.

14. The organic electroluminescent material of claim 1, wherein R₁ and R₂ are each independently selected from deuterium, fluorine, cyano, methyl, ethyl, isopropyl, tert-butyl, trimethylsilyl, phenyl, naphthyl, biphenyl, pyridyl, pyrimidinyl, quinolyl, isoquinolyl, dibenzofuranyl, dibenzothienyl, or carbazolyl.

15. The organic electroluminescent material of claim 1, wherein the organic electroluminescent material is selected from a group consisting of the following compounds:

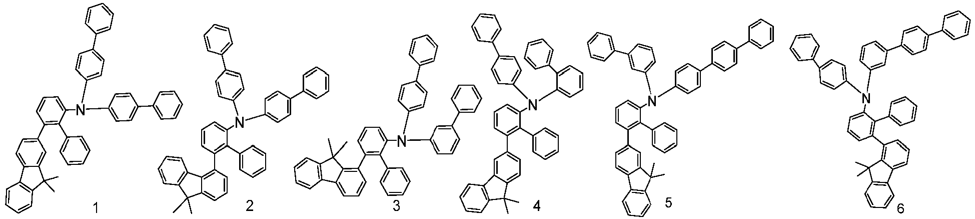

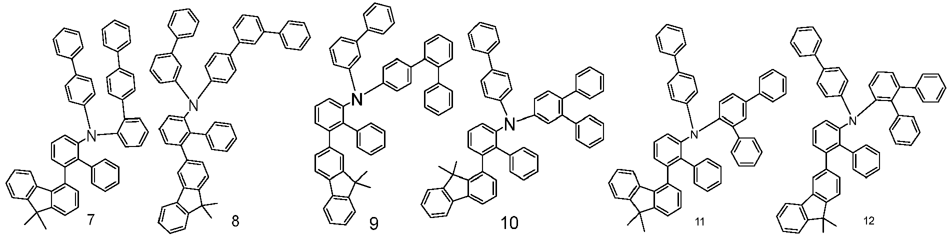

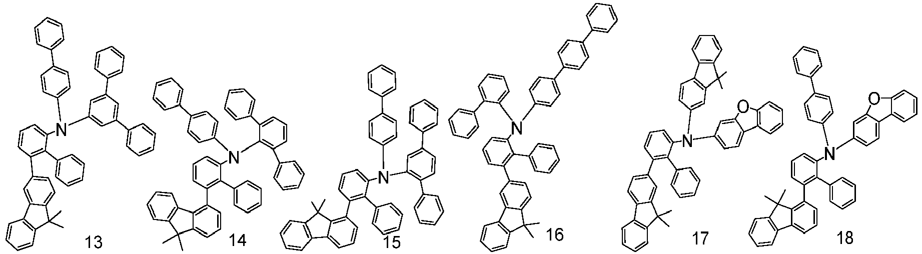

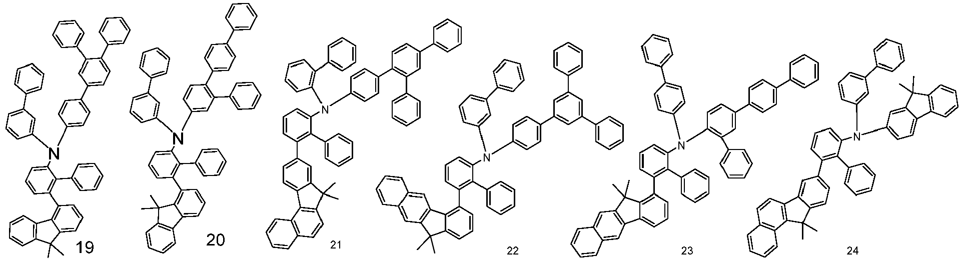

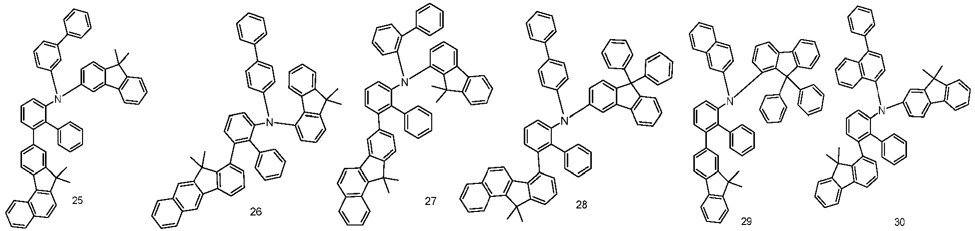

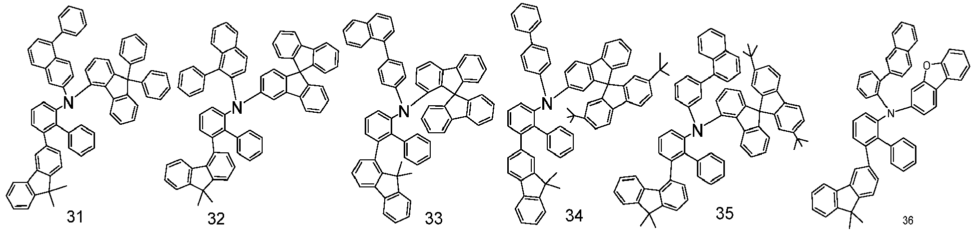

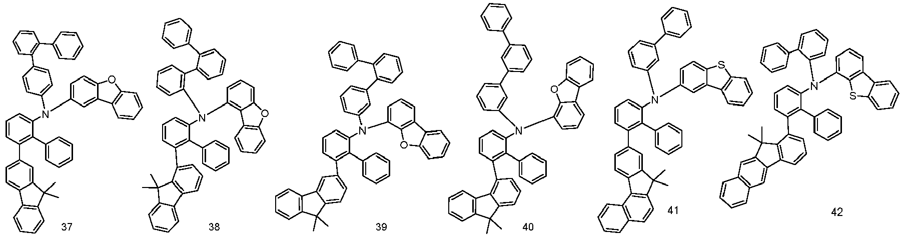

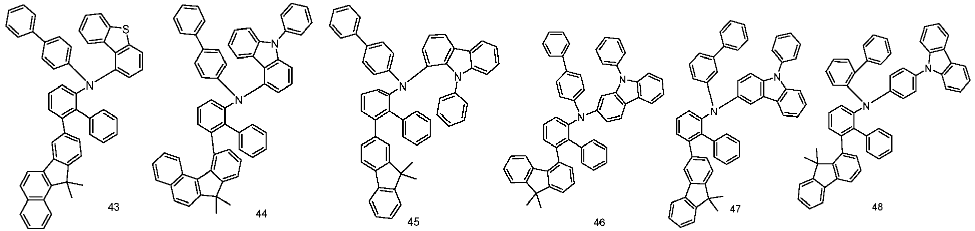

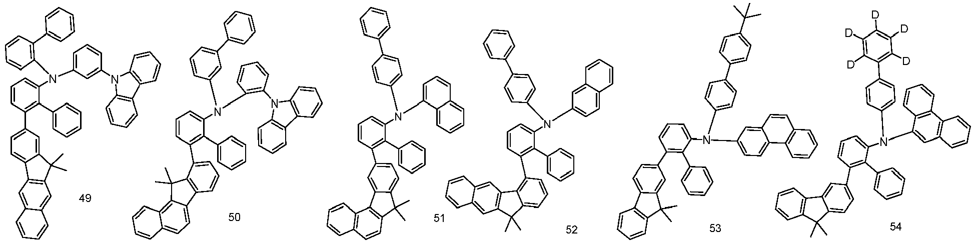

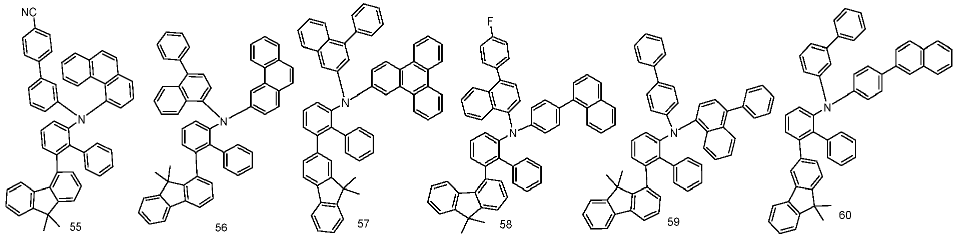

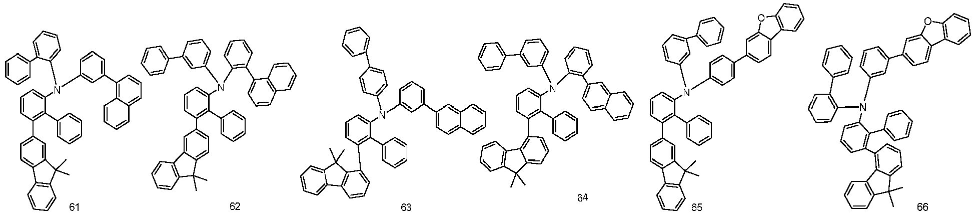

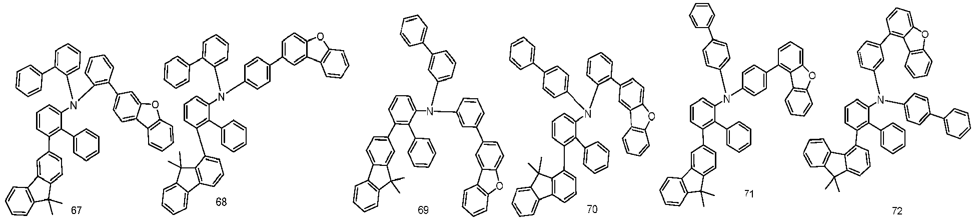

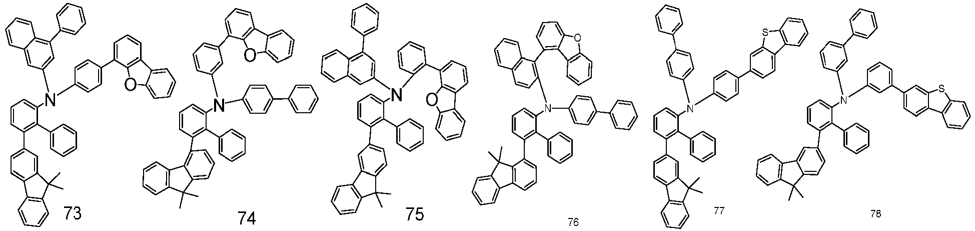

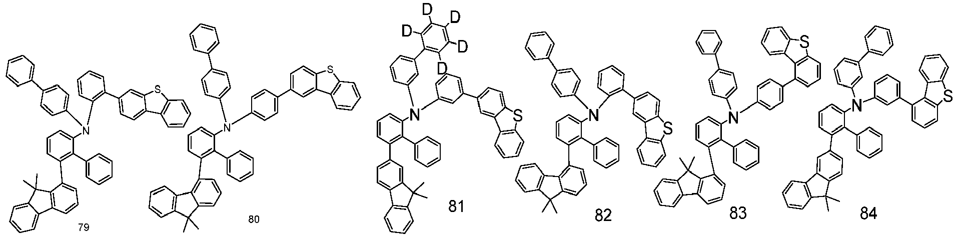

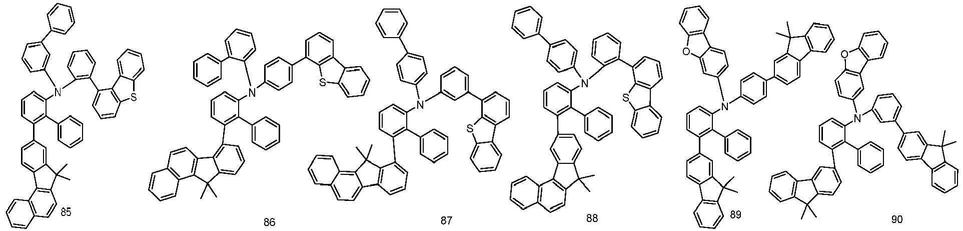

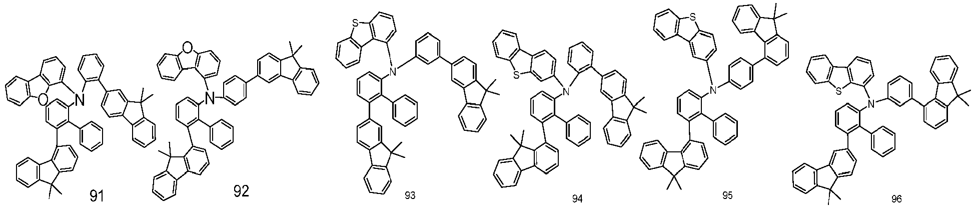

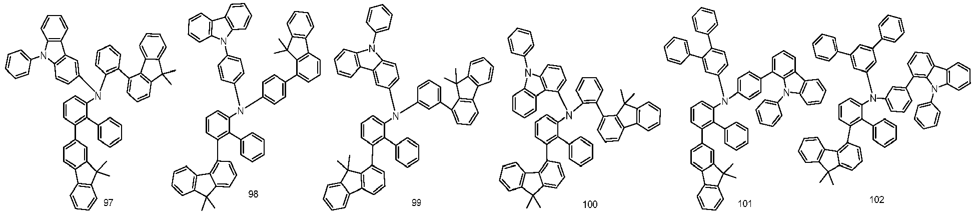

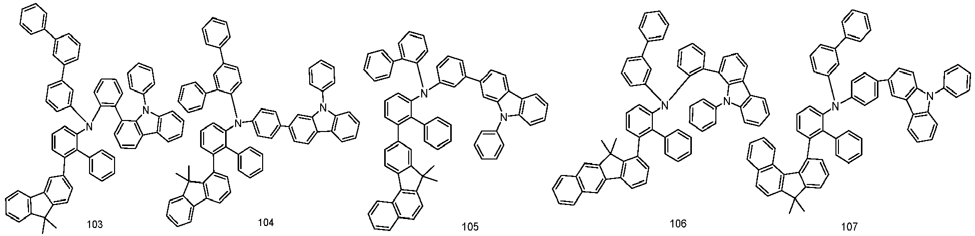

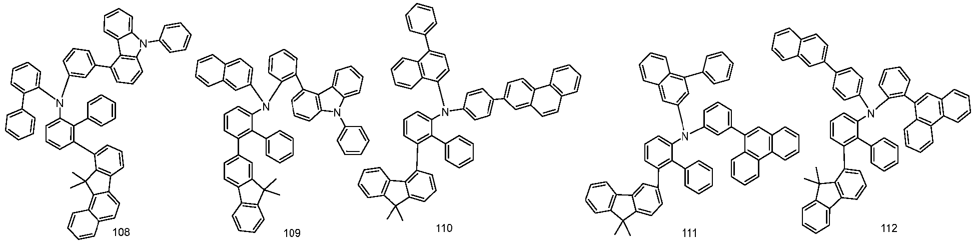

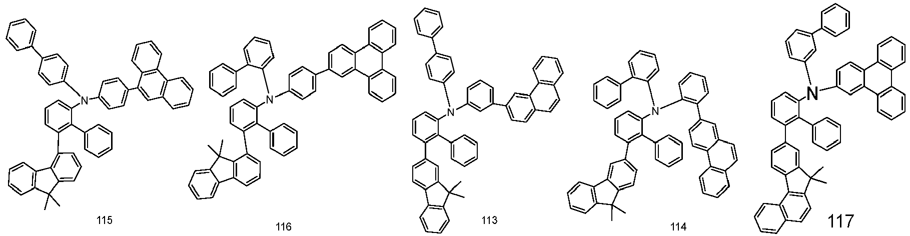

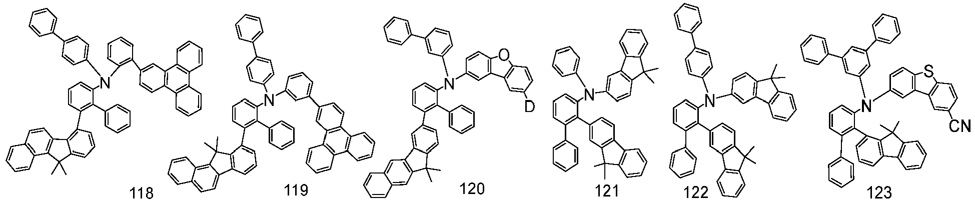

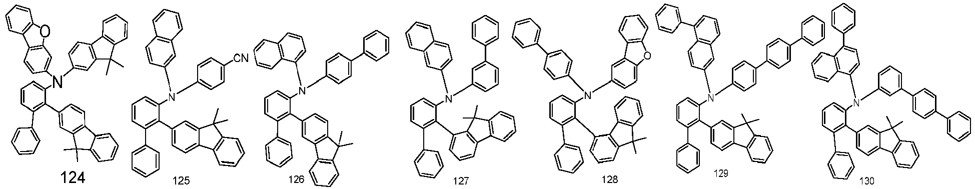

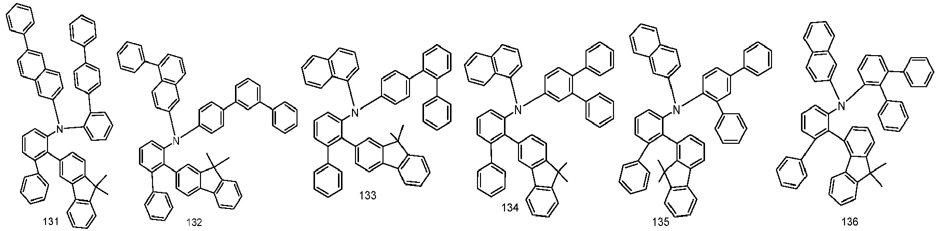

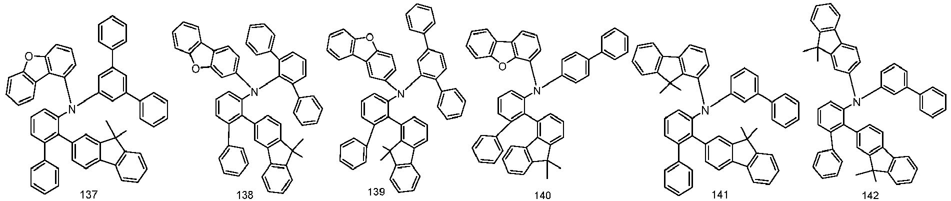

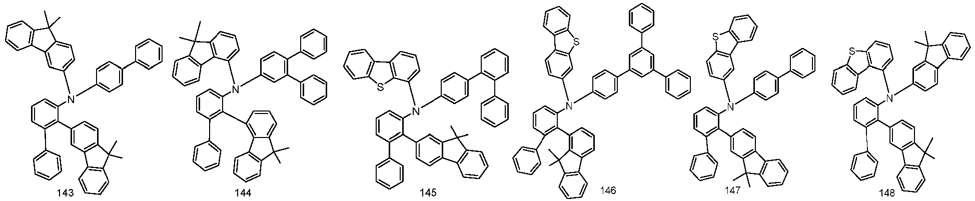

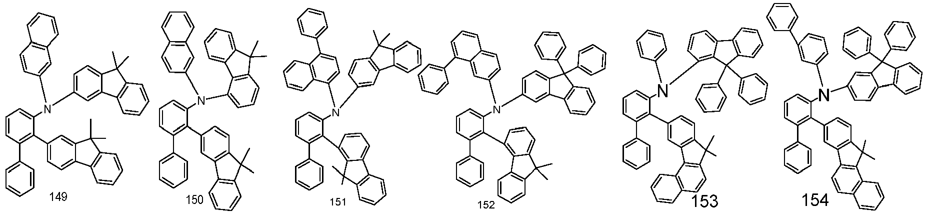

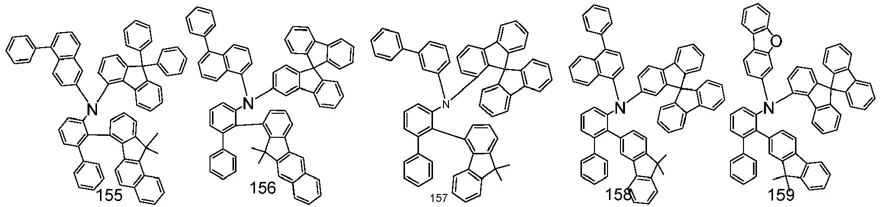

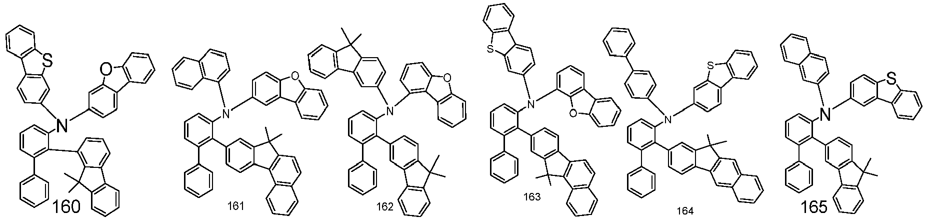

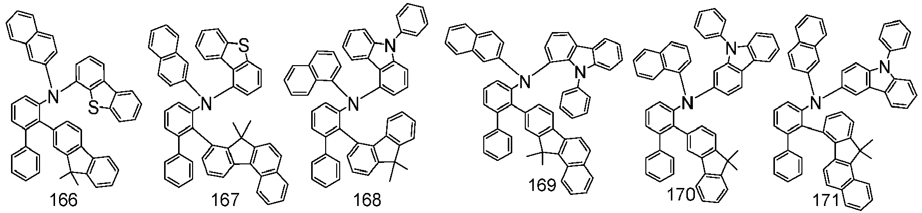

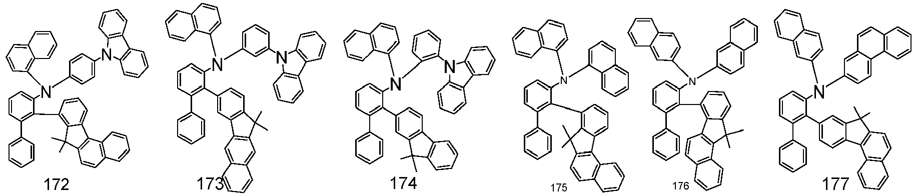

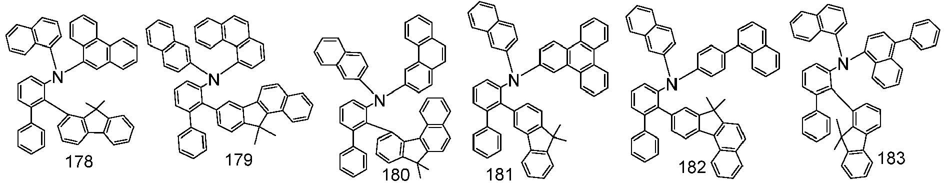

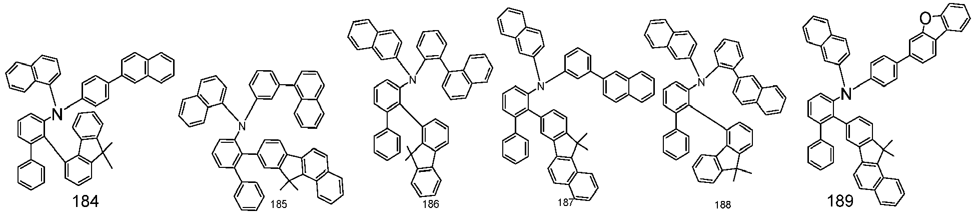

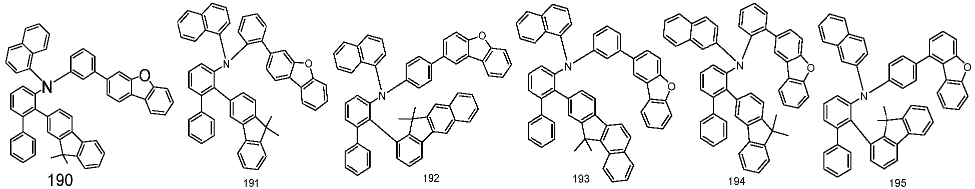

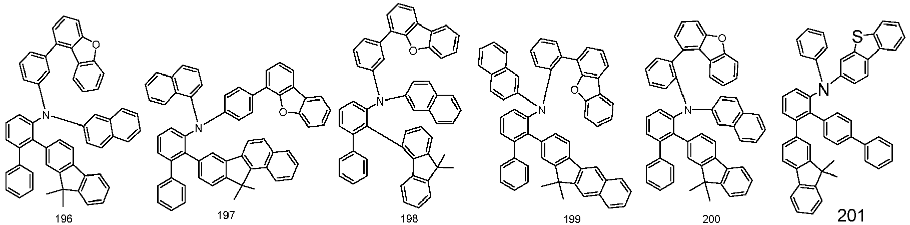

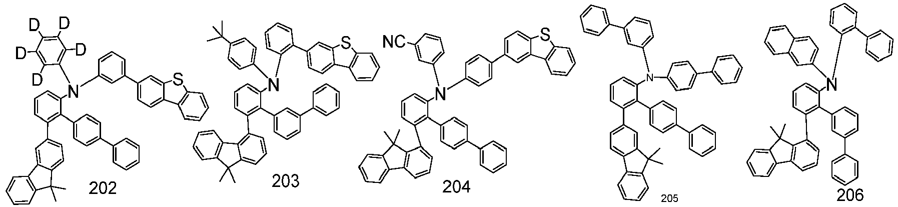

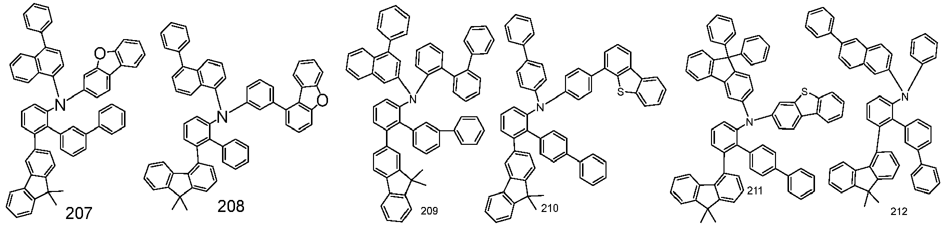

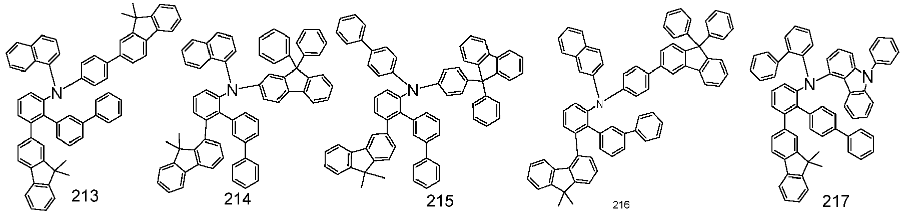

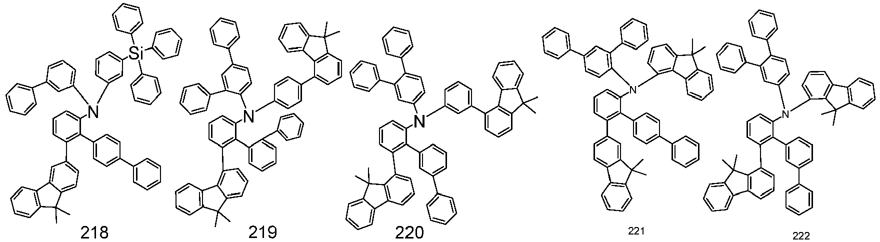

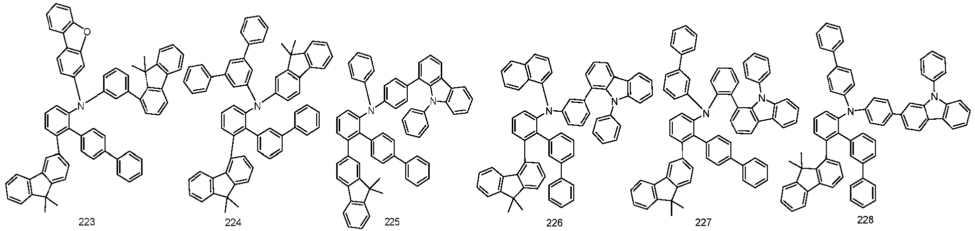

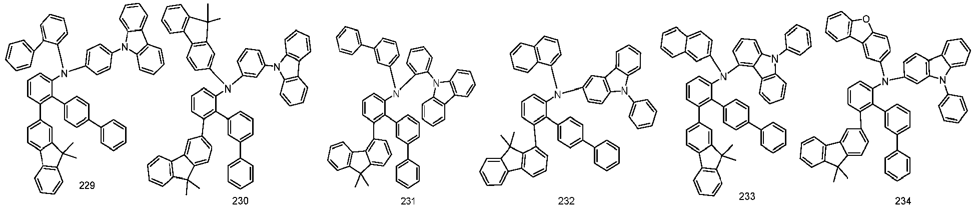

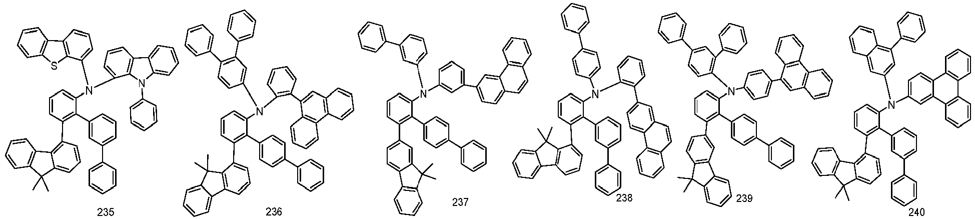

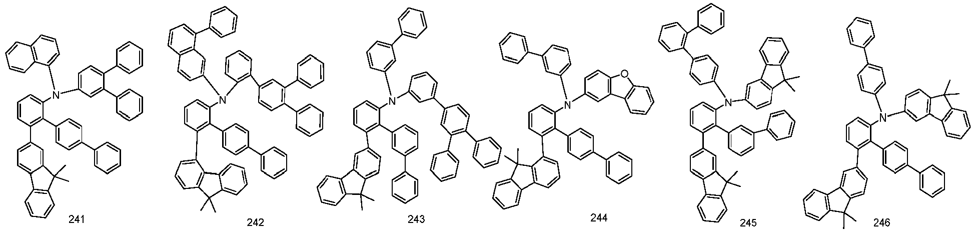

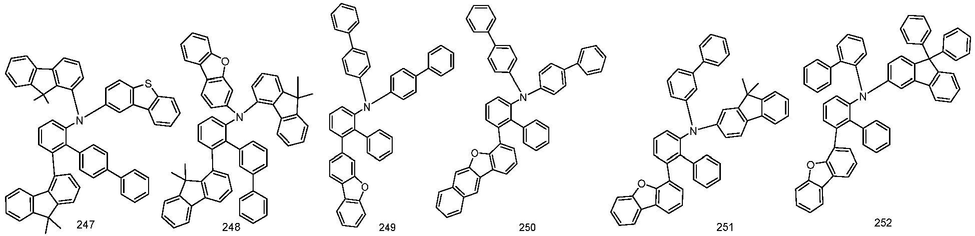

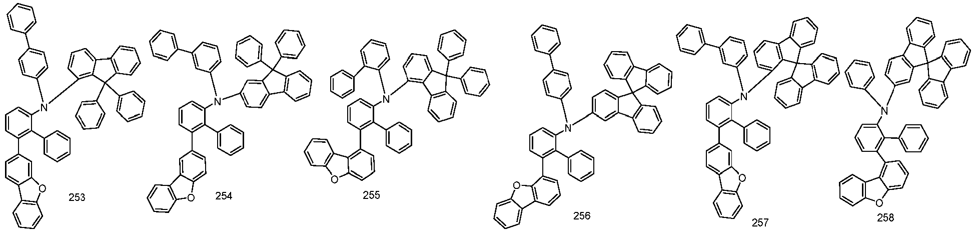

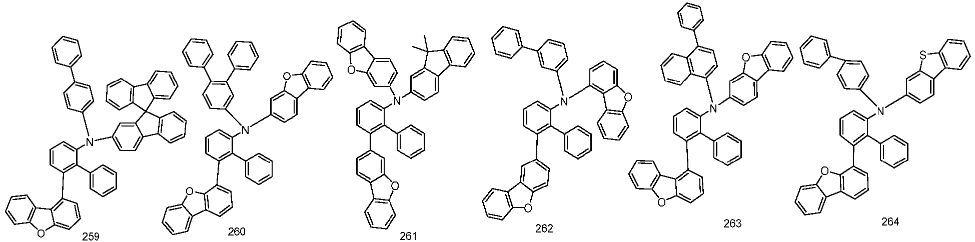

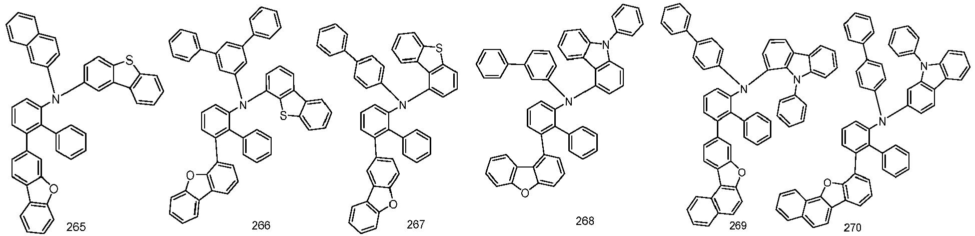

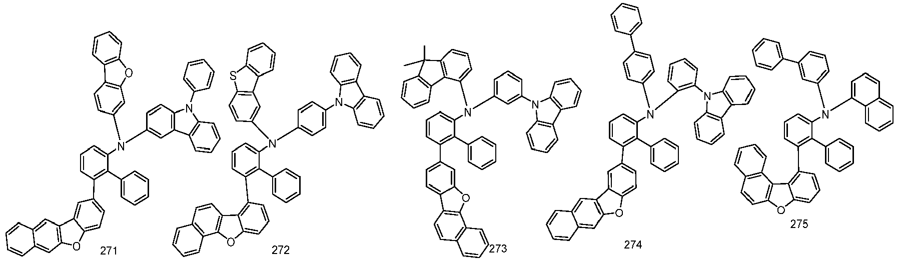

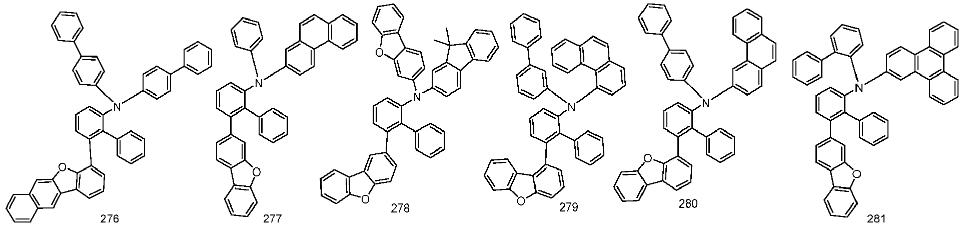

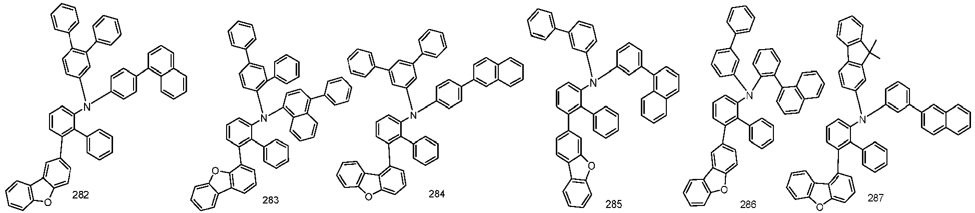

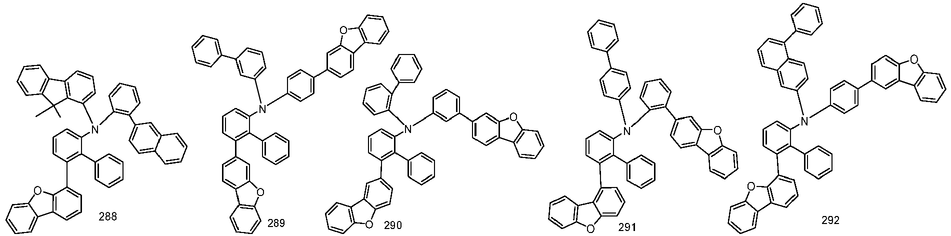

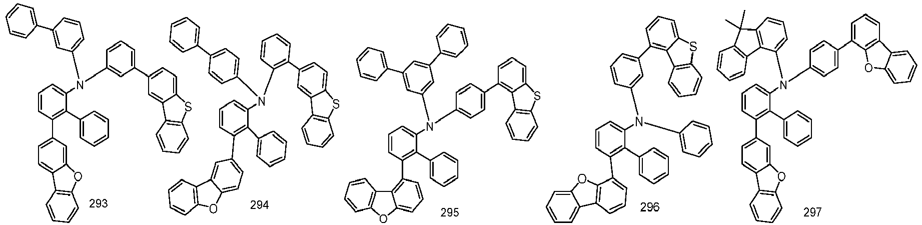

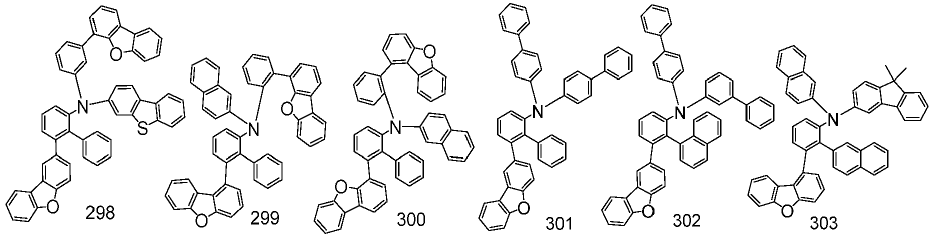

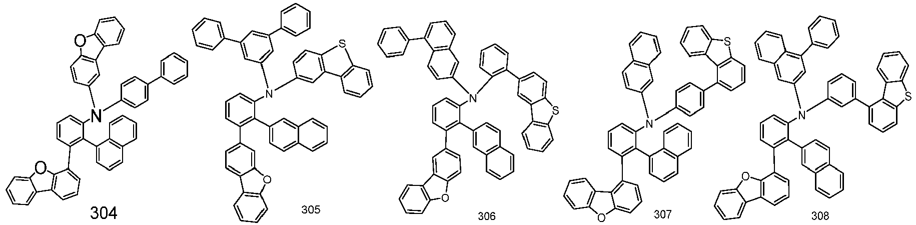

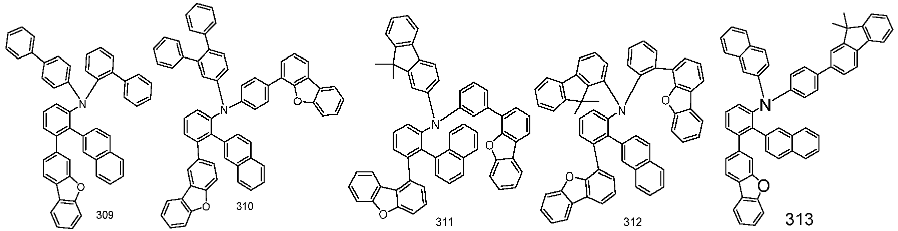

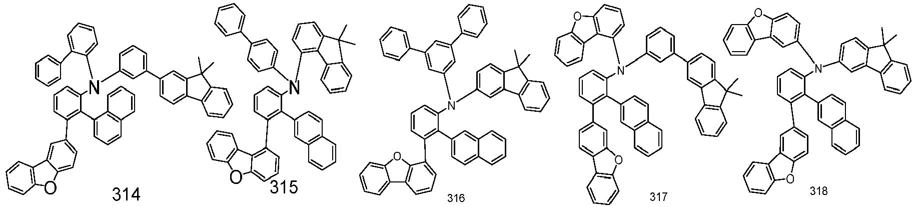

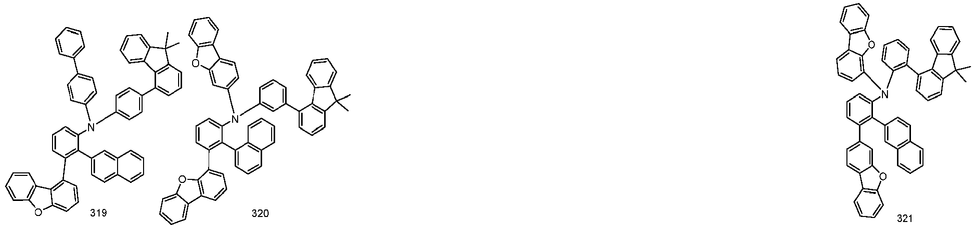

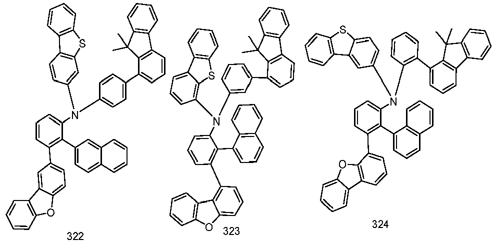

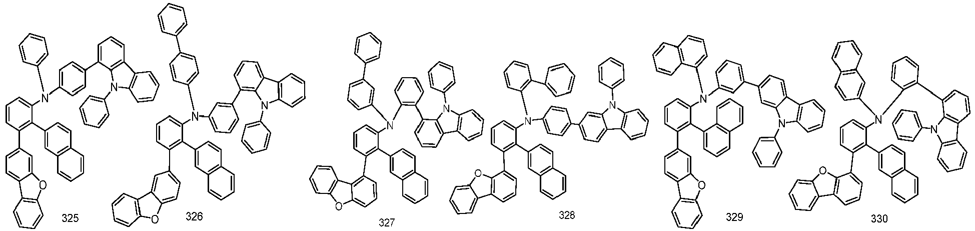

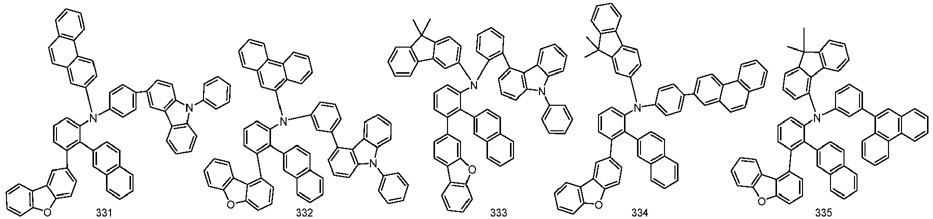

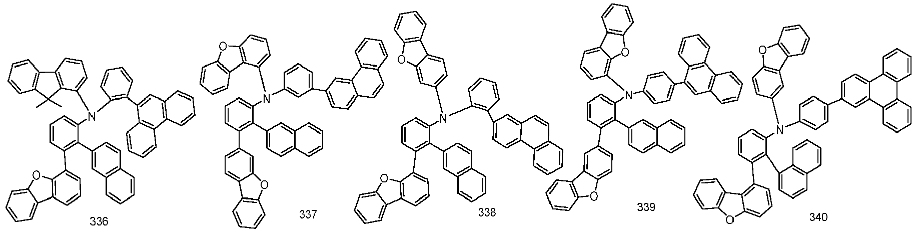

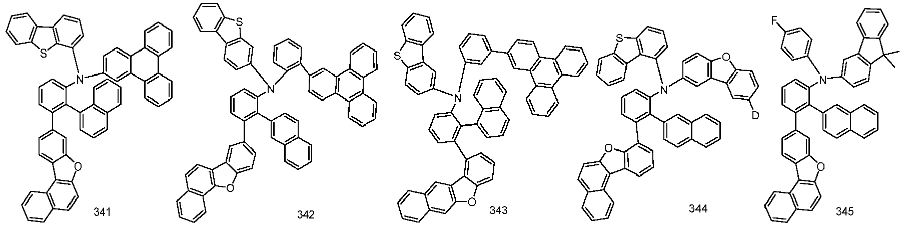

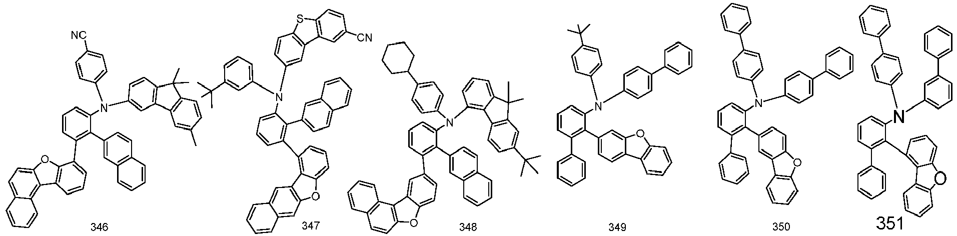

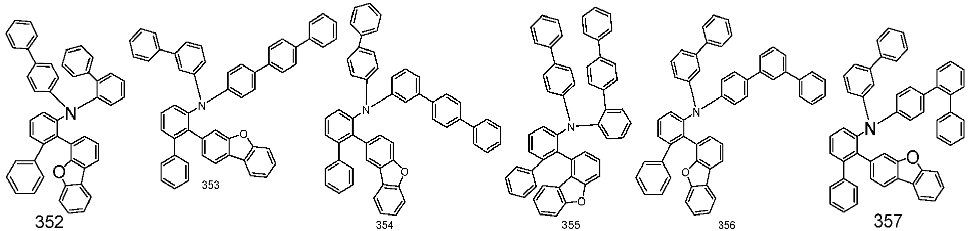

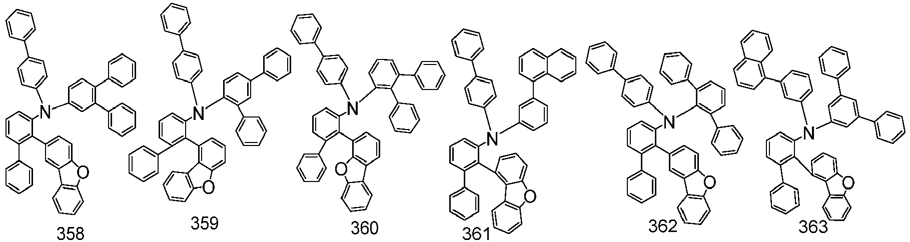

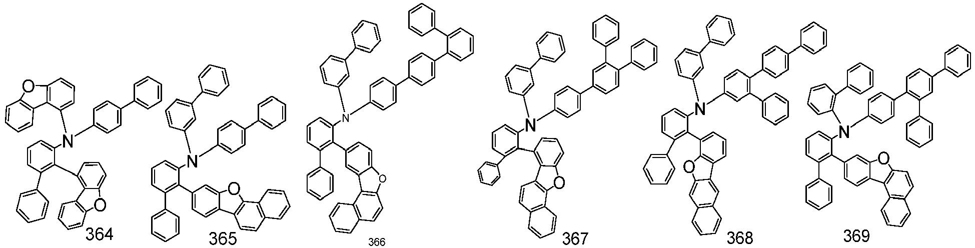

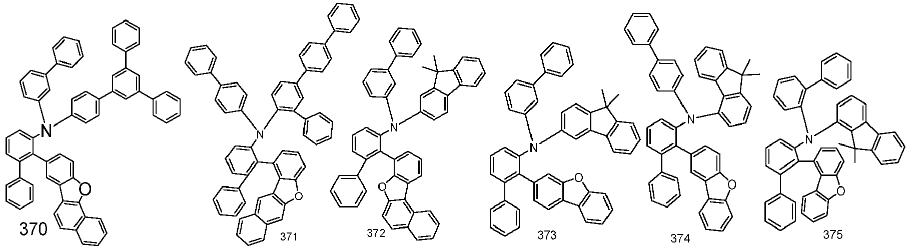

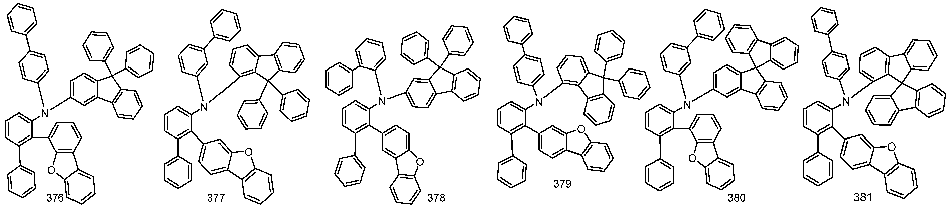

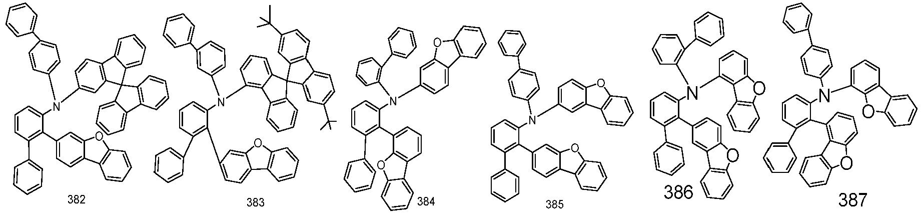

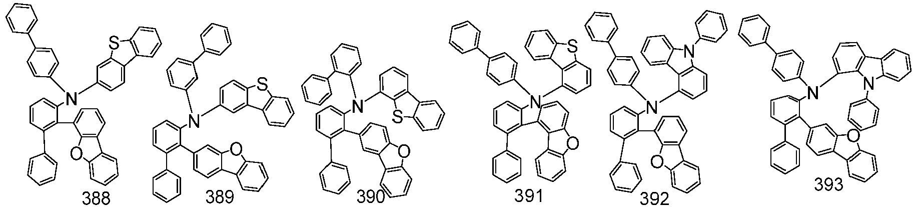

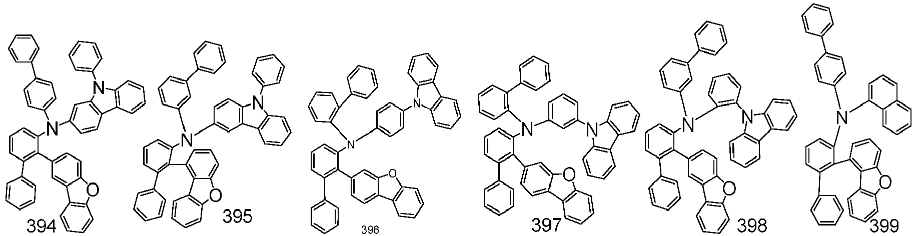

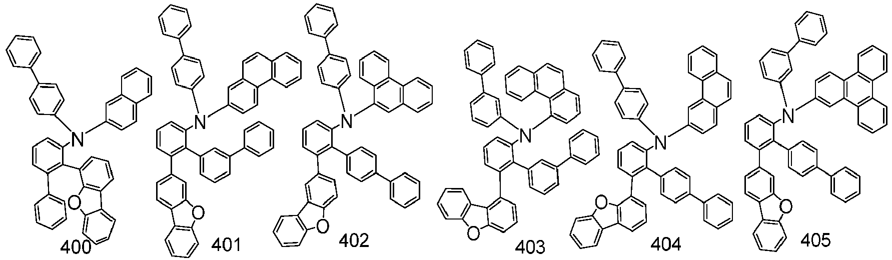

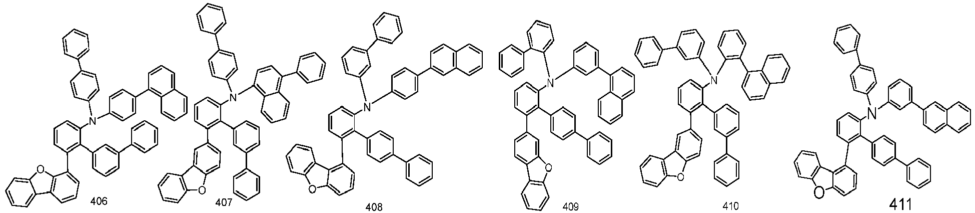

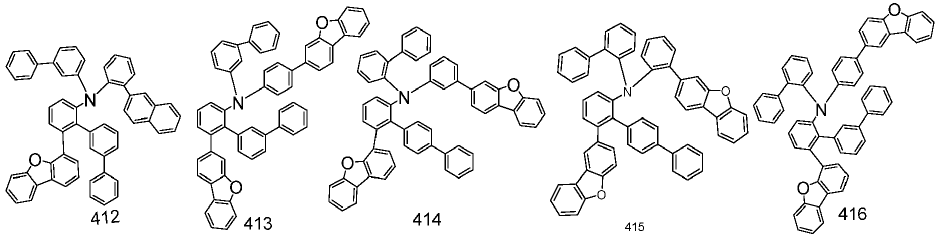

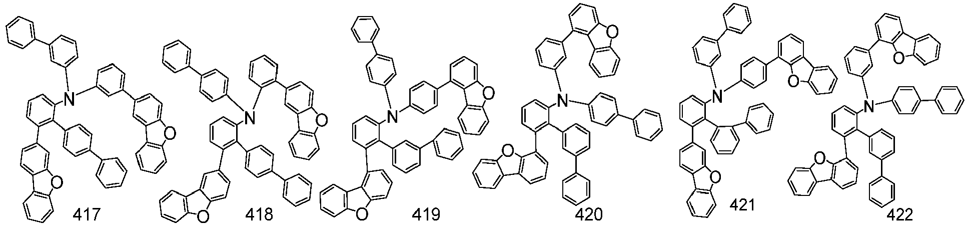

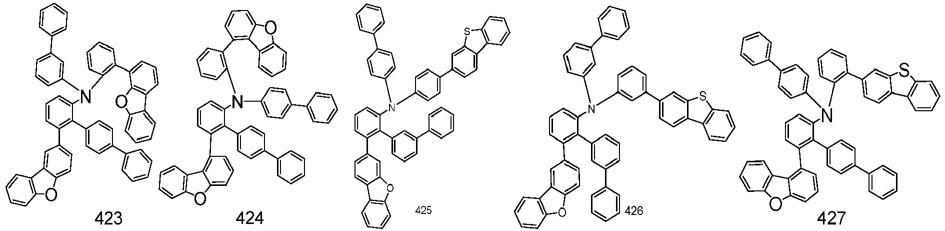

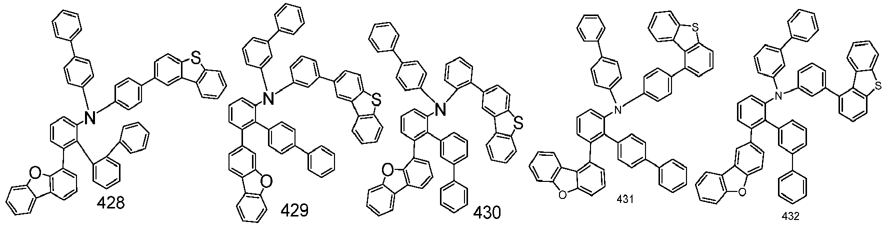

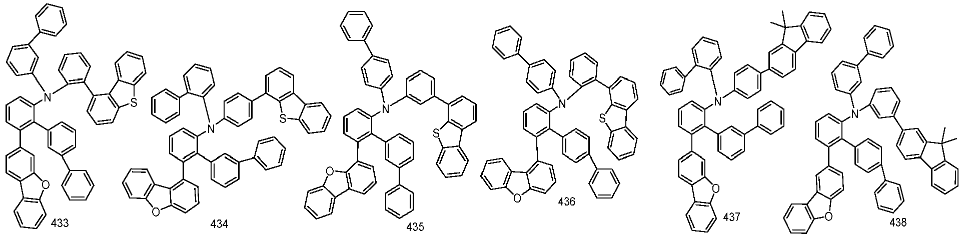

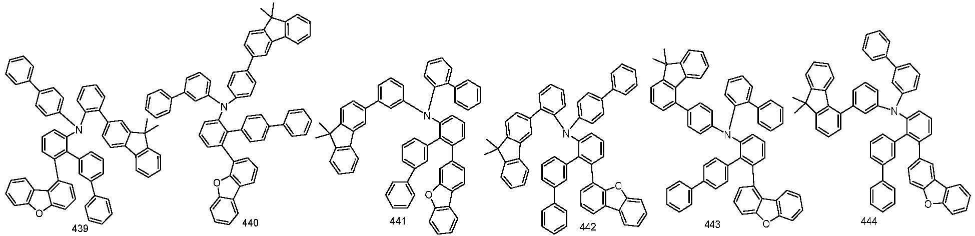

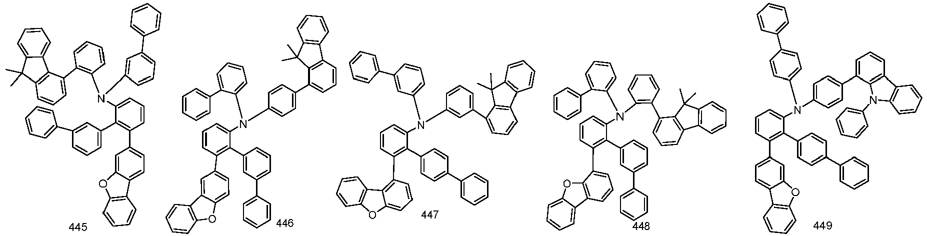

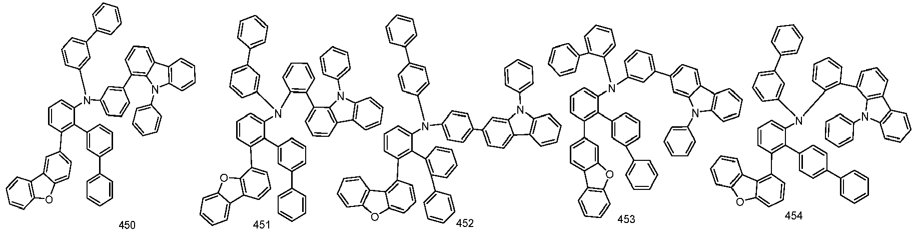

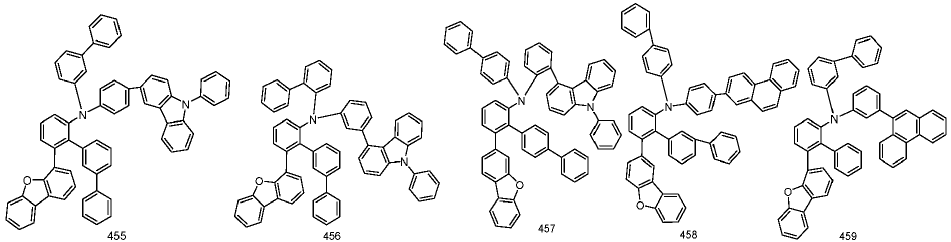

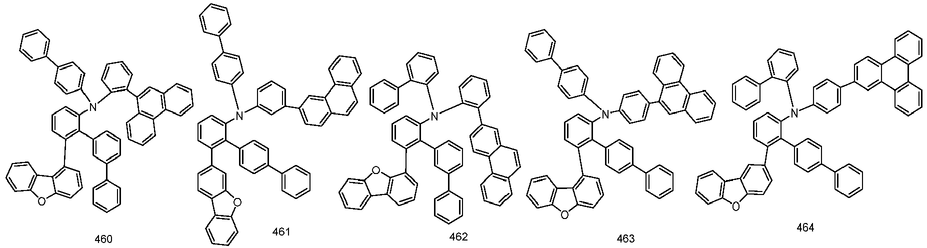

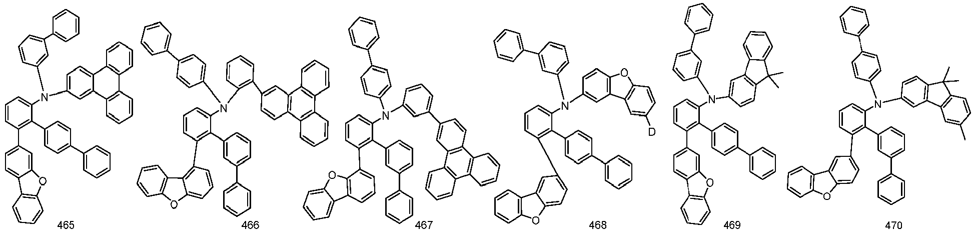

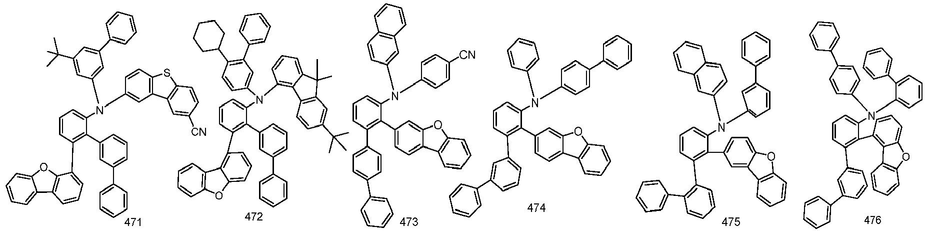

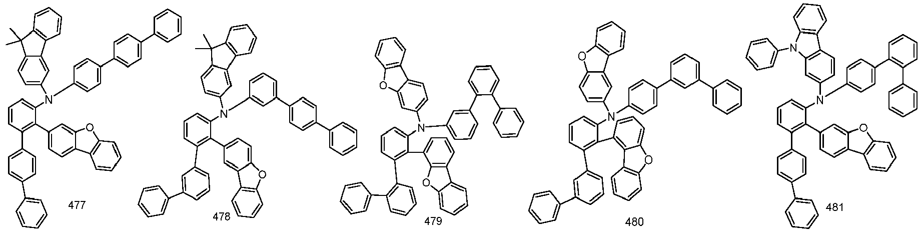

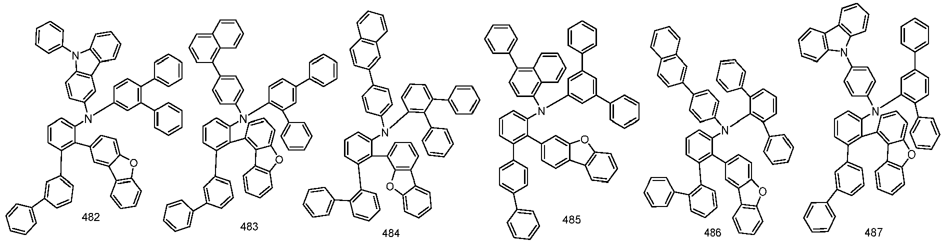

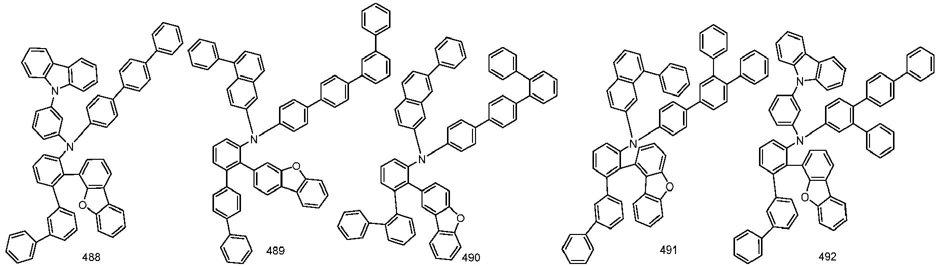

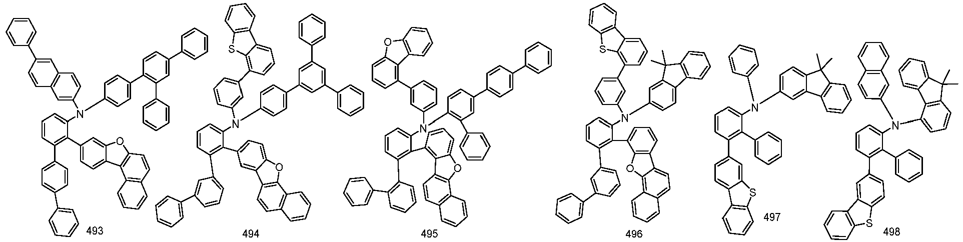

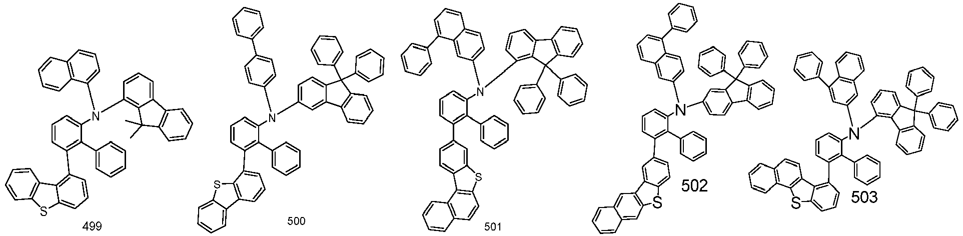

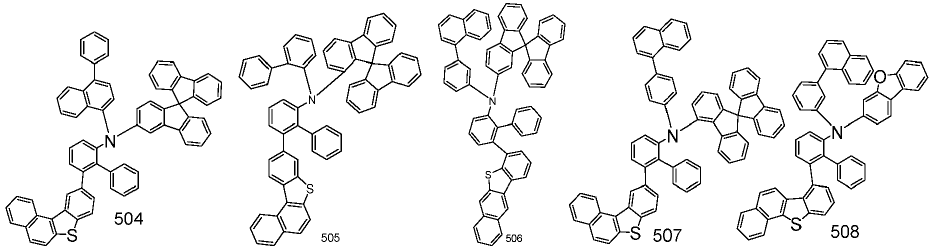

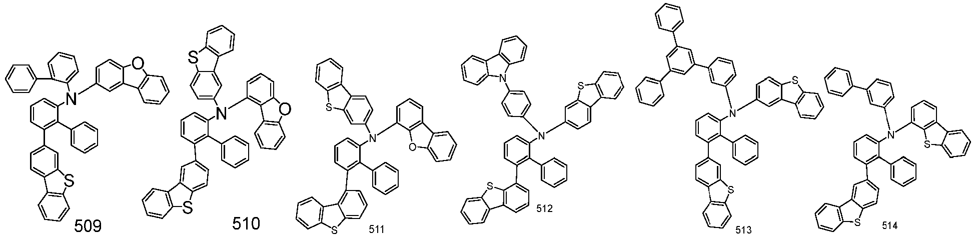

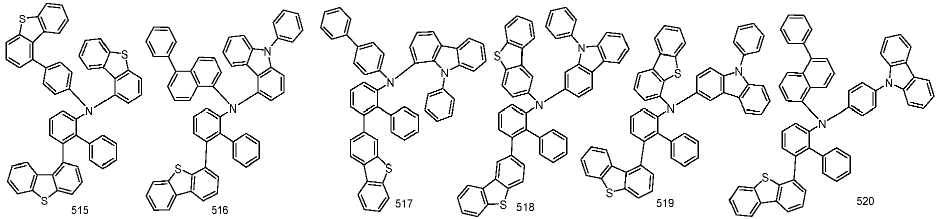

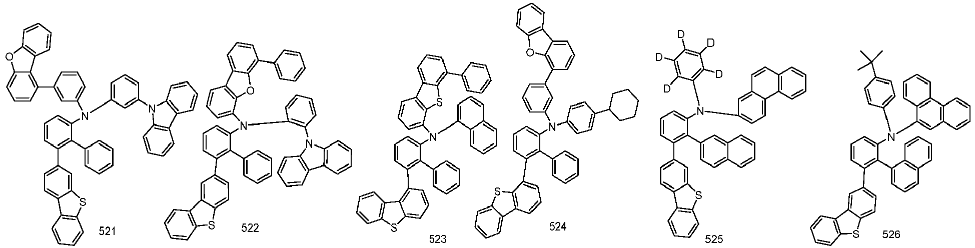

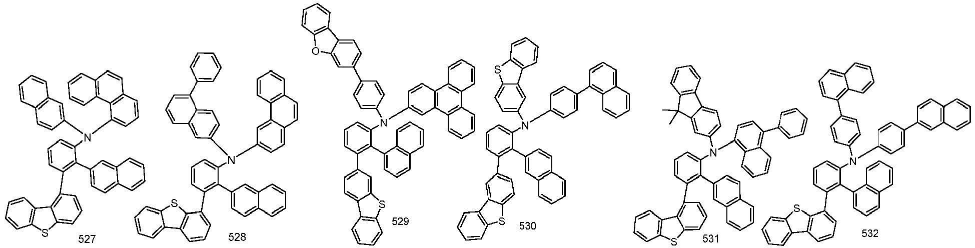

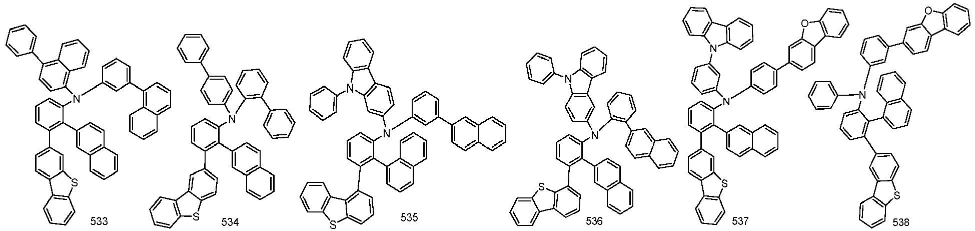

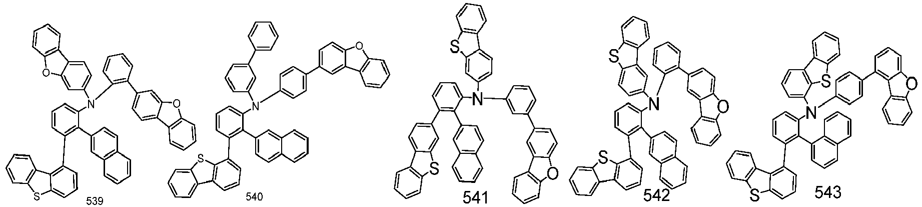

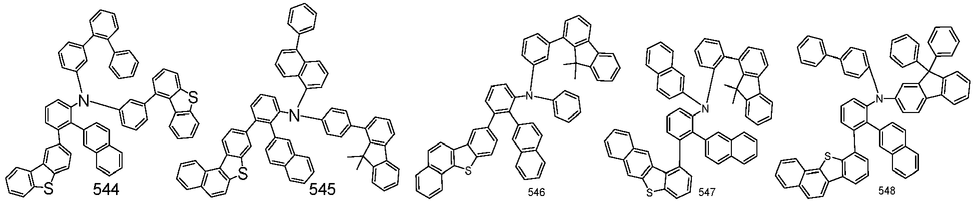

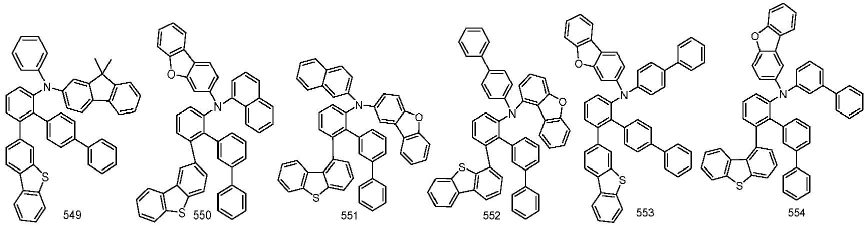

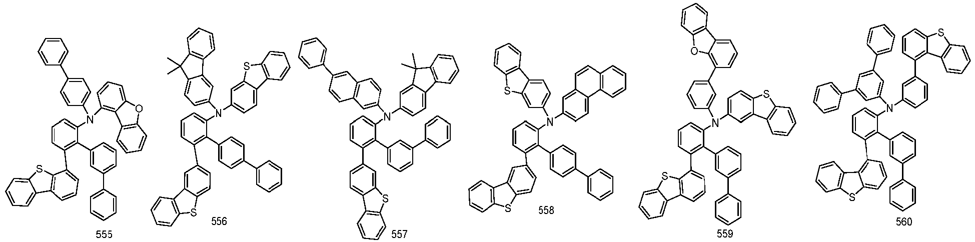

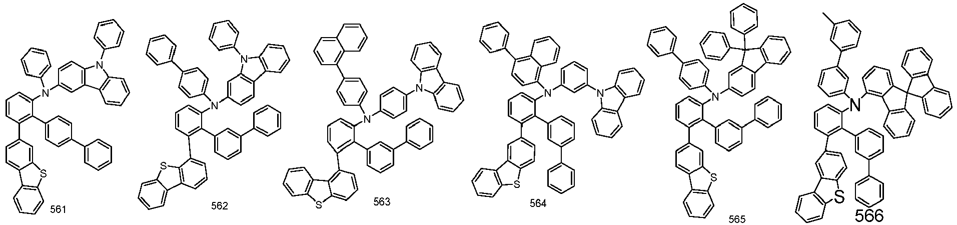

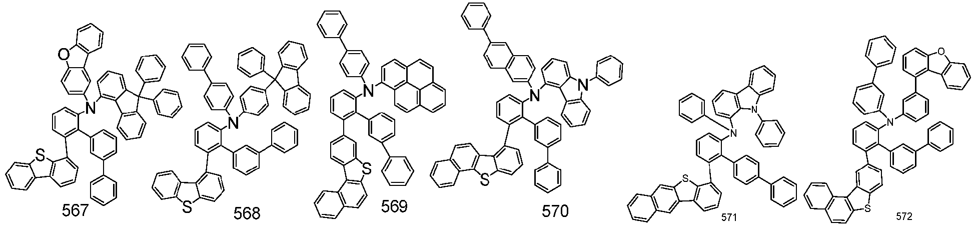

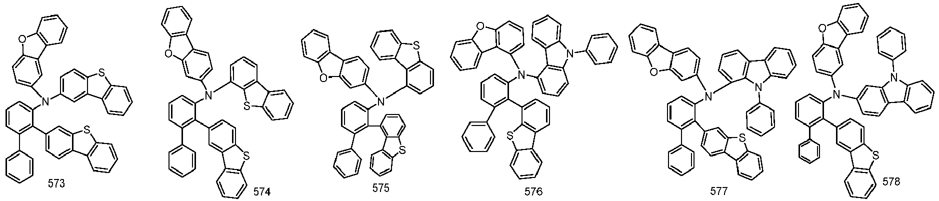

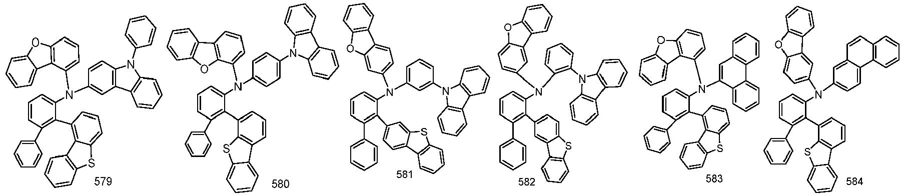

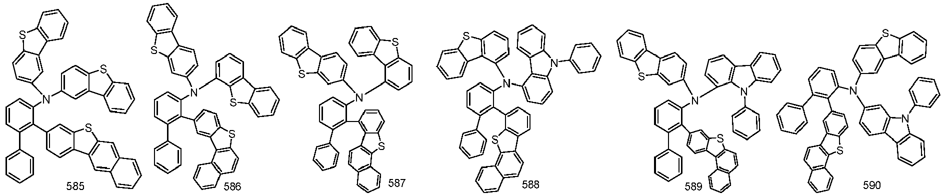

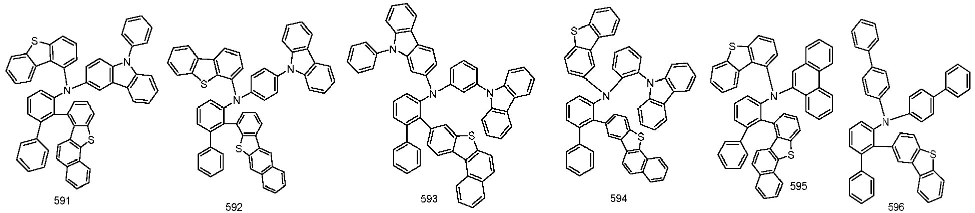

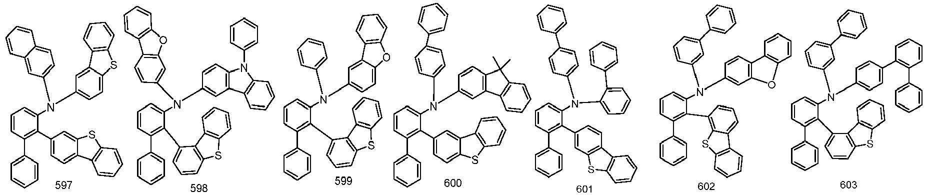

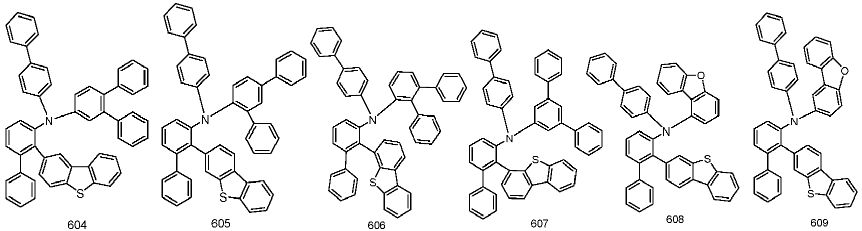

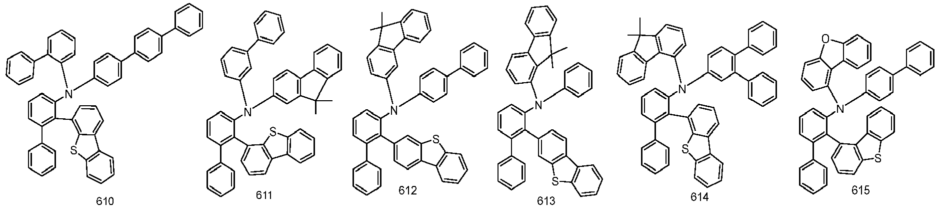

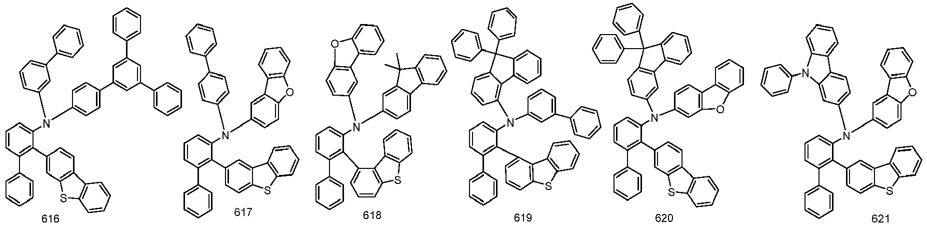

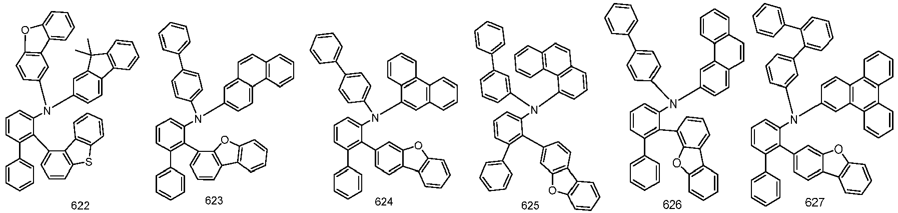

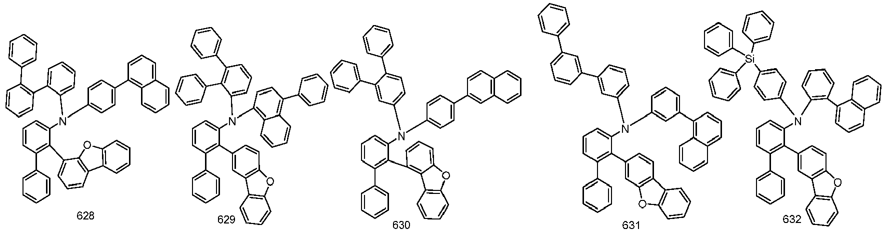

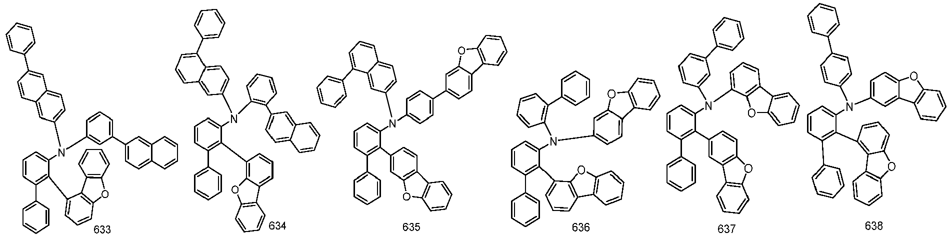

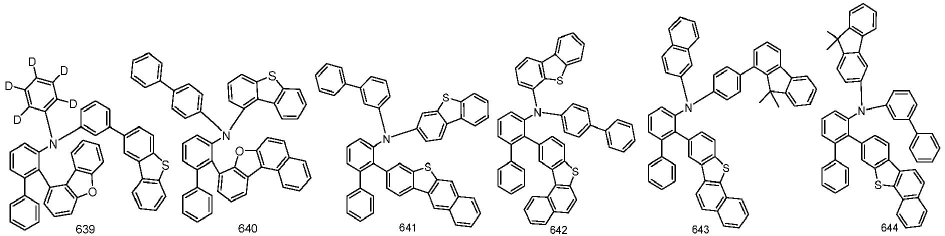

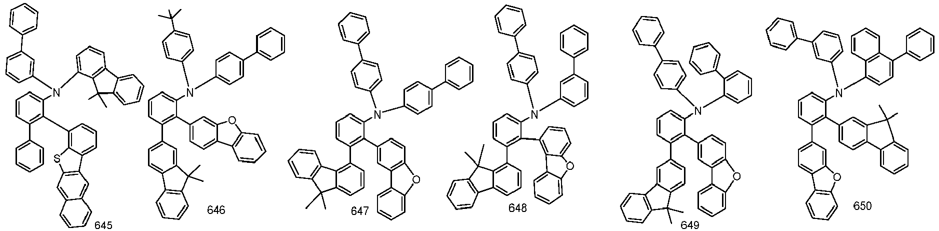

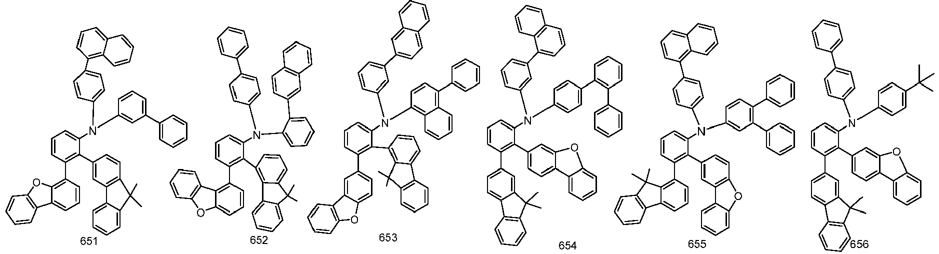

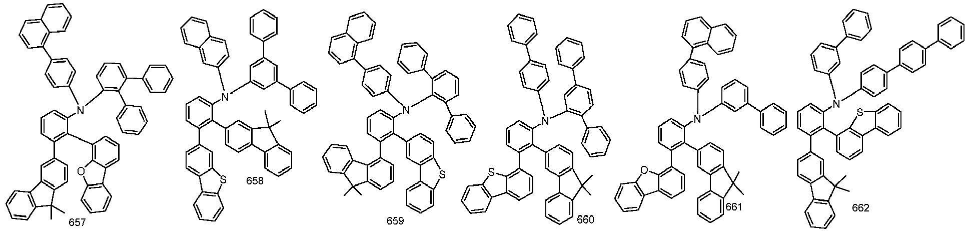

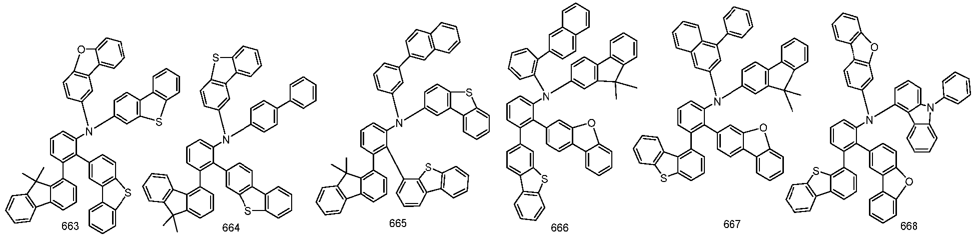

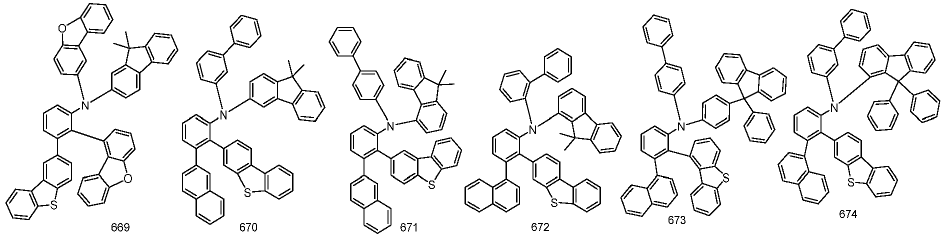

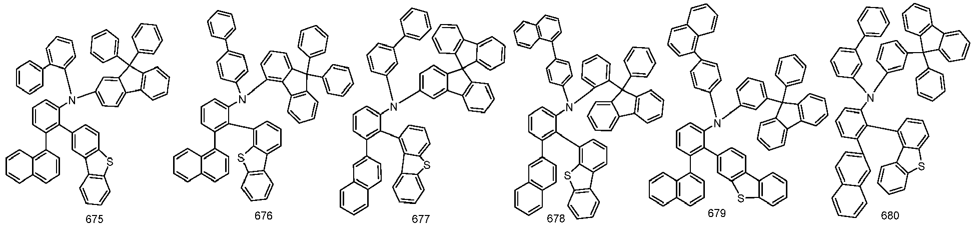

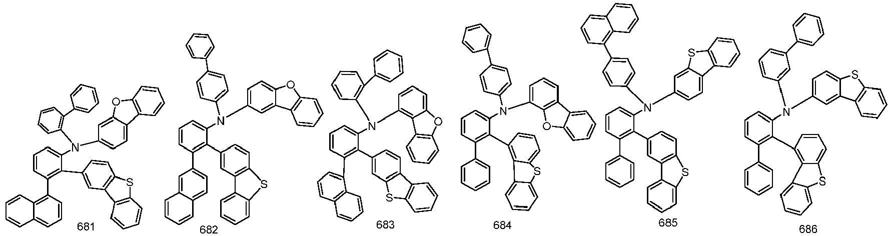

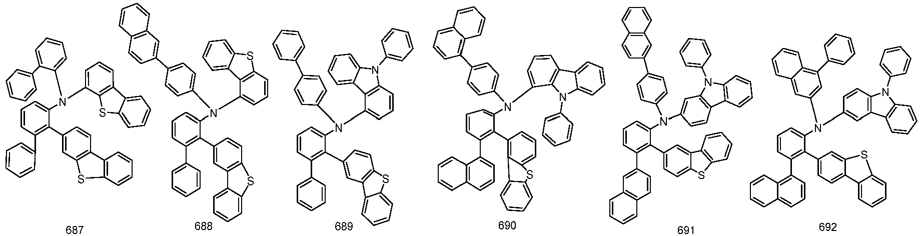

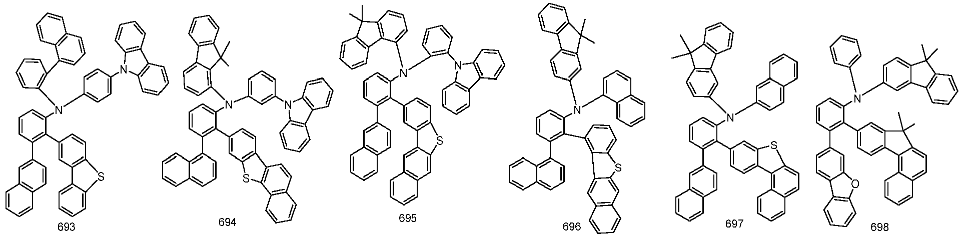

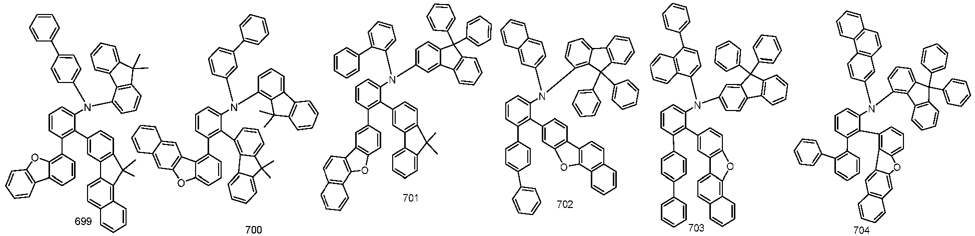

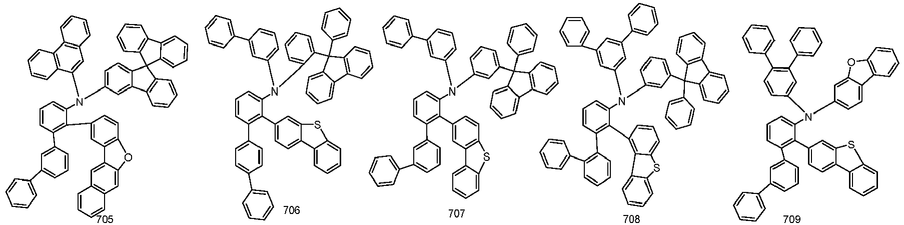

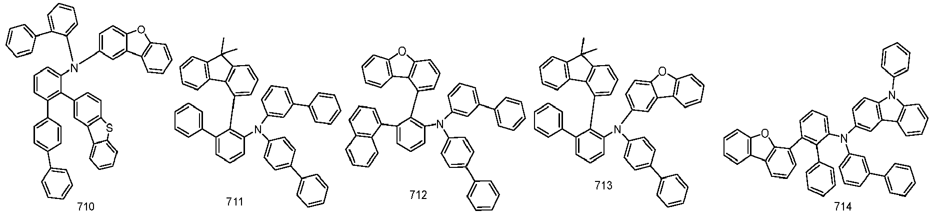

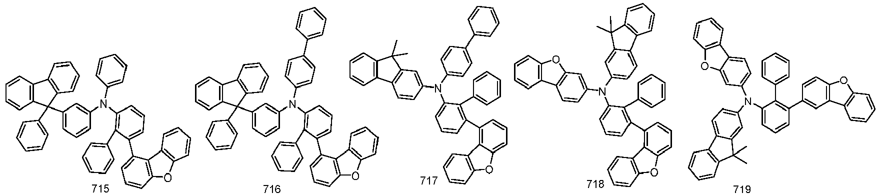

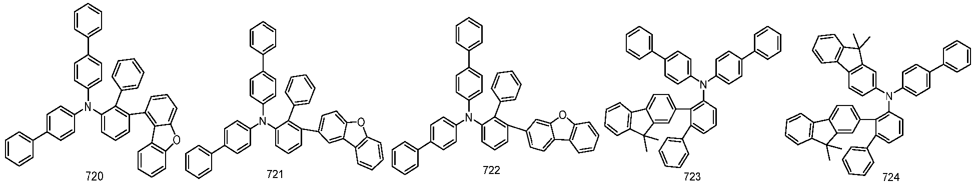

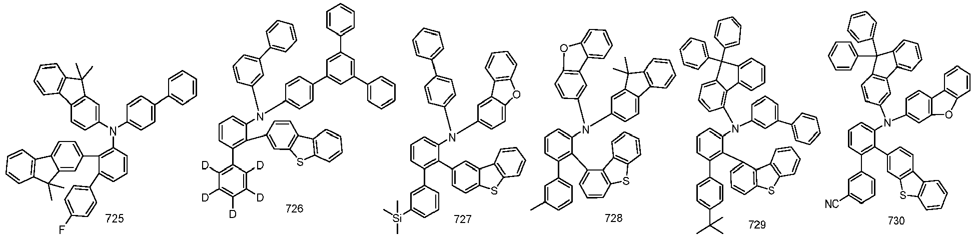

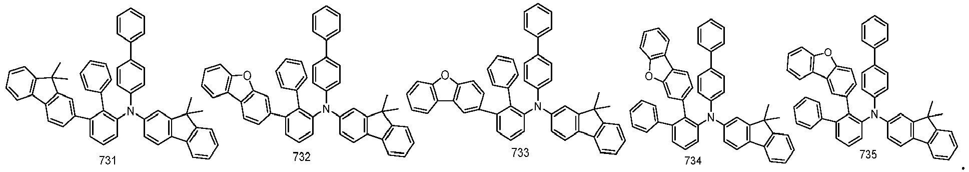

16. An electronic element, comprising an anode and a cathode which is arranged oppositely to the anode, and a functional layer arranged between the anode and the cathode, wherein
the functional layer comprises the organic electroluminescent material of any one of claims 1 to 15.

17. The electronic element of claim 16, wherein the functional layer comprises a hole transport layer, and the hole transport layer comprises the organic electroluminescent material;
preferably, the electronic element is an organic electroluminescent device or a photoelectric conversion device; and
more preferably, the electronic element is an organic electroluminescent device, the hole transport layer comprises a first hole transport layer and a second hole transport layer, and the first hole transport layer is closer to the anode than the second hole transport layer, wherein the second hole transport layer comprises the organic electroluminescent material.

18. An electronic device, comprising the electronic element of claim 16 or 17.
